# EUROPEAN PATENT APPLICATION

(11) **EP 4 595 967 A1**
(43) Date of publication of application: **06.08.2025**
(21) Application number: 23860955.6
(22) Date of filing: 01.09.2023
(51) Int. Cl.: A61K 35/742, A61K 38/16, A61P 43/00, A61P 11/00, A23L 33/135, A23L 33/195

(54) **BACILLUS SPECIES STRAIN EXPRESSING SUPEROXIDE DISMUTASE, BACILLUS SPECIES STRAIN SPORE AND/OR SUPEROXIDE DISMUTASE, AND USE THEREOF FOR PREVENTING OR TREATING OF FIBROTIC DISEASE**

(30) Priority: 01.09.2022 KR 20220111060
(71) Applicant: HLB Genex, Inc., Daejeon 34014 (KR); BiomLogic, Inc., Seoul 03923 (KR); The Asan Foundation, Seoul 05505 (KR); UNIVERSITY OF ULSAN FOUNDATION FOR INDUSTRY COOPERATION, Nam-gu Ulsan 44610 (KR)
(72) Inventor: PAN, Jae Gu, Seoul 03923 (KR); KIM, Eui Joong, Daejeon 34014 (KR); KIM, Jeong Hyun, Daejeon 34014 (KR); CHANG, In Ik, Seoul 03923 (KR); LEE, Sei Won, Seoul 06291 (KR)
(74) Representative: Ullrich & Naumann PartG mbB
(86) International application number: PCT/KR2023/013115
(87) International publication number: WO 2024/049280

(57) **Abstract**

The present invention relates to a composition for the prevention, alleviation, or treatment of fibrotic diseases, containing a Bacillus species strain, a Bacillus species strain spore, each expressing superoxide dismutase, and/or superoxide dismutase. When orally administered, the composition allows the polypeptide with superoxide dismutase activity to remove reactive oxygen species in vivo, thus being helpful for the prevention, improvement, or treatment of fibrotic diseases such as pulmonary fibrosis or hepatic fibrosis.

## Description

### Technical Field

The present application claims priority to Korean Patent Application No. 2022-0111060, filed on September 1, 2022, the disclosure of which is incorporated herein by reference in its entirety.

The present disclosure is related to the research (No. 2020R1A2C1008431, 'Exploring New Therapeutic Strategy for Intractable Fatal Disease COPD through Application of Microbial Symbiosis') conducted with the support of the Basic Research Program for Individuals of the National Research Foundation of Korea, funded by the Ministry of Science and ICT of Republic of Korea in 2022.

### Sequence listing

The present application includes a sequence listing that has been submitted electronically in XML format and is incorporated by reference herein in its entirety. A copy of the sequence listing, created on September 1, 2023, is named KC23094SEQ.xml and is 64.0 kilobytes in size.

The present disclosure relates to a composition comprising a *Bacillus sp.* strain expressing superoxide dismutase, a *Bacillus sp.* strain spore, and/or a superoxide dismutase (SOD), and a use thereof for preventing, ameliorating, or treating a fibrotic disease.

### Background Art

Fibrosis is a disease in which extracellular matrix (ECM), such as collagen, accumulates in tissues, causing structural and functional problems. Fibrosis may be either idiopathic with unknown cause or developed by various underlying factors. For example, pulmonary fibrosis may be caused by various conditions, including chronic inflammation (for example, sarcoidosis, Wegener's granulomatosis, and the like), infection, environmental factors (for example, exposure to asbestos or certain gases, and the like), exposure to ionizing radiation (for example, radiation therapy to treat breast tumor, and the like), chronic disease, and adverse effects of certain medications (for example, bleomycin, amiodarone, busulfan, methotrexate, nitrofurantoin, and the like). In addition, idiopathic pulmonary fibrosis of unknown cause is a disease that typically causes fibrosis in the lungs, and it is known that oxidative stress plays an important role in development of idiopathic pulmonary fibrosis. Patients with idiopathic pulmonary fibrosis have been found to have a high concentration of reactive oxygen species (ROS) and decreased antioxidant activity compared to healthy individuals, which can lead to irreversible lung damage and fibrosis. However, the etiology of idiopathic pulmonary fibrosis is not precisely known. Although drugs such as IFN-γ are under development, there are no effective drugs yet. As another example, hepatic fibrosis may be caused by nonalcoholic fatty liver disease (NAFLD), such as nonalcoholic steatohepatitis (NASH), viral hepatitis (HBV, HCV, and the like), alcohol, liver toxins (overdosed acetaminophen, chemicals such as arsenic, and the like), genetic causes, and the like. In particular, NASH is one of the typical diseases that cause hepatic fibrosis, accompanied by liver cell damage and inflammation along with fat deposition. Worsening of hepatic fibrosis may lead to cirrhosis; and further worsening thereof may result in liver cancer. There is a need for drugs to treat nonalcoholic steatohepatitis and prevent progression of the disease; however, there are currently no drugs that effectively improve fibrosis and steatohepatitis.

Therefore, there is a need to develop drugs that can treat fibrotic diseases including pulmonary fibrosis, NASH, and hepatic fibrosis, and the like.

### Disclosure of Invention

### Technical Problem

The object of the present disclosure is to solve all of the above-mentioned problems of the prior art.

An object of the present disclosure is to provide a *Bacillus sp.* strain, a *Bacillus sp.* strain spore, and/or a superoxide dismutase (SOD) for preventing or treating a fibrotic disease.

Another object of the present disclosure is to provide a pharmaceutical composition for preventing or treating a fibrotic disease, comprising a *Bacillus sp.* strain, a *Bacillus sp.* strain spore, and/or an SOD.

Yet another object of the present disclosure is to provide a food composition for preventing or ameliorating a fibrotic disease, comprising a *Bacillus sp.* strain, a *Bacillus sp.* strain spore, and/or an SOD.

Still yet another object of the present disclosure is to provide a method for preventing, ameliorating, or treating a fibrotic disease, comprising administering, to a subject, *a Bacillus sp.* strain, a *Bacillus sp.* strain spore, and/or an SOD.

Still yet another object of the present disclosure is to provide a use of *a Bacillus sp.* strain, *a Bacillus sp.* strain spore, and/or a superoxide dismutase for preventing, ameliorating, or treating a fibrotic disease.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided a composition for treating, ameliorating, or preventing a fibrotic disease, comprising, as an active ingredient, at least one selected from the group consisting of a *Bacillus sp.* strain, a *Bacillus sp.* strain spore, and a polypeptide having superoxide dismutase (SOD) activity.

In an embodiment, the *Bacillus sp.* strain may be an SOD-expressing strain or a strain that has undergone mutation or recombination to overexpress an SOD.

In another embodiment, the *Bacillus sp.* strain spore may comprise spores derived from an SOD-expressing strain or a strain that has undergone mutation or recombination to overexpress an SOD.

In yet another embodiment, the *Bacillus sp.* strain may be a *Bacillus velezensis* species strain.

In still yet another embodiment, the *Bacillus sp.* strain may be at least one strain selected from the *Bacillus velezensis* strain deposited under the accession number KCTC 13222 BP, the *Bacillus velezensis* strain deposited under the accession number KCTC 13227 BP, and the *Bacillus velezensis* strain deposited under the accession number KCTC 15552 BP.

In an embodiment, the polypeptide having SOD activity may be an Mn-SOD.

In another embodiment, the polypeptide having SOD activity may be a deamidated Mn-SOD.

In yet another embodiment, the polypeptide having SOD activity may be derived from a *Bacillus sp.* strain.

In still yet another embodiment, the strain from which the polypeptide having SOD activity is derived may be a *Bacillus velezensis* species strain.

In still yet another embodiment, the *Bacillus sp.* strain from which the polypeptide having SOD activity is derived may be at least one selected from the *Bacillus velezensis* strain deposited under the accession number KCTC 13222 BP, the *Bacillus velezensis* strain deposited under the accession number KCTC 13227 BP, and the *Bacillus velezensis* strain deposited under the accession number KCTC 15552 BP.

In still yet another embodiment, the polypeptide having SOD activity may comprise the amino acid sequence of SEQ ID NO: 2, SEQ ID NO: 4, SEQ ID NO: 5, or SEQ ID NO: 6.

In an embodiment, the polypeptide having SOD activity may be coated with a coating agent.

In another embodiment, the coating agent of the polypeptide having SOD activity may comprise shellac.

In an embodiment, the fibrotic disease may be a pulmonary fibrotic disease or a hepatic fibrotic disease.

In another embodiment, the pulmonary fibrotic disease may be selected from the group consisting of interstitial lung disease, pulmonary fibrosis, and idiopathic pulmonary fibrosis.

In yet another embodiment, the hepatic fibrotic disease may be selected from the group consisting of nonalcoholic steatohepatitis, hepatic fibrosis, idiopathic hepatic fibrosis, cirrhosis, alcoholic steatohepatitis, chronic liver disease, and viral hepatitis.

In an embodiment, the active ingredient of the composition may be orally administered.

In another embodiment, the composition may comprise at least two components selected from the group consisting of the *Bacillus sp.* strain, the *Bacillus sp.* strain spore, and the polypeptide having SOD activity, and the at least two components may be administered simultaneously, sequentially, or in reverse order.

In an embodiment, the composition may be a pharmaceutical composition.

In an embodiment, the composition may be a food composition.

In an embodiment, the composition may be a veterinary composition.

In an embodiment, the composition may be a feed composition.

According to another aspect of the present disclosure, there is provided a method for preventing, ameliorating, or treating a fibrotic disease, comprising administering, to a subject, a composition comprising at least one selected from the group consisting of a *Bacillus sp.* strain, *a Bacillus sp.* strain spore, and a polypeptide having superoxide dismutase activity.

According to yet another aspect of the present disclosure, there is provided a use of a composition, which comprises at least one selected from the group consisting of a *Bacillus sp.* strain, a *Bacillus sp.* strain spore, and a polypeptide having superoxide dismutase activity, for preventing, ameliorating, or treating a fibrotic disease.

### Advantageous Effects of Invention

The present inventors have identified that the composition of the present disclosure, which comprises at least one selected from the group consisting of a *Bacillus sp.* strain, a *Bacillus sp.* strain spore, and superoxide dismutase, has a prophylactic or therapeutic effect on a fibrotic disease. In an embodiment of the present disclosure, it was found that the *Bacillus sp.* strain spore of the present disclosure showed an effect of ameliorating fibrosis when administered either before or after induction of pulmonary fibrosis, and the *Bacillus sp.* strain spore with increased SOD expression level showed a better prophylactic or therapeutic effect. In addition, an SOD exhibited a superior effect in removing reactive oxygen species in the body in a case of being administered in the form of a species strain spore rather than a free SOD, which indicates that the spore form is more stable in the body. On the other hand, the oral SOD with increased stability against gastric acid was effective in ameliorating pulmonary fibrosis even in a case of being administered alone, and exhibited a synergistic effect in a case of being administered in combination with SOD-expressing normal strain spores. In another embodiment of the present disclosure, it was identified that the *Bacillus sp.* strain of the present disclosure exhibited an excellent therapeutic effect on hepatic fibrosis in a nonalcoholic steatohepatitis animal model, and this effect was further enhanced in a case where a SOD-overexpressing variant strain was administered. In addition, administration of the composition resulted in an improvement in hepatic fibrosis, an improvement in nonalcoholic steatohepatitis indicators (such as whole blood glucose level, plasma ALT level, plasma CK-18 level, and liver triglyceride content), and a significant decrease in NAFLD activity score (NAS). As such, it was identified that the composition as disclosed in the present disclosure is very effective in preventing, ameliorating, or treating a fibrotic disease.

### Brief Description of Drawings

FIG. 1 illustrates the overall procedure for producing a recombinant production strain (BSBA310) that expresses SodA2.
FIG. 2 illustrates an expression vector for overexpression of *sodA2* gene. Here, rrnBT1T2 represents a transcription terminator; rep(pBR322) represents a pBR322-derived replicon that operates in *E. coli*; rep(pUB110) represents a pUB110-derived replicon that operates in *B. subtilis;* Kan^{R} represents a kanamycin resistance gene (aminoglycoside O-nucleotidyltransferase); and BJ27 promoter represents a strong promoter for *B. subtilis.*
FIG. 3 illustrates a schematic diagram showing the cloning process of GF427 strain that is obtained by replacing the promoter of the GF423 strain to enhance SOD activity. FIG. 3A illustrates a process for constructing pUori-cm-amp-10sod using a PCR product obtained with GF423 genomic DNA as a template, and FIG. 3B illustrates a process for constructing pUori-cm-amp-P3-SOD using a PCR product obtained with pUori-cm-amp-10sod as a template.
FIG. 4 illustrates an administration schedule of test substances and bleomycin for identifying preventive effects on fibrosis in a pulmonary fibrosis mouse model.
FIG. 5 illustrates results obtained by measuring hydroxyproline content following administration of GF424 spores at different concentrations.
FIG. 6 illustrates results obtained by measuring the numbers of immune-related cells in bronchoalveolar lavage fluid following administration of GF424 spores at different concentrations. FIG. 6A shows the number of total immune cells (Total BAL cells), FIG. 6B shows the number of neutrophils, FIG. 6C shows the number of lymphocytes, and FIG. 6D shows the number of macrophages.
FIG. 7 illustrates results obtained by measuring TGF-β1 concentration following administration of GF424 spores at different concentrations.
FIG. 8 illustrates results obtained by performing staining (H&E and Masson's trichrome staining) of lung tissue sections following administration of GF424 spores at different concentrations. Scale bar represents 500 µm.
FIG. 9 illustrates results obtained by identifying, through real-time polymerase chain reaction, expression levels of pulmonary fibrosis-related gene markers (FIG. 9A, Col1a1; FIG. 9B, TGF-β; FIG. 9C, CTGF, and FIG. 9D, IL-6) following administration of GF424 spores at different concentrations.
FIG. 10 illustrates results obtained by measuring reactive oxygen byproducts (FIG. 10A, MDA; and FIG. 10B, 4-HNE) following administration of GF424 spores at different concentrations.
FIG. 11 illustrates results obtained by measuring hydroxyproline content following administration of spores from strains showing different SOD expression levels (SOD knock-out strain, SOD-expressing normal strain, and SOD-overexpressing strain).
FIG. 12 illustrates results obtained by measuring the number of immune-related cells in bronchoalveolar lavage fluid following administration of spores from strains showing differences in SOD expression level (SOD knock-out strain, SOD-expressing normal strain, and SOD-overexpressing strain). FIG. 12A shows the number of total immune cells (Total BAL cells), FIG. 12B shows the number of neutrophils, FIG. 12C shows the number of lymphocytes, and FIG. 12D shows the number of macrophages.
FIG. 13 illustrates results obtained by measuring TGF-β1 concentration following administration of spores from strains showing differences in SOD expression level (SOD knock-out strain, SOD-expressing normal strain, and SOD-overexpressing strain).
FIG. 14 illustrates results obtained by performing staining (H&E and Masson's trichrome staining) of lung tissue sections following administration of spores from strains showing differences in SOD expression level (SOD knock-out strain, SOD-expressing normal strain, and SOD-overexpressing strain). Scale bar represents 500 µm.
FIG. 15 illustrates results obtained by identifying, through real-time polymerase chain reaction, expression levels of pulmonary fibrosis-related gene markers (FIG. 15A, Col1a1; FIG. 15B, α-SMA; FIG. 15C, TGF-β; FIG. 15D, CTGF; FIG. 15E, TNF-α, and FIG. 15F, IL-6) following administration of spores from strains showing differences in SOD expression level (SOD knock-out strain, SOD-expressing normal strain, and SOD-overexpressing strain).
FIG. 16 illustrates results obtained by performing Western blotting for pulmonary fibrosis-related markers following administration of spores from strains showing differences in SOD expression level (SOD knock-out strain, SOD-expressing normal strain, and SOD-overexpressing strain).
FIG. 17 illustrates results obtained by estimating survival rate in a pulmonary fibrosis mouse model following administration of spores from strains showing differences in SOD expression level (SOD knock-out strain, SOD-expressing normal strain, and SOD-overexpressing strain).
FIG. 18 illustrates results obtained by measuring reactive oxygen byproducts (FIG. 18A, MDA; and FIG. 18B, 4-HNE) following administration of spores from strains showing differences in SOD expression level (SOD knock-out strain, SOD-expressing normal strain, and SOD-overexpressing strain).
FIG. 19 illustrates results obtained by measuring hydroxyproline content following administration of free SOD and strain spores.
FIG. 20 illustrates results obtained by measuring TGF-β1 concentration following administration of free SOD and strain spores.
FIG. 21 illustrates results obtained by performing staining (H&E and Masson's trichrome staining) of lung tissue sections following administration of free SOD and strain spores. Scale bar represents 500 µm.
FIG. 22 illustrates results obtained by identifying, through real-time polymerase chain reaction, expression levels of pulmonary fibrosis-related gene markers (FIG. 22A, Col1a1; FIG. 22B, α-SMA; FIG. 22C, TGF-β; FIG. 22D, CTGF; FIG. 22E, TNF-α, and FIG. 22F, IL-6) following administration of free SOD and strain spores.
FIG. 23 illustrates results obtained by performing Western blotting for pulmonary fibrosis-related markers following administration of free SOD and strain spores.
FIG. 24 illustrates results obtained by measuring reactive oxygen byproducts (FIG. 24A, MDA; and FIG. 24B, 4-HNE) following administration of free SOD and strain spores.
FIG. 25 illustrates results obtained by performing immunofluorescence staining of primary lung fibroblasts isolated from a pulmonary fibrosis mouse model. Respective lung fibroblasts were stained with α-smooth muscle actin (α-SMA), Collage 1 (Col1a1), and DAPI antibodies. The scale bar represents 50 µm.
FIG. 26 illustrates results obtained by identifying, through real-time polymerase chain reaction, expression levels of pulmonary fibrosis-related gene markers (FIG. 26A, TGF-β; FIG. 26B, Col1a1; FIG. 26C, TNF-α, and FIG. 26D, IL-6) in primary lung fibroblasts isolated from a pulmonary fibrosis mouse model.
FIG. 27 illustrates results obtained by measuring hydroxyproline content following administration of oral SOD (GF103), administration of strain spores, and co-administration of the oral SOD and the strain spores in a pulmonary fibrosis mouse model.
FIG. 28 illustrates results obtained by measuring TGF-β1 concentration following administration of oral SOD (GF103), administration of strain spores, and co-administration of the oral SOD and the strain spores in a pulmonary fibrosis mouse model.
FIG. 29 illustrates results obtained by performing staining (H&E and Masson's trichrome staining) of lung tissue sections following administration of oral SOD (GF103), administration of strain spores, and co-administration of the oral SOD and the strain spores in a pulmonary fibrosis mouse model. Scale bar represents 500 µm.
FIG. 30 illustrates results obtained by identifying, through real-time polymerase chain reaction, expression levels of pulmonary fibrosis-related gene markers (FIG. 30A, Col1a1; FIG. 30B, α-SMA; FIG. 30C, TGF-β; FIG. 30D, IL-6; FIG. 30E, IL-1β, and FIG. 30F, TNF-α) following administration of oral SOD (GF103), administration of strain spores, and co-administration of the oral SOD and the strain spores in a pulmonary fibrosis mouse model.
FIG. 31 shows the results of measuring reactive oxygen byproducts (FIG. 31A, MDA; FIG. 31B, 4-HNE; and FIG. 31C, 8-OHdG) following administration of oral SOD (GF103), administration of strain spores, and co-administration of the oral SOD and the strain spores in a pulmonary fibrosis mouse model.
FIG. 32 illustrates an administration schedule of test substances and bleomycin for identifying therapeutic effects on fibrosis in a pulmonary fibrosis mouse model.
FIG. 33 illustrates results obtained by measuring hydroxyproline content following administration of spores from GF424, which is a SOD-overexpressing strain, and GF427, which is a strain expressing more SOD than GF424, in a pulmonary fibrosis mouse model.
FIG. 34 illustrates results obtained by measuring TGF-β1 concentration following administration of spores from SOD-overexpressing strains (GF424 and GF427) in a pulmonary fibrosis mouse model.
FIG. 35 illustrates results obtained by performing staining (H&E and Masson's trichrome staining) of lung tissue sections following administration of spores from SOD-overexpressing strains (GF424 and GF427) in a pulmonary fibrosis mouse model. Scale bar represents 500 µm.
FIG. 36 illustrates results obtained by identifying, through real-time polymerase chain reaction, expression levels of pulmonary fibrosis-related gene markers (FIG. 36A, Col1a1; FIG. 36B, TGF-β; FIG. 36C, CTGF; FIG. 36D, TNF-α; FIG. 36E, IL-6, and FIG. 36F, IL-1β) following administration of spores from SOD-overexpressing strains (GF424 and GF427) in a pulmonary fibrosis mouse model.
FIG. 37 illustrates results obtained by performing Western blotting for pulmonary fibrosis-related markers following administration of spores from SOD-overexpressing strains (GF424 and GF427) in a pulmonary fibrosis mouse model.
FIG. 38 illustrates results obtained by measuring reactive oxygen byproducts (FIG. 38A, MDA; FIG. 38B, 4-HNE; and FIG. 38C, 8-OHdG) and thiol (FIG. 38D), which is an important marker of oxidative stress, following administration of spores from SOD-overexpressing strains (GF424 and GF427) in a pulmonary fibrosis mouse model.
FIG. 39 illustrates graphs obtained by identifying expression levels of biomarkers associated with induction of pulmonary fibrosis following administration of spores from SOD-overexpressing strains (GF424 and GF427) in a pulmonary fibrosis mouse model. The graphs show expression levels of epithelial damage marker (FIG. 39A, SP-D), fibrosis markers (FIG. 39B, MMP-7; FIG. 39C, Tenascin-C, and FIG. 39D, Periostin), inflammatory marker (FIG. 39E, CXCL13), and thrombosis marker (FIG. 39F, PAI-1).
FIG. 40 illustrates results obtained by estimating survival rate in a pulmonary fibrosis mouse model following administration of spores from SOD-overexpressing strains (GF424 and GF427) in the pulmonary fibrosis mouse model.
FIG. 41 illustrates a diagram showing the left outer lobe that has been isolated and dissected from a liver sample, in which portions thereof used for an experiment are indicated and the experiment was conducted to identify therapeutic effects of *Bacillus velezensis* species strains on hepatic fibrosis in a nonalcoholic steatohepatitis STAM^{™} model.
FIG. 42 illustrates a graph obtained by identifying body weight changes of the mice, which belong to 10 groups, over a 21-day period during which nonalcoholic steatohepatitis (NASH, STAM^{™} model) was induced and treatment was performed.
FIG. 43 illustrates results obtained by identifying organ weight changes of the mice, which belong to 10 groups, on the 21st day when all treatments were completed after induction of nonalcoholic steatohepatitis (NASH, STAM^{™} model). FIG. 43A shows a table summarizing the results of body weight, liver weight, and liver-to-body weight ratio; FIG. 43B is a graph for the body weight; FIG. 43C is a graph for the liver weight; and FIG. 43D is a graph showing the results of the liver-to-body weight ratio.

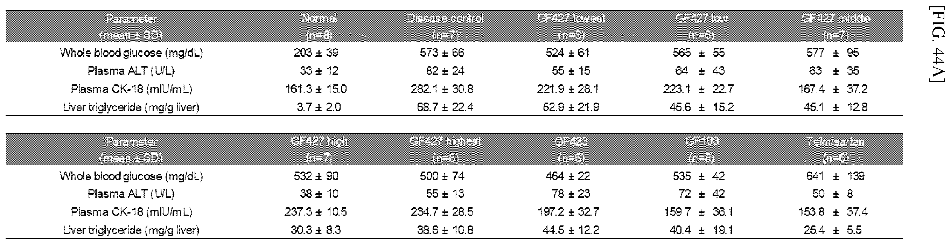

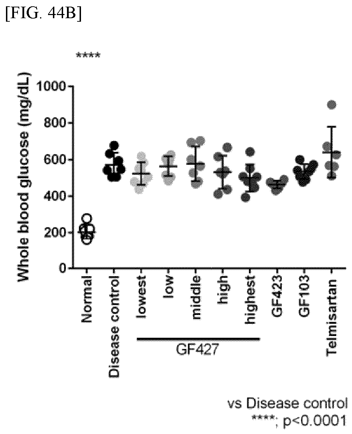

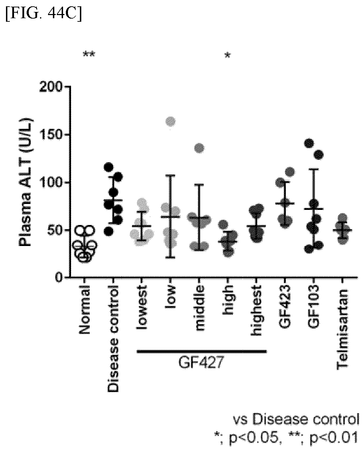

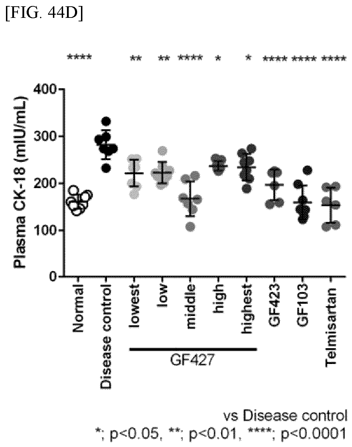

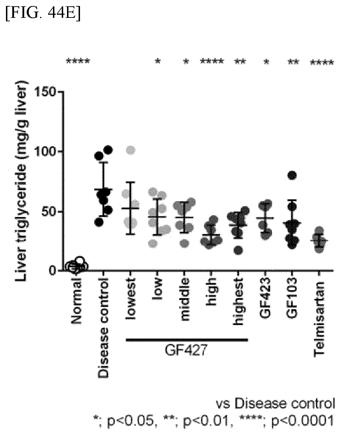

FIG. 44 illustrates results obtained by biochemical analysis performed using plasma and liver samples from a mouse model in which nonalcoholic steatohepatitis (NASH, STAM^{™} model) was induced and all treatments were completed. FIG. 44A shows a table summarizing the results of whole blood glucose level, plasma ALT level, plasma CK-18 level, and liver triglyceride content. FIG. 44B is a graph showing the whole blood glucose level, FIG. 44C is a graph showing the plasma ALT level, FIG. 44D is a graph showing the plasma CK-18 level, and FIG. 44E is a graph showing the liver triglyceride content.

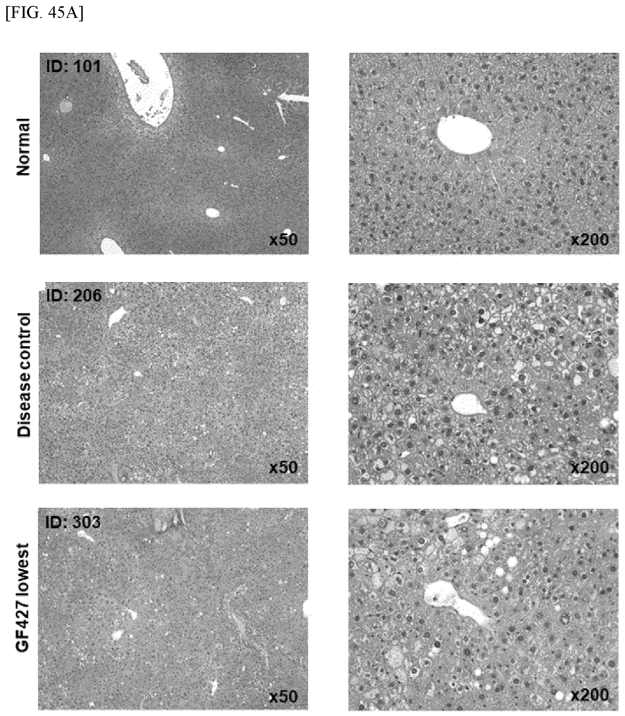

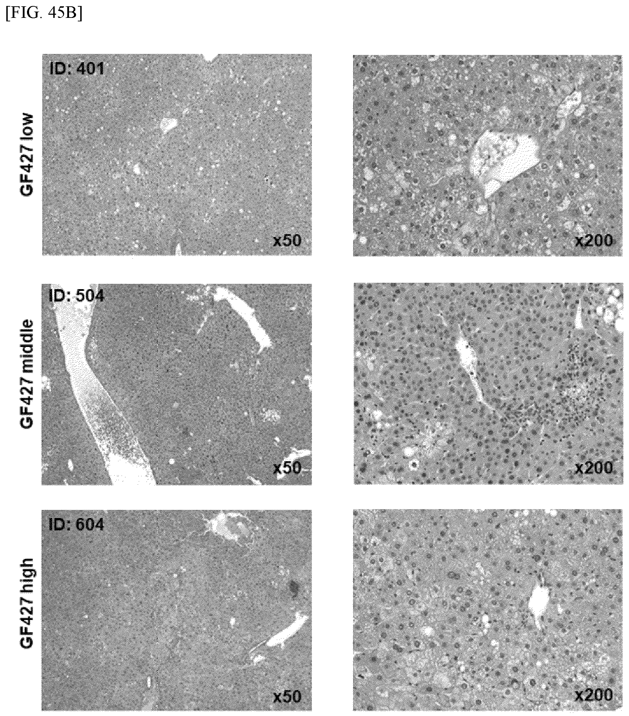

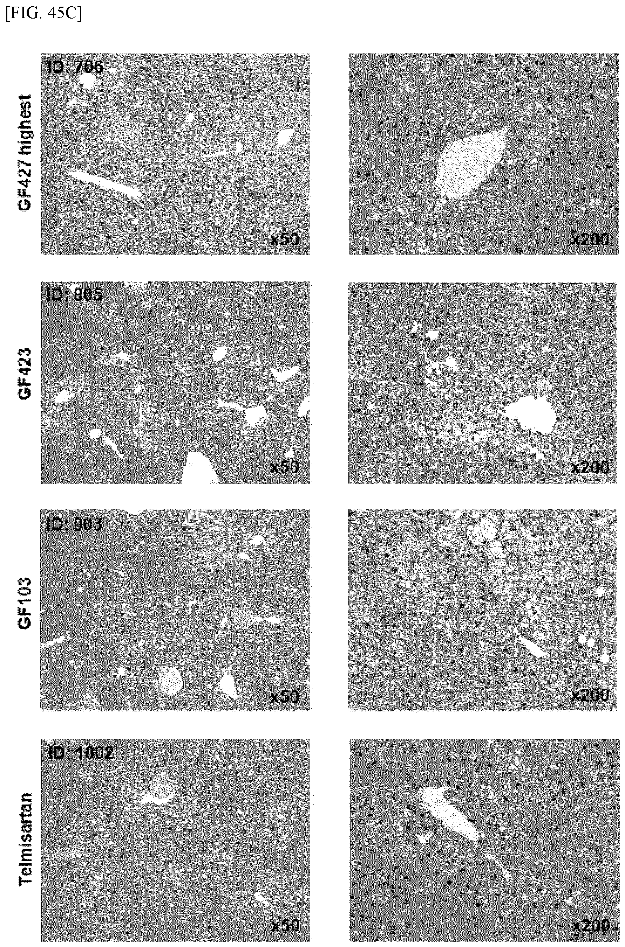

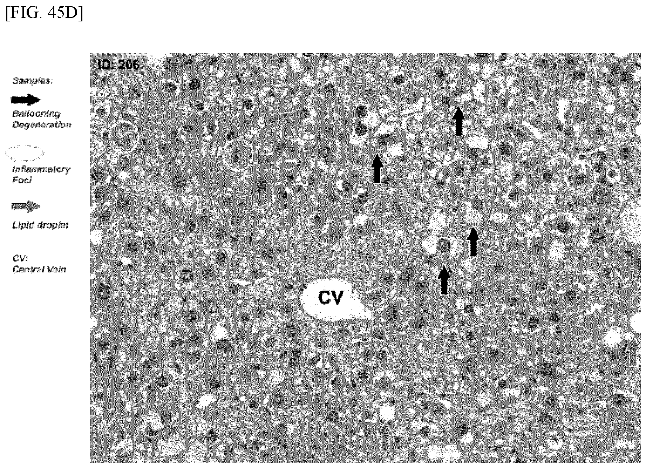

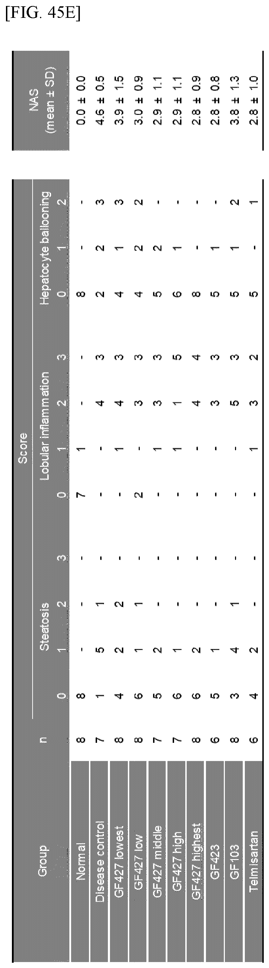

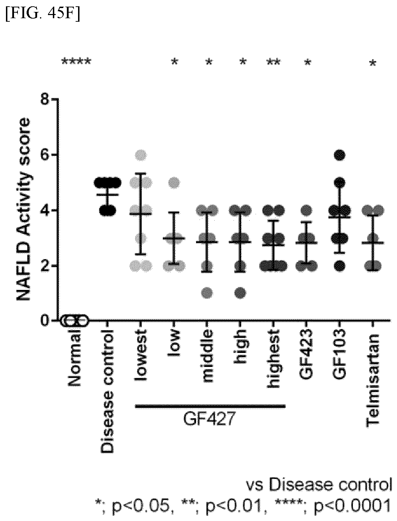

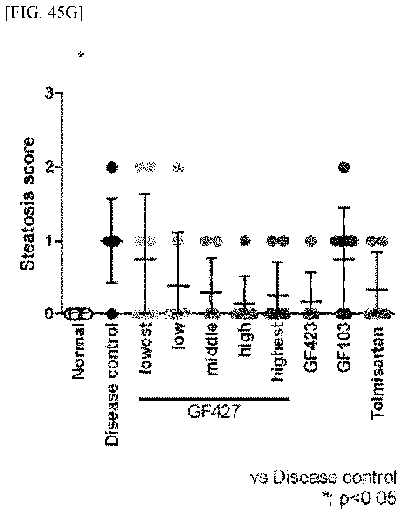

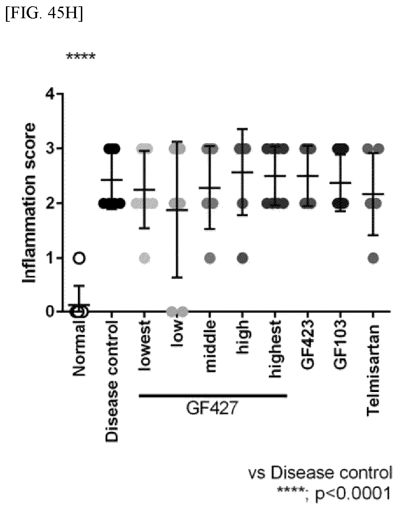

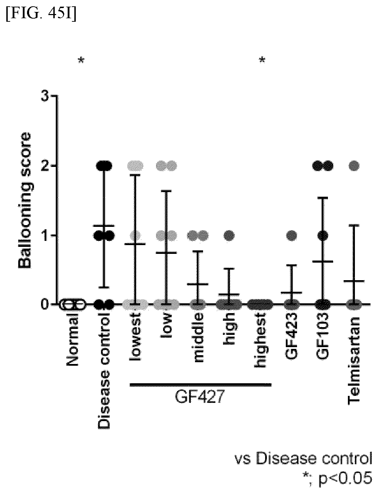

FIG. 45 illustrates results obtained by performing H&E staining and NAFLD activity score evaluation using liver samples from a mouse model in which nonalcoholic steatohepatitis (NASH, STAM^{™} model) was induced and all treatments were completed. FIGS. 45A to 45C show the results obtained by performing H&E staining of liver samples from the mice belonging to 10 groups; and FIG. 45D shows the results obtained by performing H&E staining of liver sections from the disease control group. FIG. 45E is a table that numerically shows the NAFLD activity score (NAS) calculated based on Kleiner's criteria; and FIG. 45F is a graph showing the NAS for each group. FIGS. 45G to 45I show the details of NAS, that is, steatosis score (FIG. 45G), inflammation score (FIG. 45H), and ballooning score (FIG. 45I).
FIG. 46 illustrates results obtained by performing Sirius red staining using liver samples from a mouse model in which nonalcoholic steatohepatitis (NASH, STAM^{™} model) was induced and all treatments were completed. FIG. 46A shows photomicrographs of Sirius redstained liver sections; FIG. 46B is a table that numerically shows the degree of fibrosis; and FIG. 46C is a graph that shows the degree of fibrosis (Sirius red positive) for each group.

### Modes for Carrying out Invention

The detailed description of the present disclosure set forth below will be described with reference to specific drawings with respect to specific embodiments in which the present disclosure may be practiced; however, the present disclosure is not limited thereto and, if properly described, is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment/example to another embodiment/example or implemented in combinations of embodiments/examples without departing from the spirit and scope of the present disclosure. Unless otherwise indicated, the terms used in describing the present disclosure are to be generally understood in their ordinary meaning and apply to the same terms used herein as well as to the aspect or embodiment of the disclosure for which the term is defined. For purposes of interpreting this specification, the following definitions will apply and whenever appropriate, the terms used in the singular will also include the plural and vice versa.

### Definition

The term "subject" is used interchangeably with "patient," and may be any mammal in need of prevention, amelioration or treatment of a fibrotic disease, such as primate (for example, human, monkey, chimpanzee, and the like), companion animal (for example, dog, cat, and the like), domestic animal (for example, cow, pig, horse, sheep, goat, and the like), and laboratory animal (for example, rat, mouse, guinea pig, and the like). In an embodiment of the present disclosure, the subject is a human.

The term "treatment" generally refers to obtaining a desired pharmacological and/or physiological effect. Such an effect has a therapeutic effect in that it partially or completely cures a disease and/or other unwanted or undesirable conditions (for example, a fibrotic disease such as pulmonary fibrosis or hepatic fibrosis). Desirable therapeutic effects include, but are not limited to, prevention of occurrence or recurrence of a disease, amelioration of symptoms, reduction of any direct or indirect pathological consequences of a disease, prevention of metastasis, reduction of disease progression rate, improvement or alleviation of disease state, and remission or improved prognosis. Preferably, the "treatment" may mean medical intervention for a disease or disorder that has already occurred.

The term "prevention" refers to obtaining a desired prophylactic pharmacological and/or physiological effect in terms of partially or completely preventing a disease or its symptoms.

The term "administration" refers to providing an active ingredient to a subject to achieve a prophylactic or therapeutic purpose (for example, prevention or treatment of a fibrotic disease such as pulmonary fibrosis or hepatic fibrosis).

### Use of Bacillus sp. strain, Bacillus sp. strain spore, and/or SOD for prevention or treatment of fibrotic disease

The present disclosure is based at least in part on the surprising discovery that administration of a composition, which comprises at least one selected from the group consisting of *a Bacillus sp.* strain, *a Bacillus sp.* strain spore, and a superoxide dismutase (SOD), is effective in preventing, ameliorating, or treating a fibrotic disease, such as a pulmonary fibrotic disease (interstitial lung disease, pulmonary fibrosis, idiopathic pulmonary fibrosis, and the like) or a hepatic fibrotic disease (nonalcoholic steatohepatitis, hepatic fibrosis, idiopathic hepatic fibrosis, cirrhosis, alcoholic steatohepatitis, chronic liver disease, viral hepatitis, and the like). Accordingly, according to an aspect of the present disclosure, there is provided a use of at least one selected from the group consisting of a *Bacillus sp.* strain, a *Bacillus sp.* strain spore, and a superoxide dismutase for preventing, ameliorating, or treating a fibrotic disease. In some embodiments, there is provided a use of a *Bacillus sp.* strain, a *Bacillus sp.* strain spore, or an SOD alone for preventing, ameliorating, or treating a fibrotic disease. In some embodiments, there is provided a use of a combination of two or more selected from a *Bacillus sp.* strain, a *Bacillus sp.* strain spore, and an SOD for preventing, ameliorating, or treating a fibrotic disease.

Fibrotic diseases or fibrosis are characterized by abnormal accumulation of collagen matrix following damage or inflammation that alters the structure and function of various tissues. Fibrotic diseases or fibrosis may occur in the tissues of different organs, including the kidneys, liver, lungs, heart, bone marrow, skin, and the like. For example, the fibrotic diseases or fibrosis may include, but are not limited to, hepatic fibrosis, cirrhosis, vocal cord scarring, vocal cord mucosal fibrosis, laryngeal fibrosis, pulmonary fibrosis, pancreatic fibrosis, myelofibrosis, myocardial infarction, post-myocardial infarction myocardial fibrosis myocardial fibrosis, endomyocardial fibrosis, splenic fibrosis, mediastinal fibrosis, lingual submucosal fibrosis, intestinal fibrosis (for example, accompanied by inflammatory bowel disease, and the like), retroperitoneal fibrosis, uterine fibrosis, scleroderma, and mammary fibrosis, and the like.

In some embodiments, the fibrotic disease may be pulmonary fibrosis. Pulmonary fibrosis includes not only pulmonary fibrosis (PF) in a narrow sense, but also interstitial lung disease (ILD) (pulmonary fibrosis or lung damage caused by autoimmune symptoms, viral infections, bacterial infections, administration of drugs such as anticancer agents (for example, bleomycin) and antibiotics, toxic substances, and the like) or idiopathic pulmonary fibrosis (IPF).

In some embodiments, the fibrotic disease may be pulmonary fibrosis (PF) or idiopathic pulmonary fibrosis (IPF).

In another embodiment, the fibrotic disease may be interstitial lung disease (ILD).

In some embodiments, the fibrotic disease may be hepatic fibrosis. Hepatic fibrosis includes not only hepatic fibrosis in a narrow sense, but also nonalcoholic steatohepatitis, (progressive) hepatic fibrosis, idiopathic hepatic fibrosis, cirrhosis, alcoholic steatohepatitis, chronic liver disease, or viral hepatitis.

In some embodiments, the fibrotic disease may be nonalcoholic steatohepatitis (NASH).

In an embodiment of the present disclosure, it was identified that *Bacillus velezensis* strain spores exhibited a significant effect in improving the degree of pulmonary fibrosis in a case of being orally administered before or after induction of pulmonary fibrosis (by bleomycin treatment), and this effect was further enhanced in a case where spores from an SOD-overexpressing variant strain, which expresses a higher level of SOD, are administered.

In another embodiment of the present disclosure, it was identified that the pulmonary fibrosis mouse model, in which pulmonary fibrosis had been induced by bleomycin after administration of SOD-overexpressing strain spores, exhibited an excellent 21-day survival rate that is nearly comparable to the normal mice without induced pulmonary fibrosis (preventive effect). In addition, in a case where SOD-overexpressing strain spores were administered to a mouse model in which pulmonary fibrosis had been induced by bleomycin treatment, the mouse model, although not showing a survival rate as high as the normal mice, exhibited a significantly increased survival rate compared to the bleomycin-treated group (therapeutic effect).

In yet another embodiment of the present disclosure, it was found that an oral SOD with increased stability against gastric acid was effective in ameliorating pulmonary fibrosis in a bleomycin-induced pulmonary fibrosis mouse model in a case of being administered alone, and the oral SOD exhibited an inhibitory effect on pulmonary fibrosis even in a case of being administered in combination with SOD-expressing normal strain spores.

In still yet another embodiment of the present disclosure, in a case where an animal model, in which NASH had been induced through streptozotocin solution and a high-fat diet, was orally administered with a *Bacillus velezensis* (*Bacillus amyloliquefaciens*) strain, the strain exhibited a significant effect in improving the degree of hepatic fibrosis, and this effect was further enhanced in a case of using a strain that expresses a higher level of SOD. In addition, the oral administration resulted in an improvement in nonalcoholic steatohepatitis indicators (such as whole blood glucose level, plasma ALT level, plasma CK-18 level, and liver triglyceride content), and a significant decrease in NAFLD activity score (NAS).

### Composition comprising Bacillus sp. strain, Bacillus sp. strain spore, and/or SOD

According to another aspect of the present disclosure, there is provided a composition for preventing, ameliorating, or treating a fibrotic disease, comprising, as an active ingredient, at least one selected from the group consisting of a *Bacillus sp.* strain, a *Bacillus sp.* strain spore, and an SOD. Specifically, the composition may comprise (i) a *Bacillus sp.* strain, (ii) *a Bacillus sp.* strain spore, (iii) an SOD, (iv) a *Bacillus sp.* strain and a *Bacillus sp.* strain spore, (v) a *Bacillus sp.* strain and an SOD, (vi) a *Bacillus sp.* strain spore and an SOD, or (vii) a *Bacillus sp.* strain, a *Bacillus sp.* strain spore, and an SOD.

The term "spore" as used herein may be used interchangeably with "spores", and the *Bacillus sp.* strain spores may be obtained by culturing *a Bacillus sp.* strain in a suitable medium, inducing sporulation, and then isolating the resulting spores.

In some embodiments, the *Bacillus sp.* strain spores may be obtained from GRAS bacteria that are generally regarded as safe for use in drugs or foods. *Bacillus sp.* strain spores are known to be resistant to proteases and low pH (see Cutting SM. Bacillus probiotics. Food Microbiol. 2011;28:214-220. doi: 10.1016/j.fm.2010.03.007; and Wang Y, et al., In vitro assessment of probiotic properties of Bacillus isolated from naturally fermented congee from inner Mongolia of China. World J. Microb. Biot. 2010;26:1369-1377. doi: 10.1007/s11274-010-0309-7).

In addition, *Bacillus sp.* strain spores are GRAS probiotics approved in several countries. Specifically, the *Bacillus sp.* strain may be, but is not limited to, *B. velezensis, B. amyloliquesfaciens, B. methylotrophicus, B. siamensis, B. subtilis, B. tequilensis, B. atrophaeus, B. mojavensis,* or *B. vallismortis.* The *Bacillus sp.* strain spores may be derived from, but are not limited to, the above-described strains. Preferably, the *Bacillus sp.* strain may be a *Bacillus velezensis (Bacillus amyloliquefaciens*) strain (for example, GF423 strain, GF424 strain, or GF427 strain). The *Bacillus sp.* strain spores may be derived from a *Bacillus velezensis (Bacillus amyloliquefaciens*) strain (for example, GF423 strain, GF424 strain, or GF427 strain). The GF423 strain, the GF424 strain, and the GF427 strain were deposited with the Korean Collection for Type Cultures on March 6, 2017, March 13, 2017, and August 14, 2023, respectively (Accession No. KCTC 13222 BP, Accession No. KCTC 13227 BP, and Accession No. KCTC 15552 BP). In addition, the characteristics and cultivation method of the GF423 strain are described in Korean Patent No. 1762199, the entire disclosure of which is incorporated herein by reference. Each of the above-deposited *Bacillus sp.* strains was classified and described as *Bacillus amyloliquefaciens* in previous applications. The strains were compared and analyzed again using genome-based classification methods DDH and ANI. As a result, it was suggested that each strain should be classified as a microorganism belonging to *Bacillus velezensis,* as it did not meet the species-level classification criteria. Such a strain was also listed as *"Bacillus velezensis"* in LPSN (List of Prokaryotic names with Standing in Nomenclature; https://lpsn.dsmz.de/species/bacillus-velezensis) (see Fan, Ben, et al. "Bacillus amyloliquefaciens, Bacillus velezensis, and Bacillus siamensis form an "operational group B. amyloliquefaciens" within the B. subtilis species complex." Frontiers in microbiology 8 (2017): 22). Given that *Bacillus velezensis* and *Bacillus amyloliquefaciens* are heterotypic synonyms, the two names can be used interchangeably.

In some embodiments, a *Bacillus amyloliquefaciens* strain may be used interchangeably with a *Bacillus velezensis* strain. Accordingly, *Bacillus amyloliquefaciens* strains GF423, GF424, and GF427 can be understood as the same strains as *Bacillus velezensis* strains GF423, GF424, and GF427.

The process by which the deposited *Bacillus sp.* strains were classified as *Bacillus velezensis* is as follows. Each of the deposited *Bacillus sp.* strains was isolated, and the 16S rRNA gene and whole genome thereof were compared with those of three highly homologous reference strains by DDH, ANI, and AAI analyses (genome-based classification methods) (see Table 1).

**[Table 1]**

| | *Bacillus amyloliquefaciens plantarum* | *Bacillus amyloliquefaciens amyloliquefaciens* | *Bacillus subtilis spizizenii* | Species classification criterion (threshold) (%) |
|---|---|---|---|---|
| Reference strain | FZB42 | DSM7 | NRRL B-23049 | - |
| 16S rRNA | 99.67~99.73% | 99.46~99.66% | 99.18~99.39% | 98.65% |
| DDH | 92.10% | 78.60% | 32.70% | 70% |
| ANI | 98.65% | 93.59% | 80.04% | 94% |
| AAI | 98.79% | 95.09% | 79.88% | 95% |

Referring to Table 1, the DDH and ANI results show that each of the deposited *Bacillus sp.* strains is more similar to *Bacillus amyloliquefaciens subspecies plantarum* than to *Bacillus amyloliquefaciens subspecies amyloliquefaciens* (in which the DDH species classification criterion is 70% or higher, and the ANI species classification criterion is 94% or higher). On the other hand, the *Bacillus amyloliquefaciens subspecies plantarum* was classified as a microorganism belonging to *Bacillus velezensis* rather than the same species as *Bacillus amyloliquefaciens subspecies amiriloliquefaciens,* as it did not meet the species-level classification criteria when compared, using the genome-based classification methods DDH and ANI, with *Bacillus amyloliquefaciens* DSM7 that is a type strain of *Bacillus amyloliquefaciens subspecies amiriloliquefaciens.* In some embodiments, the *Bacillus sp.* strain may be a strain that expresses or produces an SOD or may be a variant strain that has undergone mutation or recombination to overexpress or overproduce an SOD. In some embodiments, the *Bacillus sp.* strain spore may be a spore from a strain that expresses or produces an SOD. In addition, the *Bacillus sp.* strain spore may be derived from a strain that has undergone mutation or recombination to overexpress or overproduce an SOD. For example, the *Bacillus sp.* strain may be a naturally isolated strain (for example, GF423 strain) or a variant strain that has undergone mutation or recombination to overexpress or overproduce an SOD (for example, GF424 or GF427 strain).

In another embodiment, the *Bacillus sp.* strain spore may be derived from another *Bacillus sp.* strain that has undergone recombination to express an SOD of a *Bacillus velezensis* strain. This recombinant strain will be further described below.

Sporulation of *a Bacillus sp.* strain may be induced using conventional techniques known in the art. For example, spores may be obtained by pre-culturing the strain and then inducing sporulation in media such as DSM (Difco Sporulation Medium), NB (Nutrient broth), SYP (Starch Yeast extract Peptone medium), and LB2 (Luria-Bertani 2) during main culture of the pre-cultured strain, and may be obtained by culturing various sources, which include natural, variant, or recombinant hosts, removing vegetative cells therefrom, and then isolating the spores through filtration, concentration, centrifugation, or the like.

Meanwhile, a superoxide dismutase (SOD) is an enzyme that alternately catalyzes dismutation of superoxide (O₂ ⁻) radical into either ordinary molecular oxygen (O₂) or hydrogen peroxide (H₂O₂). An SOD plays a key role in decreasing oxidative stress by removing reactive oxygen species. SODs are widely distributed in prokaryotic and eukaryotic cells and are classified into four classes based on their different types of metal centers (copper/zinc, nickel, manganese, and iron). Manganese-containing SODs (Mn-SODs) are widely present in many bacteria, or chloroplasts, mitochondria, and cytosol of eukaryotic cells. In the present disclosure, the term "SOD" may be used interchangeably with a (poly)peptide having superoxide dismutase activity. In addition, the SOD may include a polypeptide having superoxide dismutase activity or a fragment thereof, or a fusion containing the same.

In some embodiments, the SOD may bind manganese (Mn-SOD). Preferably, the SOD may be a deamidated Mn-SOD. In addition, the SOD may comprise or consist of the amino acid sequence represented by SEQ ID NO: 2 (SodA). Preferably, the SOD may be one in which amino acid residues 74 and 137 have been substituted with Asp based on SEQ ID NO: 2. More preferably, the SOD may comprise or consist of the amino acid sequence represented by SEQ ID NO: 4 (SodA2). In some embodiments, the SOD may be in a form in which the start codon methionine (Met) has been removed during protein translation. Preferably, the SOD may be a deamidated Mn-SOD in a form in which methionine (Met) has been removed. The SOD may be one in which the amino acid residue Met at position 1 has been deleted based on SEQ ID NO: 2. Preferably, the SOD may comprise or consist of the amino acid sequence represented by SEQ ID NO: 5 (Met-deleted SodA). In addition, the SOD may be one in which the amino acid residue Met at position 1 has been deleted based on SEQ ID NO: 4. More preferably, the SOD may comprise or consist of the amino acid sequence represented by SEQ ID NO: 6 (Met-deleted SodA2).

The SOD or polypeptide having SOD activity of the present disclosure is interpreted to include amino acid sequences showing substantial identity to the above-mentioned amino acid sequences. The substantial identity means that amino acid sequences show sequence identity of 80% or higher, preferably 90% or higher, more preferably 95% or higher, and most preferably 98% or higher in a case where the aligned sequences are analyzed using an algorithm commonly used in the art.

In some embodiments, the SOD may be a modified or engineered polypeptide having SOD enzymatic activity, wherein the polypeptide comprises at least one mutation, for example, deletion, insertion, or substitution of one or more amino acids, which may or may not affect various aspects (in vivo, in vitro, or ex vivo stability, homogeneity, and/or conformational change). In addition, the polypeptide may further comprise a heterologous substance (for example, a tag known in the art including HIS tag, HA tag, and myc tag, GFC, and/or Fc domain of an antibody) for purification, detection, in vivo delivery, or increased stability.

In some embodiments, the SOD of the present disclosure may be derived from a variety of sources, including natural, variant, or recombinant microorganisms. For example, the SOD may be derived from bacteria. Preferably, the SOD may be derived from bacteria that are generally regarded as safe (GRAS) for use in drugs or foods, such as *Bacillus sp.* Strains, or variants or recombinants thereof. Specific examples of the *Bacillus sp.* strains are as described above. More preferably, the SOD may be obtained from a *Bacillus velezensis* strain (for example, GF423 strain, GF424 strain, or GF427 strain) or a culture supernatant thereof. The *Bacillus velezensis* GF424 strain is a strain obtained by subjecting the *Bacillus velezensis* GF423 strain to mutagenesis through UV irradiation to enhance expression of the *sod* gene. The *Bacillus velezensis* GF427 strain is a strain obtained by replacing the promoter sequence of the *Bacillus velezensis* GF423 strain with a nucleotide sequence having stronger promoter activity to enhance expression of the *sod* gene. The SOD enzyme (SodA) derived from *Bacillus velezensis* GF423, GF424, or GF427 strain is an Mn-SOD and may comprise or consist of the amino acid sequence of SEQ ID NO: 2 (the nucleotide sequence of which is represented by SEQ ID NO: 1). In addition, the SOD may be a recombinant polypeptide. For example, the SOD may be a deamidated SOD (SodA2), in which amino acid residues at positions 74 and 137 have been substituted with Asp based on SEQ ID NO: 2, and may comprise or consist of the amino acid sequence of SEQ ID NO: 4 (the nucleotide sequence of which is represented by SEQ ID NO: 3). In addition, the SOD may be a polypeptide in which the start codon methionine (Met) has been removed. For example, the SOD may be an SOD, in which the amino acid residue at position 1 has been deleted based on SEQ ID NO: 2 or 4, and may comprise or consist of the amino acid sequence of SEQ ID NO: 5 or 6. The sequences of SEQ ID NOS: 1 to 6 are as shown in Table 2.

**[Table 2]**

| Description | Sequence | SEQ ID NO |
|---|---|---|
| Nucleotide sequence of SodA | ATGGCTTACAAACTTCCAGAATTGCCTTACGCTTATGATGCTTTA GAACCTCATATCGATAAGGAAACGATGACGATTCACCATACGAA GCACCATAACACATACGTGACAAACCTCAACAAAGCGATCGAA GGATCTGCGCTTGCAGAGAAATCTGTAGATGAGCTTGTTGCTG ATTTGAACGCAGTGCCGGAGGACATCCGCACGGCAGTCCGCAA CAATGGCGGCGGACATGCAAACCACTCTTTATTCTGGACTCTTT TATCTCCGAACGGCGGAGGCGAACCGACTGGTGAGCTTGCTGA AGAGATCAAAAGCACGTTCGGAAGCTTCGATCAATTTAAAGAA AAATTCGCCGCAGCAGCTGCAGGCCGTTTCGGTTCAGGCTGGG CTTGGCTCGTTGTAAACAACGGCAAACTTGAAATTACAAGCAC GCCAAACCAAGATTCACCGCTTTCAGAAGGTAAAACACCTGTT CTCGGTCTTGATGTTTGGGAGCATGCGTACTACCTGAACTACCA AAACCGCCGTCCTGATTACATTTCAGCTTTCTGGAATGTTGTGA ACTGGGATGAAGTTGCCCGTCTTTACAGCGAAGCAAAATAA | SEQ ID NO: 1 |
| Amino acid sequence of SodA | MAYKLPELPYAYDALEPHIDKETMTIHHTKHHNTYVTNLNKAIEG SALAEKSVDELVADLNAVPEDIRTAVRNNGGGHANHSLFWTLLSP NGGGEPTGELAEEIKSTFGSFDQFKEKFAAAAAGRFGSGWAWLV VNNGKLEITSTPNQDSPLSEGKTPVLGLDVWEHAYYLNYQNRRP DYISAFWNVVNWDEVARLYSEAK | SEQ ID NO: 2 |
| Nucleotide sequence of SodA2 | ATGGCTTACAAACTTCCAGAATTGCCTTACGCTTATGATGCTTTA GAACCTCATATCGATAAGGAAACGATGACGATTCACCATACGAA GCACCATAACACATACGTGACAAACCTCAACAAAGCGATCGAA GGATCTGCGCTTGCAGAGAAATCTGTAGATGAGCTTGTTGCTG ATTTGAACGCAGTGCCGGAGGACATCCGCACGGCAGTCCGCAA CGATGGCGGCGGACATGCAAACCACTCTTTATTCTGGACTCTTT TATCTCCGAACGGCGGAGGCGAACCGACTGGTGAGCTTGCTGA AGAGATCAAAAGCACGTTCGGAAGCTTCGATCAATTTAAAGAA AAATTCGCCGCAGCAGCTGCAGGCCGTTTCGGTTCAGGCTGGG CTTGGCTCGTTGTAAACGACGGCAAACTTGAAATTACAAGCAC GCCAAACCAAGATTCACCGCTTTCAGAAGGTAAAACACCTGTT CTCGGTCTTGATGTTTGGGAGCATGCGTACTACCTGAACTACCA AAACCGCCGTCCTGATTACATTTCAGCTTTCTGGAATGTTGTGA ACTGGGATGAAGTTGCCCGTCTTTACAGCGAAGCAAAATAA | SEQ ID NO: 3 |
| Amino acid sequence of SodA2 | MAYKLPELPYAYDALEPHIDKETMTIHHTKHHNTYVTNLNKAIEG SALAEKSVDELVADLNAVPEDIRTAVRNDGGGHANHSLFWTLLSP NGGGEPTGELAEEIKSTFGSFDQFKEKFAAAAAGRFGSGWAWLV VNDGKLEITSTPNQDSPLSEGKTPVLGLDVWEHAYYLNYQNRRP DYISAFWNVVNWDEVARLYSEAK | SEQ ID NO: 4 |
| Amino acid sequence of Met-deleted SodA | AYKLPELPYAYDALEPHIDKETMTIHHTKHHNTYVTNLNKAIEGS ALAEKSVDELVADLNAVPEDIRTAVRNNGGGHANHSLFWTLLSPN GGGEPTGELAEEIKSTFGSFDQFKEKFAAAAAGRFGSGWAWLVV NNGKLEITSTPNQDSPLSEGKTPVLGLDVWEHAYYLNYQNRRPD YISAFWNVVNWDEVARLYSEAK | SEQ ID NO: 5 |
| Amino acid sequence of Met-deleted SodA2 | AYKLPELPYAYDALEPHIDKETMTIHHTKHHNTYVTNLNKAIEGS ALAEKSVDELVADLNAVPEDIRTAVRNDGGGHANHSLFWTLLSPN GGGEPTGELAEEIKSTFGSFDQFKEKFAAAAAGRFGSGWAWLVV NDGKLEITSTPNQDSPLSEGKTPVLGLDVWEHAYYLNYQNRRPD YISAFWNVVNWDEVARLYSEAK | SEQ ID NO: 6 |

In addition, the SOD may be derived from other recombinant strains (for example, *Bacillus subtilis sp.* strain) comprising the SOD-expressing gene of the *Bacillus velezensis* strain. The recombinant strain may be produced by a recombinant technique using a conventional protein-producing strain known in the art. For example, the *Bacillus subtilis* strain (which is a parent strain of the recombinant strain) may be KCTC 3135, and the KCTC 3135 strain may be obtained from the Korean Collection for Type Cultures (KCTC), Biological Resource Center, Korea Research Institute of Bioscience and Biotechnology. In addition, in the recombinant strain, one or more of the genes shown in Table 3 may be removed to facilitate subsequent processing.

**[Table 3]**

| Gene name | Protein name | Function |
|---|---|---|
| *AprE* | Serine alkaline protease (subtilisin E) | Extracellular protease |
| *NprE* | Extracellular neutral metalloprotease | Extracellular protease |
| *Bpr* | Bacillopeptidase F | Extracellular protease |
| *Epr* | Extracellular serine protease | Extracellular protease |
| *NprB* | Extracellular neutral protease B | Extracellular protease |
| *Vpr* | Extracellular serine protease | Extracellular protease |
| *Mpr* | Extracellular metalloprotease | Extracellular protease |
| *IspA* | Intracellular serine protease | Intracellular protease |
| *SrfAC* | Surfactin synthase | Surfactin synthesis |
| *spoIIAC* | RNA polymerase sporulation-specific sigma factor (sigma-F) | Sporulation |
| *EpsE* | Putative glycosyltransferase | Extracellular polysaccharide |
| *Xpf* | RNA polymerase sigma factor | Transcription of PBSX prophage gene |

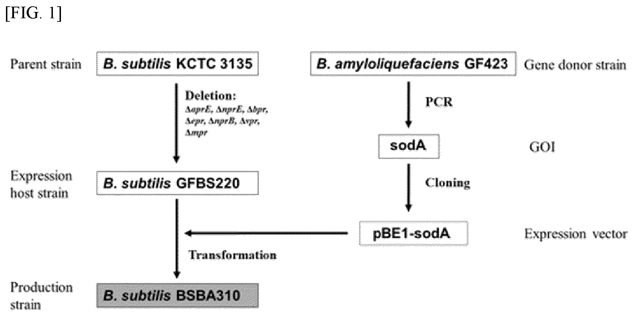

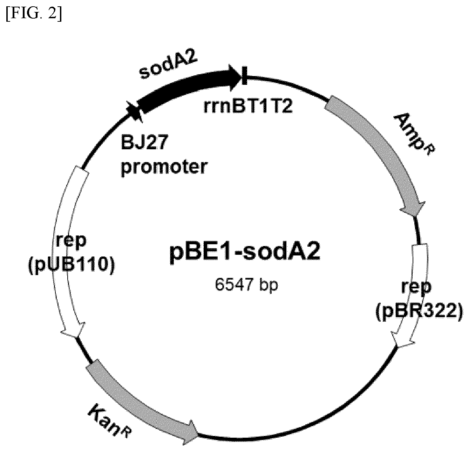

For example, the recombinant strain may be produced through the process shown in FIG. 1. In addition, the recombinant strain may comprise the expression vector shown in FIG. 2. In the drawing, sodA and sodA2 represent SOD-coding genes. From the viewpoint that the SOD derived from the above-mentioned strain is an enzyme secreted outside of a cell, in a case where the SOD is produced using the strain, it is possible to mass-produce an SOD that is safe for individuals without going through an expensive purification process (for example, column purification), which enables efficient production.

In some embodiments, the SOD may be obtained by culturing the natural, variant, or recombinant microorganism in various culture media. For example, the SOD may be isolated from a culture supernatant of the *Bacillus velezensis* GF423, GF424, or GF427 strain. Specifically, the *Bacillus Bacillus velezensis* strain may be first cultured in various types of media to obtain a culture. For example, the strain is grown at about 25°C to about 42°C for about 1 to about 4 days using a complex medium (pH 6.0 to 7.0). Other suitable media for culturing the *Bacillus velezensis* strain include Luria-Bertani (LB) medium, International Streptomyces Project (ISP) medium, nutrient agar (NA) medium, brain heart infusion agar (BHI) medium, sabouraud dextrose agar (SDA) medium, potato dextrose agar (PDA) medium, nutrient broth (NB) medium, and the like. In a preferred embodiment, LB medium, ISP medium, BHI medium, SDA medium, or NB medium may be used. In addition, the SOD may be obtained from other natural, variant, or recombinant hosts using information provided in databases such as PubMed or BRENDA (www.brenda-enzymes.org).

In some embodiments, the SOD may be isolated or purified from a culture of a natural, variant, or recombinant strain. Here, the isolated or purified SOD, or a biologically active portion thereof is substantially free of cellular material or other contaminating proteins from the cell or tissue source from which it is derived. For example, the purified product may be separated purely from a strain culture through ultrafiltration, ammonium sulfate treatment, column purification, concentration, or the like, or may be a culture concentrate obtained therefrom through ultrafiltration, concentration, or the like. The phrase "substantially free of cellular material" includes preparations of a protein, in which the protein is separated from cellular components of the cell from which the protein is isolated or recombinantly produced. In some embodiments, the phrase "substantially free of cellular material" includes preparations of a protein, having less than about 30%, preferably less than about 20%, more preferably less than about 10%, and most preferably less than about 5% by dry weight of unwanted protein.

The SOD may be preferably purified by the following purification method, but the method is not limited thereto. For example, the culture previously obtained by culturing the *Bacillus velezensis* strain is centrifuged to collect a culture supernatant. The supernatant fraction is pretreated by solid-phase extraction, and then isolated and purified by chromatography. Various modes of chromatography may be used to purify an SOD. Preferably, hydrophobic interaction chromatography is used.

In some embodiments, the SOD may be included as a strain lysate, strain culture, strain culture concentrate, or strain culture extract, or a dried form thereof. Here, the "strain lysate" refers to a product obtained by culturing the strain and disrupting it mechanically or chemically, and may include any product obtained therefrom through additional processes such as extraction, dilution, concentration, and purification. The "strain culture" may refer to a culture itself obtained by culturing the strain or a supernatant thereof. The "strain culture concentrate" refers to a product that is separated purely from the strain culture through ultrafiltration, ammonium sulfate treatment, column purification, concentration, or the like, or a culture concentrate obtained therefrom through ultrafiltration, concentration, or the like. The "strain culture extract" refers to a product that is extracted from the culture or a concentrate thereof, and may include an extract, a diluted or concentrated liquid of the extract, a dried product obtained by drying the extract, or a crude or purified product thereof, or a fraction obtained by fractionating the same. The dried form may include a lyophilized form.

In some embodiments, the SOD may comprise a cellular material from the cell or tissue source from which it is derived, such as extracellular vesicle. In this case, the cellular material containing an SOD may be obtained by culturing various sources, including natural, variant, or recombinant hosts, using conventional techniques known in the art as described above, and isolating the cellular material from the culture using methods such as filtration and concentration.

### Prophylactic or therapeutic method for fibrotic disease

According to still yet another aspect of the present disclosure, there is provided a method for preventing, ameliorating, or treating a fibrotic disease, comprising administering, to a subject, a composition that comprises at least one selected from the group consisting of a *Bacillus sp.* strain, a *Bacillus sp.* strain spore, and an SOD as disclosed herein. For example, the method may comprise administering, to a subject who has developed or is likely to develop a fibrotic disease, a therapeutically or nutritionally effective amount of a composition that comprises at least one selected from the group consisting of a *Bacillus sp.* strain, a *Bacillus sp.* strain spore, and an SOD as disclosed herein. The fibrotic disease is as described herein.

In some embodiments, the method may comprise administering, to the subject, *a Bacillus sp.* strain, a *Bacillus sp.* strain spore, or an SOD alone as disclosed herein. In another embodiment, the method may comprise administering, to the subject, a combination of one or more selected from the group consisting of a *Bacillus sp.* strain, a *Bacillus sp.* strain spore, and an SOD as disclosed herein. The administration may be performed orally or parenterally, with oral administration preferred. In an embodiment of the present disclosure, it was found that a fibrotic disease could be effectively prevented or treated even in a case where the *Bacillus sp.* strain, the *Bacillus sp.* strain spore, or the SOD is orally administered to the subject.

An effective amount or effective non-toxic amount of the composition according to the present disclosure may be determined by conventional experimentation. For example, a therapeutically active amount of the composition, which comprises a *Bacillus sp.* strain, a *Bacillus sp.* strain spore, and/or an SOD of the present disclosure, may vary depending on factors such as disease stage, disease severity, the subject's age, gender, medical comorbidities, body weight, and the SOD-expressing ability of the *Bacillus sp.* strain spore that elicits a desired response in the subject. Dosage and dosing regimen of the composition of the present disclosure may be adjusted to provide an optimal therapeutic response. For example, several divided doses may be administered daily, weekly, every two weeks, every three weeks, every four weeks and the like, and/or the dose may be proportionally decreased or increased depending on exigencies of therapeutic situation.

As an example, the *Bacillus sp.* strain or the *Bacillus sp.* strain spore may be included in the composition at 10⁴ to 10¹², 10⁴ to 10¹⁰, 10⁴ to 10⁹, 10⁵ to 10¹², 10⁵ to 10¹¹, 10⁵ to 10¹⁰, or 10⁵ to 10⁹ cfu.

The composition according to the present disclosure may be administered together with one or more other agents for prevention, amelioration, or treatment of a fibrotic disease in a case where the one or more other agents cause or are likely to cause a fibrotic disease. The other agents are drugs administered to improve or ameliorate other diseases, such as antibiotics, immunosuppressants, drugs for cardiac diseases, and anticancer chemotherapeutic agents, and include, but are not limited to, any agent that is likely to unintentionally induce a fibrotic disease. In a case where a composition comprising at least one selected from the group consisting of a *Bacillus sp.* strain, a *Bacillus sp.* strain spore, and/or an SOD of the present disclosure is administered together with another agent, they may be administered simultaneously, sequentially, or in reverse order, and the composition may be administered from before administration of the other agent. The respective components may be administered to a subject in such a manner that one component is administered at a different time from when the other component is administered. In certain embodiments, each administration may be given non-simultaneously (for example, separately or sequentially) at multiple intervals over a given period of time. In addition, the individual components may be administered to the subject by the same or different routes. The routes by which the components may be administered are described in detail in the pharmaceutical composition.

In an embodiment, the *Bacillus sp.* strain, the *Bacillus sp.* strain spore, and/or the polypeptide having SOD activity of the present disclosure may be administered simultaneously, sequentially, or in reverse order. That is, a composition comprising one of the following configurations (i) to (vii) may be administered simultaneously, sequentially, or in reverse order to exhibit a prophylactic or therapeutic effect on a fibrotic disease: (i) *Bacillus sp.* strain and *Bacillus sp.* strain spore, (ii) *Bacillus sp.* strain and SOD, (iii) *Bacillus sp.* strain spore and SOD or (iv) *Bacillus sp.* strain, *Bacillus sp.* strain spore, and SOD. Preferably, the *Bacillus sp.* strain spore and the SOD may be administered simultaneously, sequentially or in reverse order to exhibit a prophylactic or therapeutic effect on a fibrotic disease.

### Pharmaceutical composition

According to still yet another aspect of the present disclosure, there is provided a pharmaceutical or veterinary composition comprising the composition as described above. In other words, there is provided a pharmaceutical or veterinary composition comprising, as an active ingredient, at least one selected from the group consisting of the *Bacillus sp.* strain, the *Bacillus sp.* strain spore, and the SOD. The pharmaceutical composition of the present disclosure may be used interchangeably with the term "veterinary composition" in a case of being applied to an animal other than a human.

In some embodiments, the pharmaceutical composition may be used for prevention or treatment of a fibrotic disease. The details on the fibrotic disease are as described above.

The pharmaceutical or veterinary composition of the present disclosure may further comprise at least one selected from the group consisting of pharmaceutically acceptable carriers, excipients, and diluents. The pharmaceutically acceptable carrier, excipient, and/or diluent may be those commonly used in the art. The carrier, excipient, or diluent may include for example, but not limited thereto, lactose, dextrose, sucrose, sorbitol, mannitol, xylitol, erythritol, maltitol, starch, gum acacia, alginate, gelatin, calcium phosphate, calcium silicate, cellulose, methyl cellulose, hydroxypropyl methyl cellulose, microcrystalline cellulose, polyvinyl pyrrolidone, water, methyl hydroxybenzoate, propyl hydroxybenzoate, propyl hydroxybenzoate, talc, magnesium stearate, silicon dioxide, and mineral oil.

In a case of performing preparation, the preparation may be achieved by using an additive such as a filler, an extender, a binder, a wetting agent, a disintegrant, or a surfactant. The additive for the preparation may be appropriately selected from those commonly used in the pharmaceutical field.

In addition, the pharmaceutical or veterinary composition of the present disclosure may be formulated into a desired form depending on the method of use, and in particular, the formulation may be achieved by employing a method known in the art to provide rapid, sustained, or delayed release of the active ingredient after being administered to a mammal. Specific examples of such a formulation include tablet, pill, powder, granule, syrup, solution, capsule, suspension, emulsion, injection solution, plaster, lotion, liniment, lemonade, aerosol, extract, elixir, ointment, fluid extract, needle, cream, soft or hard gelatin capsule, patch, and the like.

Furthermore, the pharmaceutical or veterinary composition of the present disclosure may preferably be formulated using an appropriate method known in the art or a method disclosed in Remington's Pharmaceutical Science (latest edition), Mack Publishing Company, Easton PA.

The pharmaceutical or veterinary composition of the present disclosure may be administered orally or parenterally depending on the intended method. The parenteral administration may include, but is not limited to, intravenous, subcutaneous, intraperitoneal, intrapulmonary, intraarterial, intramuscular, rectal, intravaginal, intraarticular, intraprostatic, intranasal, intraocular, intravesical, intrathecal, or intraventricular administration (for example, intracerebroventricular administration).

Solid preparations for oral administration include tablets, pills, powders, granules, capsules, and the like, and these solid preparations may be prepared by mixing the composition with at least one excipient such as starch, calcium carbonate, sucrose, lactose, and gelatin. Further, in addition to simple excipients, a lubricant such as magnesium stearate and talc may also be used. Examples of liquid preparations for oral administration include suspensions, solutions, emulsions, and syrups. In addition to commonly used simple diluents such as water and liquid paraffin, various types of excipients, such as a wetting agent, a sweetener, a fragrance, and a preservative, may be used.

For oral administration, the SOD may be coated with shellac for protection from gastric acid, but the coating agent is not limited thereto. Examples of the coating agent suitable for use in the present disclosure include shellac, ethyl cellulose, hydroxypropyl methylcellulose, hydroxypropyl methylcellulose phthalate, zein, Eudragit, and combinations thereof. In a case where the SOD is coated, the SOD may be coated in solution. Specifically, a purified solution and a shellac-containing solution are mixed and then lyophilized. This lyophilized sample may be made into powder and stored at about 4°C until use. In some embodiments, the SOD may be an oral SOD with increased stability against gastric acid through a shellac coating. Preferably, the SOD may be a shellac-coated SOD in which the amino acid residue Met at position 1 has been deleted based on SEQ ID NO: 4. More preferably, the SOD may be a shellac-coated SOD comprising or consisting of the amino acid sequence represented by SEQ ID NO: 6.

The pharmaceutical or veterinary composition of the present disclosure is administered in a pharmaceutically or veterinary effective amount. The term "pharmaceutically effective amount" or "veterinary effective amount" refers to an amount sufficient to treat a disease at a reasonable benefit/risk ratio applicable to any medical or veterinary treatment. A level of the effective amount may be determined depending on factors including the patient's or animal's body weight, gender, age, health status, severity of disease, activity of the drug, sensitivity to the drug, administration time, administration route and excretion rate, duration of treatment, and drugs used concurrently therewith, and other factors well known in the medical field.

The pharmaceutical or veterinary composition of the present disclosure may be administered as an individual therapeutic agent or in combination with other therapeutic agents, in which the administration may be carried out in a sequential or simultaneous manner. In addition, the pharmaceutical composition may be administered once or multiple times as needed. Taking all of the above factors into consideration, it is important to administer a minimum amount that allows the maximum effect to be obtained with no adverse effects, and such an amount may be easily determined by those skilled in the art.

In some embodiments, the composition can have various forms applicable to human administration known in the art, including a liquid form, a solid form, a gel form, a powder form, a paste form, and the like.

### Food or feed composition

According to still yet another aspect of the present disclosure, there is provided a food composition, comprising a composition that comprises at least one selected from the group consisting of the *Bacillus sp.* strain, the *Bacillus sp.* strain spore, and the SOD as described above. Such a food composition includes a medical or nutraceutical food composition. Therefore, the food composition provides a food composition for preventing or ameliorating a fibrotic disease.

The term "medical food" or "nutraceutical food" refers to a food manufactured from raw materials or ingredients that are likely to have beneficial functions in the human body, the food maintaining normal function or activating physiological functions of the human body to maintain or improve health. This food is defined by the Ministry of Food and Drug Safety, but is not limited thereto, and does not exclude any common health food from its meaning.

In addition, the food includes, but is not limited to, various foods, food additives, beverages (for example, functional drinks, natural fruit juices, and vegetable drinks), gum, tea, vitamin complexes, health functional foods, other functional foods, and the like.

The food may be manufactured by conventional methods known in the art. For example, the medical food, nutraceutical food, or health functional food may be formulated into one selected from the group consisting of tablet, pill, powder, granule, capsule, and liquid formulation by further comprising at least one of a carrier, a diluent, an excipient, and an additive, in addition to the SOD, for the purpose of preventing or ameliorating a fibrotic disease. Specific examples of the carrier, excipient, diluent, and additive are well known in the art, and those skilled in the art are capable of combining appropriate ingredients depending on the formulation to manufacture the food.

An amount of the *Bacillus sp.* strain, the strain spore, and/or the SOD according to the present disclosure, which are contained as an active ingredient(s) in the above-described formulation, may be adjusted appropriately depending on the form and purpose of use, the patient's condition, type and severity of symptoms, and the like. The amount may be, but is not limited to, 0.001 to 99.9% by weight, and preferably 0.01 to 50% by weight, based on the weight of solid content.

A dose of the food of the present disclosure may vary depending on the patient's age, body weight, gender, dosage form, health status, and severity of disease, and the administration may be done once a day or in divided doses several times a day at regular time intervals depending on the judgment of a doctor or pharmacist. For example, the daily dosage may be 10 to 1,000 mg/kg based on the active ingredient content. The dosage is an example of an average case and may increase or decrease depending on individual differences. In a case where the daily dosage of the health functional food of the present disclosure is less than the above-mentioned dosage, it is not possible to obtain significant effects. In a case where the daily dosage exceeds the above-mentioned dosage, it is not only uneconomical but also undesirable side effects may occur because such a dosage is outside the usual dosage range.

According to still yet another aspect of the present disclosure, there is provided a feed composition, comprising at least one selected from the group consisting of the *Bacillus sp.* strain, the *Bacillus sp.* strain spore, and the SOD as described above. In addition, the feed composition of the present disclosure may be prepared in any formulation commonly used in the art. For example, the feed composition of the present disclosure may further comprise an auxiliary ingredient such as amino acids, inorganic salts, vitamins, antibiotics, antibacterial substances, antioxidants, antifungal enzymes, and other microbial agents in the form of live bacteria; grain such as ground or broken wheat, oats, barley, corn, and rice; vegetable protein feed such as those comprising rapeseed, soybean, and sunflower as main ingredients; animal protein feed such as blood meal, meat meal, bone meal, and fish meal; a dry ingredient consisting of sugar and a dairy product such as various powdered milk and whey powder; lipid such as animal fat and vegetable fat, optionally liquefied by heating; and an additive such as nutritional supplements, digestion and absorption enhancers, growth promoters, and disease prevention agents.

The feed composition of the present disclosure may be in a form of a powder or liquid preparation, and may comprise an excipient that is added to feed (calcium carbonate, wheat shorts, zeolite, corn meal, rice bran, or the like).

Hereinafter, the present disclosure will be described in more detail by the following examples. The following examples are presented to aid in understanding the present disclosure and are not intended to, and should not be construed as limiting the scope thereof in any way.

### Examples

### Preparation Example 1. Preparation of SOD-expressing Bacillus velezensis strain spores

The spores from the SOD-expressing *Bacillus velezensis (Bacillus amyloliquefaciens*) strains used in the present examples were prepared, according to the following method, from the *Bacillus velezensis* strain deposited at the Korean Collection for Type Cultures (KCTC) (Accession No.: KCTC 13227BP, Accession Date: March 13, 2017) (hereinafter referred to as "GF424 strain"), the *Bacillus velezensis* strain deposited at KCTC (Accession No.: KCTC 13222BP, Accession Date: March 6, 2017) (hereinafter referred to as "GF423 strain"), and the *Bacillus velezensis* strain deposited at KCTC (Accession No.: KCTC 15552BP, Accession Date: August 14, 2023) (hereinafter referred to as "GF427 strain").

A single colony of each *Bacillus velezensis* strain was inoculated into 1 mL of LB in a 14 mL tube and cultured at 37°C and 200 rpm for 12 hours. 1 mL of the culture was transferred to 50 mL of LB medium in a 500 mL flask and cultured at 37°C and 200 rpm for 12 hours. Then, 20 mL of the culture medium was transferred to 1 L of SYP or DSM in a 2.5 L baffled flask. The inoculated culture was cultured at 37°C and 200 rpm for 24 to 120 hours.

The SYP medium contains 1.5% soy tone, 0.5% yeast extract, 0.5% K₂HPO₄, 0.1% MnSO₄, 0.1% MgSO₄, 10 mM FeSO₄, 0.04% (NH₄)₂SO₄, 0.04% (NH₄)₂PO₄, 0.1% CaCl₂, and 2% glucose, and the DSM medium contains 8 g/L bacto nutrient broth, 1 g/L KCl, 0.25 g/L MgSO₄, 0.16415 g/L Ca(NO₃)₂, 0.9521 mg/L MnCl₂, and 0.152 mg FeSO₄. The MnSO₄, MgSO₄, FeSO₄, (NH₄)₂SO₄, (NH₄)₂PO₄, and CaCl₂ added to the medium were dissolved in ddH₂O before use, and then added.

After culture, lysozyme (0.5 g/L) was added to the culture broth, and cultured at 37°C and 200 rpm for 1 hour to remove the remaining vegetative cells. Crude spores were collected by centrifugation at 6000 rpm for 10 minutes. The collected crude spores were purified by being washed twice with water, washed with 0.02% SDS, washed again twice with water, and then suspended in PBS solution. The spore suspension was stored at -20°C. The number of spores was determined by spreading the diluted spore solution on an LB agar plate and then counting colonies.

The spores were dissolved in phosphate-buffered saline (PBS) depending on the experimental conditions and prepared in a volume of 100 µl.

### Preparation Example 2. Preparation of GF427 strain

The SOD-expressing *Bacillus velezensis* GF427 strain was prepared from the GF423 strain, according to the following method, by replacing the promoter sequence of the GF423 sodA gene with a nucleotide sequence having stronger promoter activity to increase SOD activity.

The promoter sequence of the original GF423 strain and the promoter sequence of the variant GF427 strain are as follows: GF423 promoter sequence (5'-TTGATTACCACGCTTTCTTTTGTTACATT-3') (SEQ ID NO: 7) and GF427 promoter sequence (5'-TTGACTTTACGCTTTCTTATAGGTTATAAT-3') (SEQ ID NO: 8).

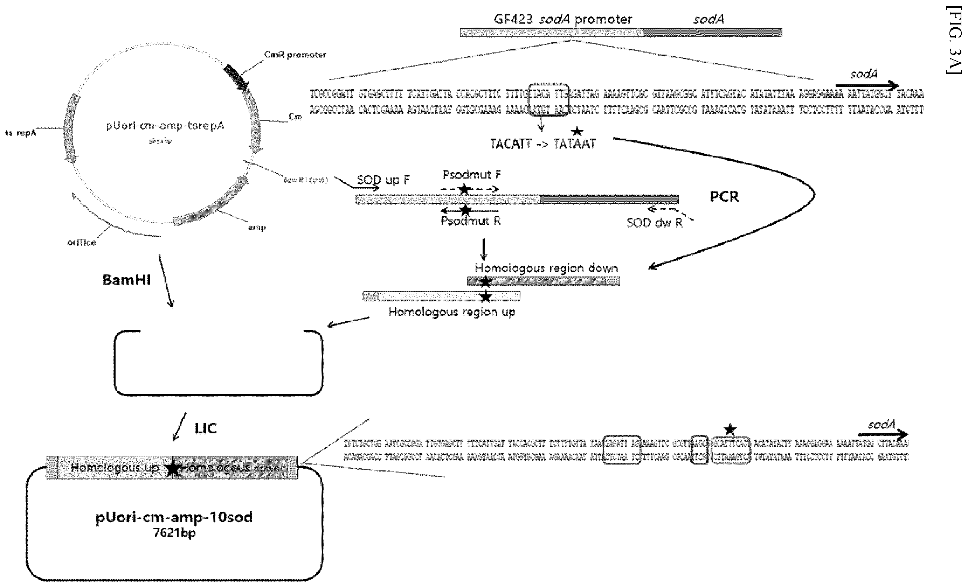

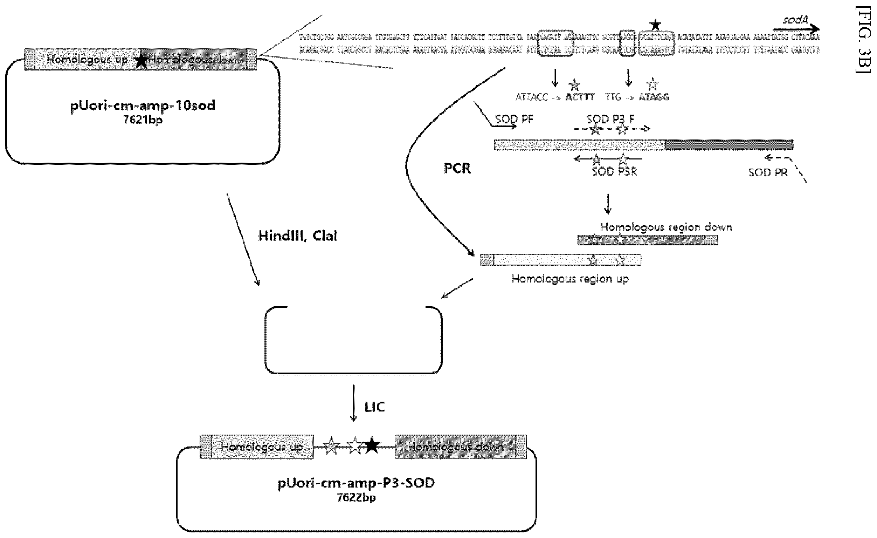

Nucleotide sequence replacement on the genome was performed by double crossover recombination (FIG. 3). PCR products were prepared by performing PCR using the GF423 genomic DNA as a template with the primers SOD up F and Psodmut R, and PCR products were prepared by performing PCR using the GF423 genomic DNA as a template with the primers Psodmut F and SOD dw R. Next, using the LIC method, the PCR products were cloned into pUori-cm-amp-tsrepA cleaved with BamHI, thereby producing pUori-cm-amp-10sod (FIG. 3A).

Then, PCR products were prepared by performing PCR using the pUori-cm-amp-10sod as a template and the primers SOD PF and SOD P3R, and PCR products wer prepared by performing PCR using the same template and the primers SOD P3F and SOD PR. Using the LIC method, the PCR products were cloned into pUori-cm-amp-10sod cleaved with HindIII and ClaI, thereby producing pUori-cm-amp-P3-SOD to be used for replacement (FIG. 3B). A vector was constructed using *E. coli* C2984H, and the nucleotide sequences of the primers used, the PCR conditions, and the composition of LIC reaction mixture are shown in Tables 4, 5, and 6, respectively.

**[Table 4]**

| Primer | Sequence (5'→3') | SEQ ID NO |
|---|---|---|
| SOD up F | tccagatcctctacgggcgctgcgtatgctggaa | 9 |
| Psodmut R | aacttttctaatctcattataacaaaagaaagcg | 10 |
| Psodmut F | cgctttcttttgttataatgagattagaaaagtt | 11 |
| SOD dw R | gagttttcgttcggatctgtcctccggcactgcg | 12 |
| SOD PF | gtaatggaataagccgaaagcttccagagctg | 13 |
| SOD P3R | attataacctataagaaagcgtaaagtcaatgaaaaagctcacaatcc | 14 |
| SOD P3F | ttgactttacgctttcttataggttataatgagattagaaaagttc | 15 |
| SOD PR | catcgtttccttatcgatatgaggttctaaag | 16 |
| Cm C F | ccgctatctttacaggtacatca | 17 |
| SOD mid R | gaaccgaaacggcctgcag | 18 |
| 424 conf F | ggcaggcattatattaggcc | 19 |
| 424 conf R | gcactgcgttcaaatcagca | 20 |
| SOD DC F2 | ggattcagcgcttcaaaatc | 21 |
| -10 SOD R | cgcgaacttttctaatctcatta | 22 |
| SOD P2-R | ctaatctcattataacctat | 23 |
| cm conf F | acctttctgatgtagagaaatataatggttcggg | 24 |
| cm conf R | cggcattatctcatattataaaagccagtcatt | 25 |

**[Table 5]**

| PCR step | Temperature | Time |
|---|---|---|
| Initial denaturation | 95°C | 30 sec |
| 30 cycles | 95°C | 30 sec |
| | 50°C | 30 sec |
| | 72°C | 1 min/kb |
| Final extension | 72°C | 10 min |
| The DNA polymerase used for PCR is *Taq* DNA polymerase (TAKARA, Japan). | | |

**[Table 6]**

| Mixture | Volume (ul) |
|---|---|
| Linearized vector | 2 |
| PCR product 1 | 1 |
| PCR product 2 | 1 |
| 10X T4 polymerase buffer | 1 |
| 10X BSA | 1 |
| T4 polymerase | 0.5 |
| DW | 3.5 (up to 10 µl) |
| Total | 10 |

The pUori-cm-amp-10sod was transformed into the GF423 strain by electroporation. The GF423 strain was inoculated into LB-Sor medium (1x LB, 0.5 M sorbitol) and cultured at 37°C and 200 rpm. When the OD reached 0.8, glycine was added thereto to a final concentration of 10 mg/ml, and then the culture was further performed for 1.5 hours. After the culture was completed, the cells were placed on ice and cooled for 15 minutes. Then, centrifugation was performed at 4000 rpm and 4°C for 10 minutes. The collected cells were washed three times with electroporation buffer (containing 0.5 M sorbitol, 0.5 M mannitol, and 10% glycerol). Then, the cells were resuspended in the same buffer to 1/50 of the culture volume and used for electroporation. 0.5 ug of DNA of the pUori-cm-amp-10sod was mixed with the prepared cells. Then, the mixture was placed in a pre-chilled 1 mm gap electroporation cuvette and incubated on ice for 3 minutes. Next, a single electric pulse was applied to the resultant using a MicroPulser Electroporation system (purchased from Bio-rad) at a field strength of 2.5 V, 25 uF, and 200 Ω (time constant = 4.8-5.8). Immediately after the pulse, 1 mL of LB-Sor medium was added thereto, and then the cells were allowed to recover at 37°C and 200 rpm for 2 hours. Then, the cells were spread on antibiotic solid medium (1x LB, cm 2.5 ug/ml, 1.5% agar) and cultured in a 37°C incubator for 48 hours. It was identified whether the resulting colonies have been transformed, using PCR with the primers cm conf F and cm conf R. Induction and identification of double crossover recombination were performed as follows. The identified colonies were added to LB medium and cultured at 37°C and 200 rpm for 24 hours. Then, the resultant was diluted 1/10⁵, spread at 100 µl on antibiotic solid medium (1x LB, agar 1.5%), and cultured in a 37°C incubator for 20 hours. The colonies obtained by the culture were subjected to PCR using the primers Cm CF and SOD mid R to obtain colonies that had undergone single crossover recombination. The obtained colonies were inoculated again into LB broth and cultured at 28°C for 20 hours. Then, the resultant was diluted 1/10⁵, spread on LB solid medium, and cultured. The identified colonies were streaked on both antibiotic solid medium and regular solid medium to identify plasmid-removed colonies. For the plasmid-removed colonies, PCR conditions were set using mutation site-containing primers, so that only the successfully engineered colony showed a band, thereby selecting successfully mutated colonies. The primers used were the pair SOD DC F2 and -10 SOD R, and the pair SOD DC F2 and SOD P2 R. The selected colony was subjected to PCR using the primers 424 conf F and 424 conf R, and the PCR product was gelpurified. Then, the sample was sequenced to check whether the replacement was accurately made, and then named 'GF427.' This strain was deposited with the Korean Collection for Type Cultures on August 14, 2023 (KCTC 15552 BP). To check whether the GF427 strain has an increased SOD expression level, its SOD expression level was compared with that of the GF424 strain that is a strain with improved SOD activity. SOD activity of the culture supernatants was measured as follows. For both strains GF424 and GF427, colonies were inoculated into 1x SYPG (1.5% soytone, 1% yeast extract, 0.5% potassium phosphate dibasic, 1% glucose) containing 50 ug/mL MnSO₄ and cultured for 20 hours. The culture was centrifuged at 12,000 rpm for 10 minutes. The culture supernatant was collected and used for measuring SOD activity. The SOD activity was measured as 22.2 U/mL for GF424 and 67.8 U/mL for GF427, which was approximately three times higher than GF424.

In addition, GF427 strain spores were prepared using the same method as in Preparation Example 1.

### Example 1. Identification of fibrosis prevention effect of SOD-overexpressing strain spores in pulmonary fibrosis animal model

### Example 1.1. Establishment of pulmonary fibrosis animal model and testing

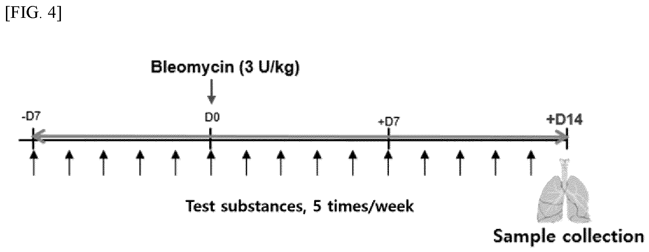

To identify whether spores from SOD-overexpressing strain (GF424 spores) have a preventive effect in a pulmonary fibrosis animal model, a pulmonary fibrosis animal model was established and experiments were conducted using the following method. 7-week-old C57BL/6 male mice were acclimated for one week. Then, the animals were observed for general conditions to determine their health status, and only the healthy animals were used for the experiments. The mice were divided into three groups: control group (CTL), bleomycin-treated group (Bleo), and test group (GF424 spore-administered group; B+Spore SOD). The test group mice were again divided into three groups, and the GF424 spores prepared in Preparation Example 1 were orally administered to the mice of the respective test groups at three concentrations (1×10⁶CFU, 1×10⁷CFU, and 1×10⁸CFU) once a day for three weeks (total of 5 times per week). On the 7th day (D0) from the day the spore administration began (-D7), bleomycin (bleomycin sulfate, purchased from Millipore) was administered intratracheally once at a dose of 3 U/kg to induce pulmonary fibrosis. The administration schedule of spores and bleomycin over a total of 21 days is shown in FIG. 4. On the other hand, the control group (CTL) was neither subj ected to induction of pulmonary fibrosis nor spore administration. For the bleomycin-treated group (Bleo), pulmonary fibrosis was induced by single intratracheal administration of bleomycin at a dose of 3 U/kg on D0 without spore administration (see FIG. 4).

The experiment was terminated on the 21st day from the initial spore administration, and the mice in each group were sacrificed.

### Example 1.2. Measurement of collagen concentration by measurement of hydroxyproline concentration

Hydroxyproline is an amino acid found almost exclusively in collagen in mammals and serves as a representative indicator that allows for direct measurement of the amount of collagen. The amount of hydroxyproline was measured through the following process. The left lung of a pulmonary fibrosis mouse model was extracted, 100 µl of sterile water was added per 10 mg of lung tissue, and then the tissue was thoroughly homogenized. To the homogenized tissue suspension was added an equal volume of hydrochloric acid (HCl, ~12N) was to the suspension. Then, the resultant was completely sealed and boiled at 120°C for 3 hours. The resulting cell suspension was centrifuged (4°C, 10,000×g, 3 minutes), and only the clean supernatant was transferred to a new tube. Measurement was performed at 560 nm using the Hydroxyproline Colorimetric Assay Kit (purchased from Biovison) and a multiplate spectrometer.

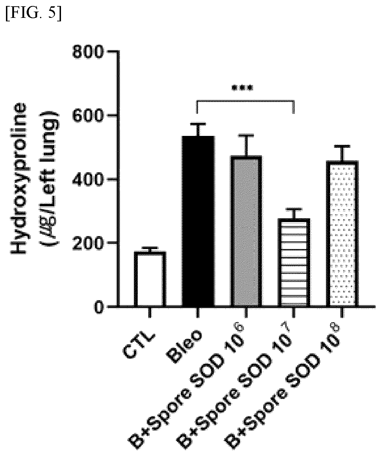

As a result, referring to FIG. 5, in the bleomycin-treated group, where pulmonary fibrosis was induced by bleomycin so that the amount of collagen was increased, hydroxyproline was measured to be higher than the control group. In contrast, it was found that in the GF424 spore-administered group, the amount of hydroxyproline (that is, the amount of collagen) was decreased at all concentrations compared to the bleomycin-treated group. In particular, the most significant decrease was observed in the group administered at a concentration of 1×10⁷ CFU.

### Example 1.3. Measurement of number of immune cells in bronchoalveolar lavage fluid

The trachea of the pulmonary fibrosis mouse model was partially incised. 1 ml of sterile PBS was instilled thereinto and then retrieved. This procedure was repeated twice. Approximately 2 ml of the collected bronchoalveolar lavage fluid (BALF) was rapidly centrifuged at 2,200 rpm for 10 minutes at 4°C. The centrifuged supernatant was placed in a new 2 ml tube, and the cell pellet was resuspended in 0.5 ml of fresh PBS. The total cell number in the cell suspension was measured using a hemocytometer, and then diluted in PBS to a concentration of 1×10⁵ cells/0.5 ml per sample. The thus prepared cells were mounted on a microscope slide using a cytocentrifuge at a speed of 600 rpm for 5 minutes. The cells on the slide were stained with Diff-Quik reagent (purchased from Sysmex), and then the numbers of macrophages, lymphocytes, and neutrophils among the total cells in the bronchoalveolar lavage fluid were measured.

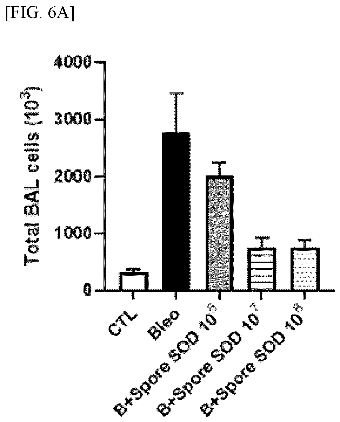

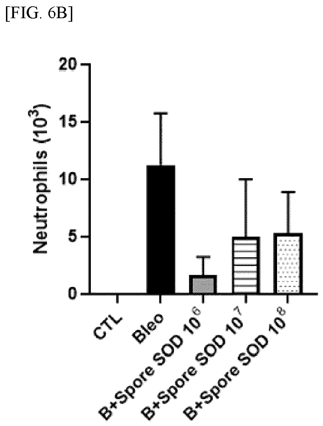

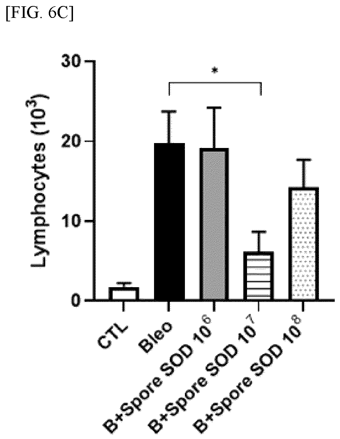

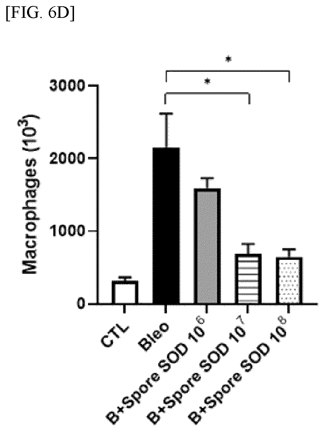

As a result, referring to FIGS. 6A to 6D, it was identified that for the bleomycin-treated group, both the toal number of the above-described three cell types (Total BAL cells) and the number of each cell type were increased compared to the control group; and for the GF424 spore-administered group, both the total cell number and the number of each cell type were decreased compared to the bleomycin-treated group. In particular, it was identified that most of the immune cells found in pulmonary fibrosis were macrophages, and the numbers of macrophages and lymphocytes were most significantly decreased in the group administered at a concentration of 1×10 7 CFU.

### Example 1.4. Measurement of TGF-β1 (transforming growth factor beta 1) concentration in bronchoalveolar lavage fluid

TGF-β1 (transforming growth factor beta 1) is a factor that plays a very important role in development of pulmonary fibrosis. When cells are stimulated by reactive oxygen or various factors, TGF-β1 is activated, which in turn induces pulmonary fibrosis through downstream mechanisms. To measure TGF-β1, the bronchoalveolar lavage fluid from Example 1.3 was centrifuged, and then the supernatant collected separately was concentrated four-fold. The TGF-β1 concentration in the concentrated bronchoalveolar lavage fluid was measured at 450 nm using a TGF-β1 ELISA kit (purchased from R&D systems) and a multiplate spectrometer.

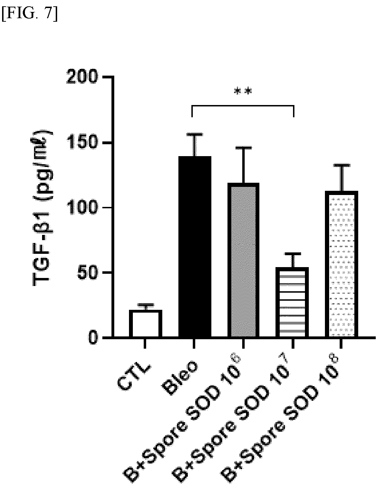

As a result, referring to FIG. 7, for the bleomycin-treated group, the amount of TGF-β1 was significantly increased due to pulmonary fibrosis compared to the control group. In contrast, for the GF424 spore-administered group, the amount of TGF-β1 was decreased at all concentrations compared to the bleomycin-treated group. In particular, the group administered at a concentration of 1×10⁷ CFU showed a significant decrease in the amount of TGF-β1.

### Example 1.5. Identification of pulmonary fibrosis through pathological tissue staining

Lung tissue extracted from the mice sacrificed in Example 1.1 was pretreated by immersing it in a 4% paraformaldehyde (PFA) solution for more than one day. Next, the lung tissue was made into a paraffin block, cut into 5 µm thick sections, and mounted on slides. The tissue sections were stained with hematoxylin and eosin (H&E) and Masson's trichrome stain and observed under a microscope to compare histopathological changes.

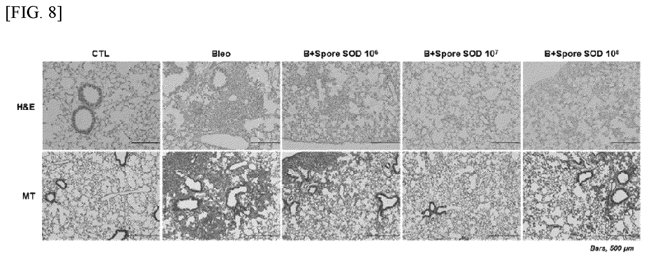

As a result of the observation, referring to FIG. 8, it was found that increased pulmonary fibrosis caused by bleomycin was effectively decreased in the GF424 spore-administered groups at all concentrations; and in particular, the best effects were observed in the group administered at a concentration of 1×10⁷ CFU.

### Example 1.6. Measurement of expression levels of pulmonary fibrosis gene markers

Expression levels of Col1a1 (a factor that increases collagen synthesis), TGF-β1 (a factor that activates myofibroblasts and promotes fibrosis), CTGF (connective tissue growth factor), and IL-6 (inflammatory cytokine), which are representative gene markers known to be activated as pulmonary fibrosis progresses, were checked using real-time polymerase chain reaction (qRT-PCR).

A portion of the right lung lobe extracted from the mice sacrificed in Example 1.1 was finely homogenized using TRIzol reagent (purchased from Thermo Fisher Scientific), and then total RNA was extracted therefrom. cDNA was synthesized from the extracted RNA using M-MLV RT reagent (purchased from Promega), and the synthesized cDNA was subjected to real-time polymerase chain reaction (qRT-PCR) according to a protocol for the Go Taq qPCR master mix (purchased from Promega). The relative mRNA expression levels were normalized using 18S rRNA as an internal control, and the values were calculated using the 2-ΔΔCq method. Information on the primers used in this experiment and the subsequent qRT-PCR experiment is summarized in Table 7.

**[Table 7]**

| **Gene name** | **Primer sequence (5'→3')** | **SEQ ID NO** |
|---|---|---|
| Col1a1 (Type 1 collage) | F: GCTCCTCTTAGGGGCCACT | 26 |
| | R: CCACGTCTCACCATTGGGG | 27 |
| α-SMA (α-Smooth Muscle Actin) | F: GCTCCTCTTAGGGGCCACT | 28 |
| | R: CCCAGACATCAGGGAGTAATG | 29 |
| TGF-β1 (Transforming growth factor beta 1) | F: AGACCACATCAGCATTGAGTG | 30 |
| | R: GGTGGCAACGAATGTAGCTGT | 31 |
| CTGF (Connective tissue growth factor) | F: GGGCCTCTTCTGCGATTTC | 32 |
| | R: ATCCAGGCAAGTGCATTGGTA | 33 |
| IL-6 (InterLeukin-6) | F: CAAAGCCAGAGTCCTTCAGAG | 34 |
| | R: GTCCTTAGCCACTCCTTCTG | 35 |
| TNF-α (Tumor necrosis factor-α) | F: CTTCTGTCTACTGAACTTCGGG | 36 |
| | R: CAGGCTTGTCACTCGAATTTTG | 37 |
| IL-1β | F: ACGGACCCCAAAAGATGAAG | 38 |
| | R: TTCTCCACAGCCACAATGAG | 39 |
| 18S rRNA | F: CAGCGTGGTCAGGATAGAAC | 40 |
| | R: CTTGATTTGAATGCAGCGGAC | 41 |

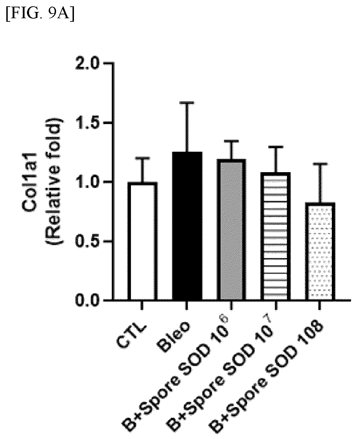

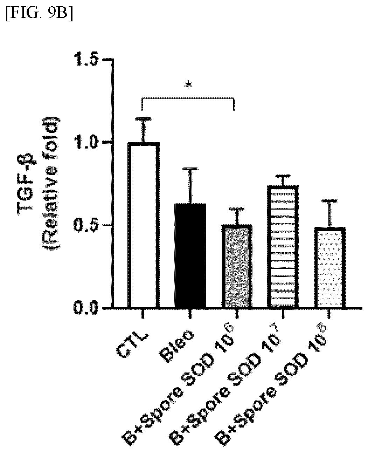

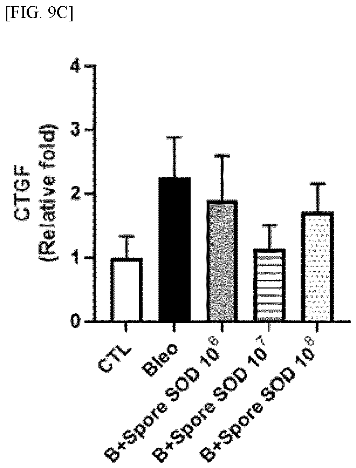

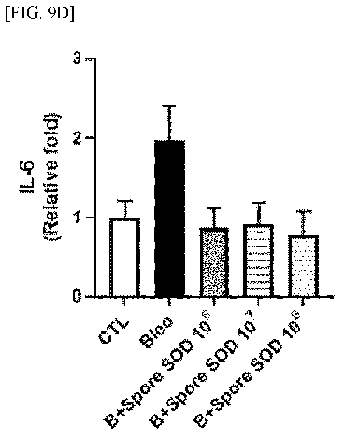

As a result, referring to FIGS. 9A to 9D, overall, the expression of all four genes tended to decrease in the GF424 spore-administered groups compared to the bleomycin-treated group.

### Example 1.7. Measurement of concentrations of reactive oxygen byproducts

Lipid peroxidation is a representative mechanism of in vivo cellular damage that occurs in both animals and plants. Concentrations of malondialdehyde (MDA) and 4-hydroxynonenal (4-HNE), which are the most common byproducts of lipid peroxidase, in lung tissue were measured as follows.

A portion of the lung tissue extracted from the mice sacrificed in Example 1.1 was finely homogenized in 1X RIPA buffer (containing protease inhibitor cocktail, purchased from Abcam). The tissue suspension was centrifuged at 4°C and 12,000 rpm for 10 minutes. Then, only the clean supernatant was separated and transferred to a new 1.5 ml tube. Protein quantification was carried out using a BCA assay kit (purchased from Thermo Fisher Scientific), and measurement was performed at 562 nm with a multiplate spectrometer. For byproduct quantification, an experiment was performed with an equal amount of the sample using the MDA ELISA kit (purchased from Cell Biolabs, Cat.# STA-832) and HNE ELISA kit (purchased from Cell Biolabs, Cat.# STA-838) according to the manufacturers' manuals, and the concentration was measured at 450 nm using a multiplate spectrometer.

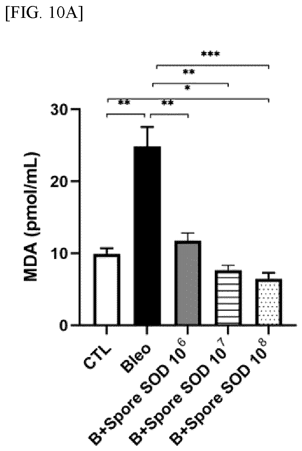

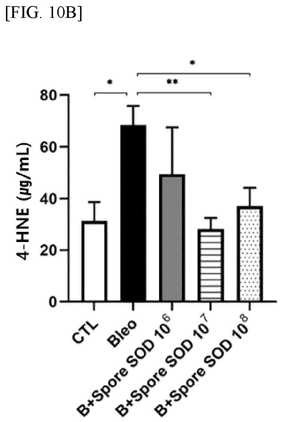

As a result, referring to FIGS. 10A and 10B, it was found that the amount of reactive oxygen byproducts (MDA and 4-HNE) present in lung tissue was statistically significantly decreased in all GF424 spore-administered groups compared to the bleomycin-treated group. Overall, a more effective decrease was observed in the group administered at a concentration of 1 × 10⁷ CFU.

### Example 2. Identification of preventive effects of spores from strains having different expression levels of SOD in pulmonary fibrosis animal model

### Example 2.1. Preparation of spores from strains having different SOD expression levels and testing

To identify whether the expression level of superoxide dismutase (SOD) directly affects preventive effects on pulmonary fibrosis, spores from three *Bacillus velezensis* strains having different expression levels of SOD were prepared. The three strains are a strain with SOD gene deleted (knock-out, denoted as '-', GF423Δ), an SOD-expressing normal strain isolated from nature (wild, denoted as '+', GF423), and a strain genetically engineered to overexpress an SOD (overexpression, denoted as '++', GF424).

The knock-out strain *Bacillus velezensis* Δ*sodA,* which lacks *sodA* function, was produced by double crossover recombination as follows. First, for homologous recombination, a pair of DNA fragments flanking the *sodA* gene were amplified by PCR from its chromosome using primers for the upstream fragment (5' aaacagctgggatgaacacaagtgagag 3' (SEQ ID NO: 42) and 5' cacactcttaagtttgcttccaattctggaagtttgtaag 3' (SEQ ID NO: 43)) and primers for the downstream fragment (5' ctactgacagcttccaaggatacctgaactaccaaaaccg 3' (SEQ ID NO: 44) and 5' aaacagctgaagctcatgaccacagcaag 3' (SEQ ID NO: 45)). The erythromycin resistance (EmR) gene was amplified by PCR from pDG1664 (see Guerout-Fleury, AM ey et al., Gene 180:57-61 (1996)) using primers (5' gaagcaaacttaagagtgtg 3' (SEQ ID NO: 46) and 5' tccttggaagctgtcagtag 3' (SEQ ID NO: 47)). The upstream fragment, the EmR gene, and the downstream fragment were sequentially assembled and cloned into the *Pvu*II site of the pUori-ts plasmid that contains a temperature-sensitive replication origin operating in *Bacillus subtilis* (see J Bacteriol 176, no. 6;1761-1763). The resulting plasmid was transformed into *Bacillus velezensis* GF423 by electroporation (see Zhang GQ et al., Anal Biochem. 409:130-137 (2011)). The *sodA* knock-out mutants were selected from transformants resistant to erythromycin (5 µg/ml) at 42°C, followed by plasmid curing at 30°C. The genomic status around the sodA region was identified by PCR and DNA sequencing of the PCR products.

The normal strain is *Bacillus velezensis* GF423 strain (Accession No.: KCTC 13222BP, Accession Date: 20170306), and the overexpression strain is *Bacillus velezensis* GF424 strain (Accession No.: KCTC 13227BP, Accession Date: 20170313). The *Bacillus velezensis* GF424 strain is a strain obtained by subjecting the *Bacillus velezensis* GF423 strain to mutagenesis through UV irradiation to improve expression of the sodA gene.

The spores prepared from the strains using the method described in Preparation Example 1 were orally administered at a concentration of 1×10⁷ CFU to the mice with bleomycin-induced pulmonary fibrosis. Spore administration and bleomycin treatment were performed in the same manner as described in Example 1.1, and the control group and the bleomycin-treated group were prepared in the same manner. The experiment was terminated on the 21st day after the initial spore administration, and the mice in each group were sacrificed (see FIG. 4). For information on *Bacillus sp.* strains and species strain spores, see Table 8.

**[Table 8]**

| Spore material name | Description | Strain name | SOD activity (relative value) |
|---|---|---|---|
| GF423 spore | Wild-type | GF423 | 1 |
| GF424 spore | SOD-overexpressing strain | GF424 | ~ 3.5-fold |
| GF427 spore | SOD-overexpressing strain | GF427 | ~ 11-fold |
| GF423Δ spore | SOD knock-out strain | GF423Δ | 0.04-fold |

### Example 2.2. Measurement of collagen concentration by measurement of hydroxyproline concentration

Measurement of hydroxyproline, which is a representative indicator that allows for direct measurement of the amount of collagen, was performed in the same manner as in Example 1.2.

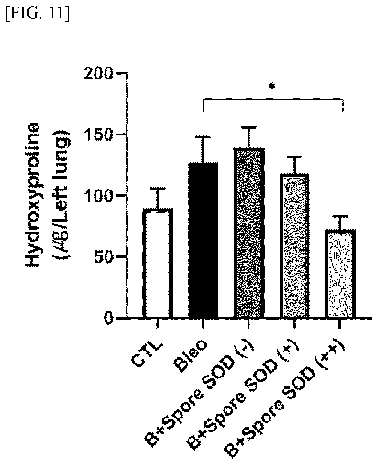

As a result of the measurement, referring to FIG. 11, in the group administered with the spores from the SOD knock-out strain (B+Spore SOD(-)), the amount of hydroxyproline was measured to be higher than that in the bleomycin-treated group. In contrast, the groups administered with the SOD-expressing normal strain spores (B+Spore SOD(+)) and the SOD-overexpressing strain spores (B+Spore SOD(++)) showed a decrease in hydroxyproline. In particular, the greatest decrease was observed in the group administered with the spores from the SOD-overexpressing strain.

### Example 2.3. Measurement of number of immune cells in bronchoalveolar lavage fluid

The method for measuring the numbers of macrophages, lymphocytes, and neutrophils among total bronchoalveolar lavage fluid cells was performed in the same manner as in Example 1.3.

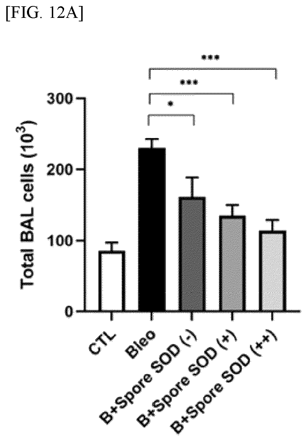

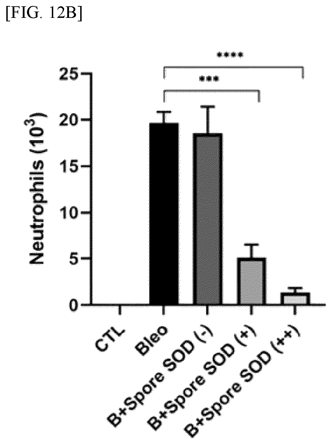

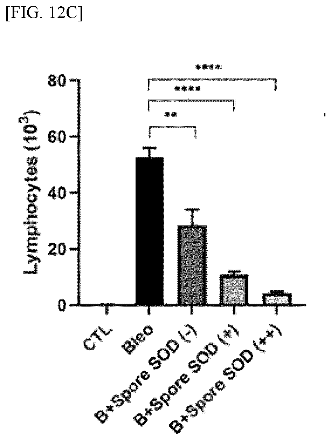

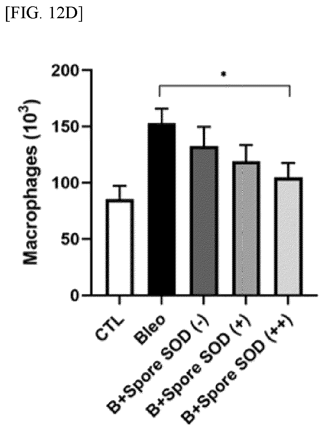

As a result of the measurement, referring to FIGS. 12A to 12D, a decrease in the number of immune cells in the bronchoalveolar lavage fluid was observed in all groups administered with the spores from the SOD knock-out strain, the spores from the SOD-expressing normal strain, and the spores from the SOD-overexpressing strain, compared to the bleomycin-treated group. The group administered with the spores from the SOD-expressing normal strain showed a greater decrease in the number of immune cells than the group administered with the spores from the SOD knock-out strain, and the group administered with the spores from the SOD-overexpressing strain showed the most significant decrease.

### Example 2.4. Measurement of concentration of transformation growth factor beta 1 in bronchoalveolar lavage fluid

Measurement of the TGF-β1 concentration using bronchoalveolar lavage fluid was performed in the same manner as in Example 1.4.

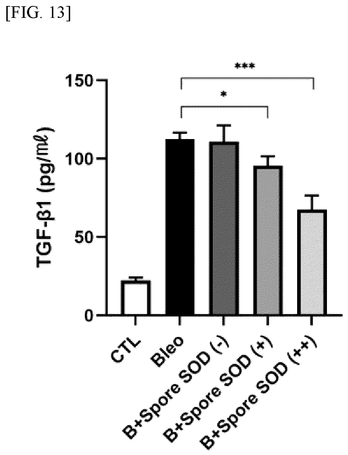

As a result of the measurement, referring to FIG. 13, the amount of TGF-β1, which had increased due to pulmonary fibrosis in the bleomycin-treated group, decreased in the following order: the group administered with the SOD knock-out strain spores, the group administered with the SOD-expressing normal strain spores, and the group administered with the SOD-overexpressing strain spores.

### Example 2.5. Identification of of pulmonary fibrosis through pathological tissue staining

The pathological tissue staining method using the extracted lung tissue was performed in the same manner as in Example 1.5.

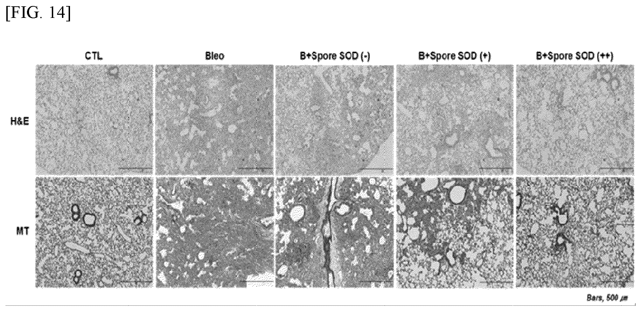

Referring to FIG. 14, which shows tissue staining results for the control group, the bleomycin-treated group, and the groups administered with the spores from the strains having different SOD expression levels (spores from the knock-out, normal, and overexpression strains), it was found that bleomycin-induced pulmonary fibrosis was improved by a spore SOD. In particular, the most significant improvement in pulmonary fibrosis was observed in the group administered with the spores from the SOD-overexpressing strain.

### Example 2.6. Measurement of expression levels of pulmonary fibrosis gene markers

Gene expression of Col1a1, α-SMA, TGF-β1, CTGF, TNF-α, and IL-6, which are representative gene markers known to be activated as pulmonary fibrosis progresses, was checked by real-time polymerase chain reaction using the primers in Table 7 (which is the same method as in Example 1.6).

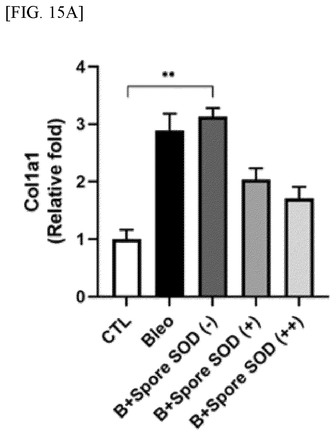

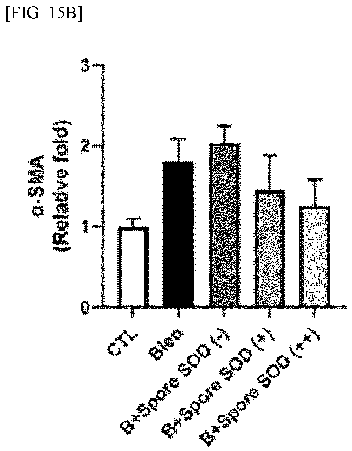

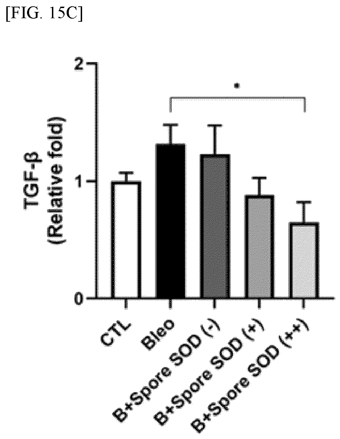

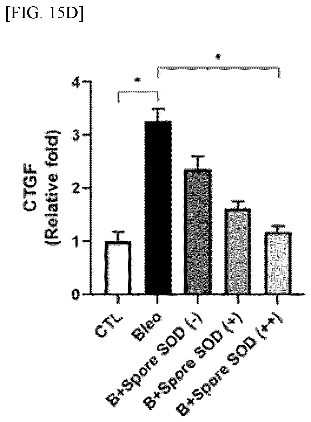

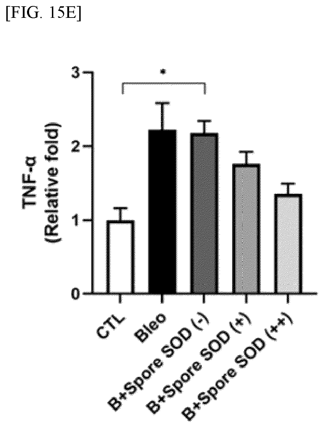

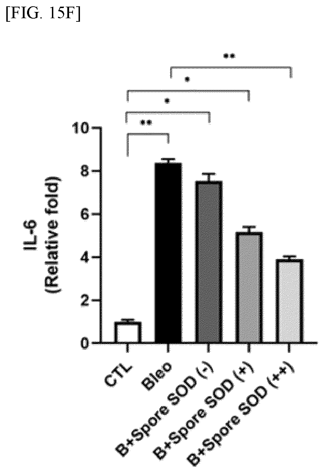

As a result of the measurement, referring to FIGS. 15A to 15F, it was found that the expression of the gene markers, which had been increased by bleomycin, significantly decreased depending on SOD expression levels of the administered spores. The group administered with the SOD knock-out strain spores showed similar or higher expression levels of Col1a1 (FIG. 15A), α-SMA (FIG. 15B), and TGF-β1 (FIG. 15C) compared to the bleomycin-treated group. On the other hand, the group administered with the SOD-expressing normal strain spores and the group administered with the SOD-overexpressing strain spores showed significantly decreased expression levels of the six markers compared to the bleomycin-treated group and the group administered with the SOD knock-out strain spores. In particular, the group administered with the SOD-overexpressing strain spores showed statistically significantly decreased expression levels of TGF-β1 (FIG. 15C), CTGF (FIG. 15A), and IL-6 (FIG. 15F) genes.

### Example 2.7. Measurement of protein expression levels of pulmonary fibrosis markers

A portion of the right lung lobe from the pulmonary fibrosis model mice sacrificed in Example 2.1 was finely homogenized in 1X RIPA buffer (containing protease and phosphatase inhibitor cocktail, purchased from Abcam). The tissue suspension was centrifuged (at 4°C and 12,000 rpm for 10 minutes). Then, only the clean supernatant was separated and transferred to a new 1.5 ml tube. For Western blotting, protein quantification was carried out using a BCA assay kit (purchased from Thermo Fisher Scientific) and measurement was performed at 562 nm with a multiplate spectrometer. An equal amount of the sample, calculated through protein quantification, was loaded on each lane of the gel and subjected to electrophoresis. The gel was transferred to a PVDF membrane, and incubation was performed overnight at 4°C with primary antibodies (TGF-β (1:1000, purchased from Abcam), phospho-Smad2/3 (1:1000, purchased from Abcam), Smad2/3 (1:1000, purchased from Abcam), α-SMA (1:1000, purchased from CST), Col1a1 (1:1000, purchased from CST), and GAPDH (1:2000, purchased from CST)). The next day, the membrane was thoroughly washed with 1X PBST buffer, and then incubation was performed with a secondary antibody (anti-rabbit) at room temperature for 1 hour. Band patterns were measured using imaging equipment (ChemiDoc imaging system, purchased from Bio-rad).

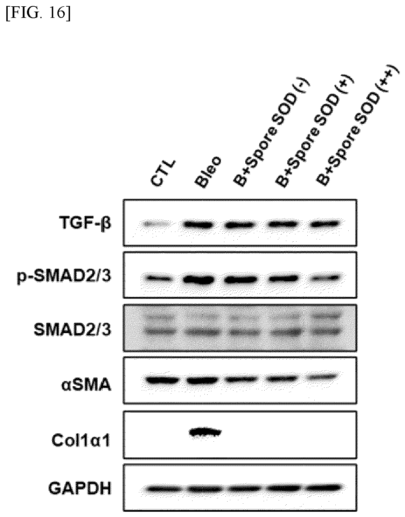

As a result of the measurement, referring to FIG. 16, it was found that in the bleomycin-treated group, TGF-β1-Smad2/3 signal transduction, which is a signaling pathway associated with pulmonary fibrosis, was activated, thereby leading to increased expression of TGF-β1 and phospho-Smad2/3 proteins. In addition, the bleomycin-treated group also showed increased production of α-SMA and Col1a1 which are downstream products of the signal transduction (Lane 2). On the other hand, it was found that compared to the bleomycin-treated group, the group administered with the SOD knock-out strain spores and the group administered with the SOD-expressing normal strain spores showed a slightly decreased expression level of phospho-Smad2/3 protein as well as slightly decreased expression levels of α-SMA and Col1a1 (Lanes 3 and 4). It was found that in the group administered with SOD-overexpressing strain spores, expression of upstream TGF-β1 was suppressed, which lead to decreased phosphorylation of downstream Smad2/3, and production of α-SMA and Col1a1, which are downstream products of the signal transduction, was significantly decreased (Lane 5).

### Example 2.8. Measurement of survival rate

In order to check the survival rate of the pulmonary fibrosis mouse model in a case where the pulmonary fibrosis was induced after administration of the spores from the strains having different SOD expression levels, the survival rate was estimated by the Kaplan-Meier method, and statistical differences were calculated using the log-rank test.

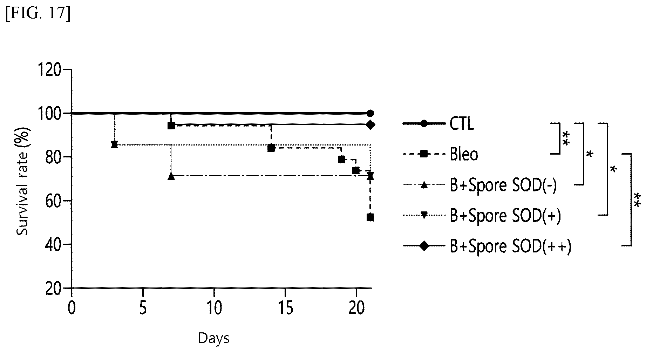

As a result of the estimation, referring to FIG. 17, the survival rate of the control group (CTL, N=13/13) was 100%, whereas the survival rate of the bleomycin-treated group (Bleo, N=10/19) decreased to 52.6%. On the other hand, the survival rate of the group administered with SOD knock-out strain spores (B+Spore SOD(-), N=5/7) was 71.4%, and the survival rate of the group administered with the SOD-expressing normal strain spores (B+Spore SOD(+), N=5/7) also showed 71.4%, indicating no difference compared to the group administered with the SOD knock-out strain spores. In contrast, the survival rate of the group administered with the SOD-overexpressing strain spores (B+Spore SOD(++), N=19/20) significantly increased to 95%. For the survival rate results of the experimental groups, see Table 9.

**[Table 9]**

| **Model name** | **Survival rate (%), based on Day 21** | **P-value** | |
|---|---|---|---|
| | | **CTL vs. group** | **Bleo vs. group** |
| Control group (CTL) | 100 | - | ** |
| Bleomycin-treated group (Bleo) | 52.6 | ** | - |
| SOD knock-out strain spore (Spore SOD (-)) administration group | 71.4 | * | ns |
| SOD-expressing normal strain spore (Spore SOD (+)) administration group | 71.4 | * | ns |
| SOD-overexpressing strain spore (Spore SOD (++)) administration group | 95 | ns | ** |

From the results, it was found that an SOD had a preventive effect on pulmonary fibrosis and thus also contributed to increased survival rate.

### Example 2.9. Measurement of concentrations of reactive oxygen byproducts

The method for measuring concentrations of reactive oxygen byproducts (MDA and 4-HNE) in the extracted lung tissue was performed in the same manner as in Example 1.7.

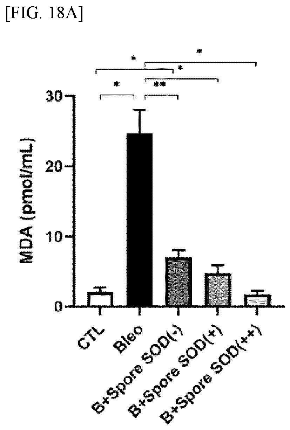

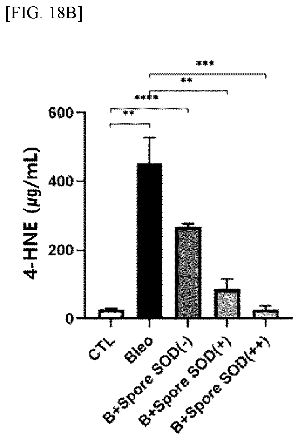

As a result, it was found that the concentrations of byproducts, which had been increased by bleomycin, significantly decreased depending on the SOD expression levels of the administered spores. The concentrations of MDA and 4-HNE significantly decreased in the following order: the group administered with the SOD knock-out strain spores, the group administered with the SOD-expressing normal strain spores, and the group administered with the SOD-overexpressing strain spores (FIGS. 18A and 18B).

### Example 3. Comparison of preventive effects between SOD-overexpressing strain spores and SOD in pulmonary fibrosis animal model

### Example 3.1. Preparation of samples and testing

Since an SOD has difficulty to maintain its protein activity and stability when administered orally alone, it may be mostly degraded by gastric acid before exerting its effects in the body. On the other hand, spores produced by bacteria are resistant to harsh environments (for example, extreme temperatures, acids or alkalis, desiccation, and toxic chemicals, and the like), and thus expected to be effective even when administered orally. To compare effects of the two substances, an SOD was orally administered to pulmonary fibrosis model mice in the form of free SOD (B+Free SOD, 2 units/mouse) and GF424 spores (B+Spore SOD, 1×10⁷CFU/mouse) and their preventive effects on pulmonary fibrosis were compared.

The free SOD was obtained by preparing a culture of *Bacillus velezensis* GF424 strain (KCTC 13227BP, Accession Date: March 13, 2017) and extracting and purifying it using the following method.

First, a single colony formed on Luria-Bertani (LB) agar medium (10 g/L tryptophan, 5 g/L yeast extract, 10 g/L NaCl, 15 g/L agar) was inoculated into 30 mL of LB medium and cultured at 37°C for 12 hours. This seed culture was then inoculated into 3 L of LB medium containing 1 mM manganese sulfate (MnSO₄) and cultured at 37°C for 20 hours.

Next, the cell culture was centrifuged at 4°C and 3,578×g for 20 minutes, and the supernatant was collected. Then, the supernatant was concentrated 10-fold using ultrafiltration (UF, MWCO 10,000). The concentrate was filtered through a sterilizing filter, and then lyophilized. SOD activity was analyzed using a SOD assay kit (purchased from Cayman Chemical, Michigan, USA). One unit of SOD activity is defined as the amount of enzyme that inhibits 50% of superoxide radicals. The dried SOD enzyme was dissolved in PBS to exhibit activity of 2 units and prepared in a volume of 100 µl.

Administration of GF424 spores or free SOD and bleomycin treatment for the two test groups were performed in the same manner as described in Example 1.1, and the control group and the bleomycin-treated group were also prepared in the same manner. The experiment was terminated on the 21st day after the initial spore administration, and the mice in each group were sacrificed (see FIG. 4).

### Example 3.2. Measurement of collagen concentration by measurement of hydroxyproline concentration

Measurement of hydroxyproline, which allows for determination of collagen level, was performed in the same manner as in Example 1.2.

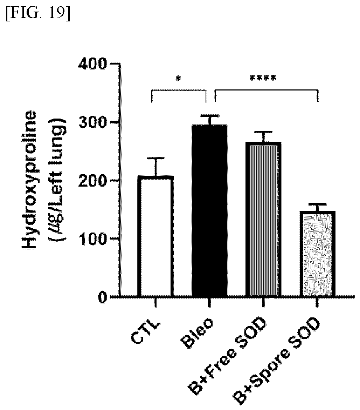

Referring to FIG. 19, the measurement of hydroxyproline concentrations in the free SOD-administered group and the GF424 spore-administered group showed that unlike the free SOD-administered group having almost no difference in hydroxyproline concentration compared to the bleomycin-treated group, the GF424 spore-administered group had a statistically significantly decreased hydroxyproline level. Therefore, it was found that administration of GF424 spores was effective in preventing pulmonary fibrosis.

### Example 3.3. Measurement of concentration of growth factor beta 1 in bronchoalveolar lavage fluid

Measurement of the TGF-β1 concentration using bronchoalveolar lavage fluid was performed in the same manner as in Example 1.4.

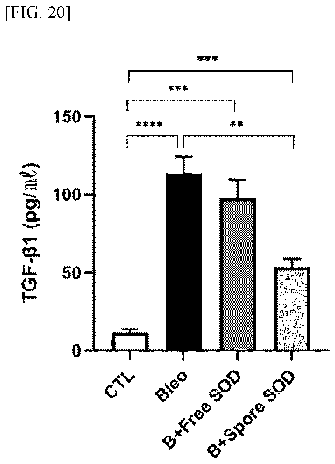

As a result of the measurement, referring to FIG. 20, the amount of TGF-β1, which had increased due to pulmonary fibrosis in the bleomycin-treated group, decreased very effectively in the GF424 spore-administered group, whereas the effect was minimal in the free SOD-administered group.

### Example 3.4. Identification of pulmonary fibrosis through pathological tissue staining

The pathological tissue staining method using the extracted lung tissue was performed in the same manner as in Example 1.5.

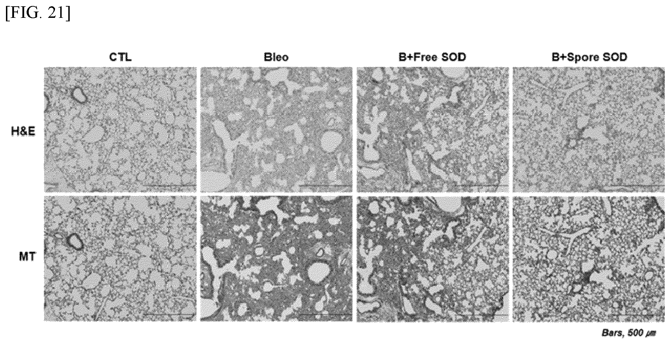

Referring to FIG. 21, which shows tissue staining results for the control group, the bleomycin-treated group, the free SOD-administered group, and the GF424 spore-administered group, it was found that bleomycin-induced pulmonary fibrosis was slightly improved in the free SOD-administered group but was more significantly improved in the GF424 spore-administered group

### Example 3.5. Measurement of expression levels of pulmonary fibrosis gene markers

Gene expression of Col1a1, α-SMA, TGF-β1, CTGF, TNF-α, and IL-6, which are representative gene markers known to be activated as pulmonary fibrosis progresses, was checked by real-time polymerase chain reaction using the primers in Table 7 (which is the same method as in Example 1.6).

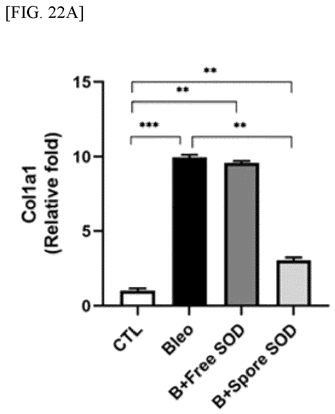

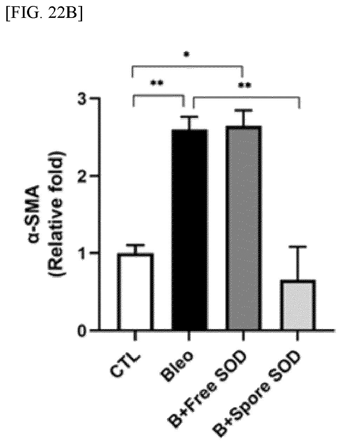

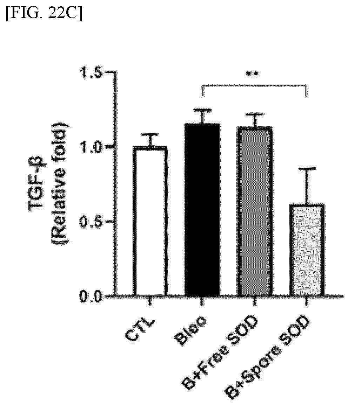

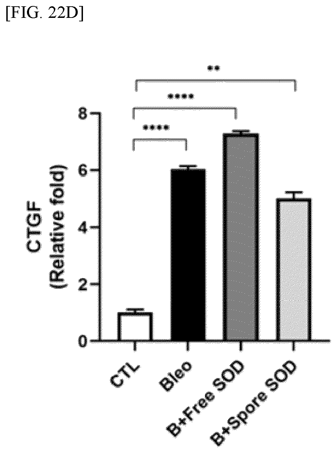

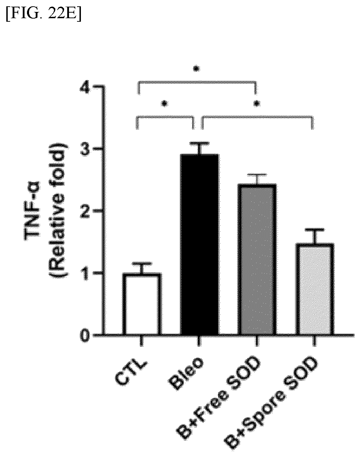

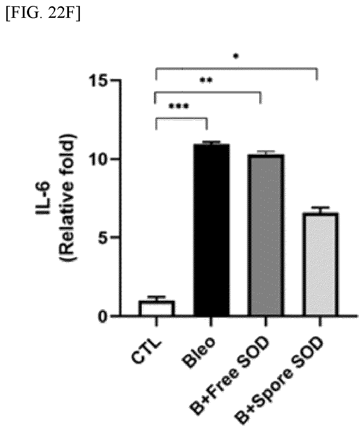

As a result of the measurement, referring to FIGS. 22A to 22F, the pro-fibrotic markers (Col1a1, α-SMA, TGF-β1, and CTGF) and pro-inflammatory cytokines (TNF-α and IL-6), which had been increased by bleomycin, showed almost no change in the free SOD-administered group, but were significantly decreased in the GF424 spore-administered group.

### Example 3.6. Measurement of protein expression levels of pulmonary fibrosis markers

Western blotting for measurement of protein expression levels of pulmonary fibrosis markers in pulmonary fibrosis model mice was performed in the same manner as in Example 2.7.

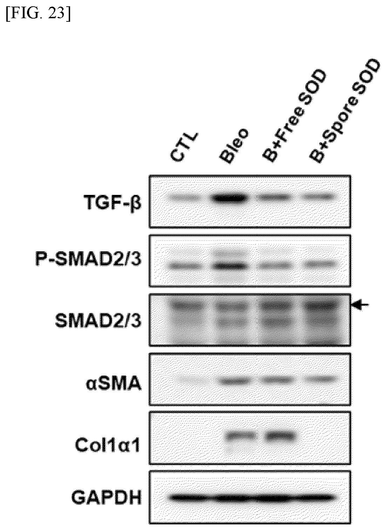

As a result of the measurement, referring to FIG. 23, it was found that the expression of TGF-β1 and phospho-Smad2/3 proteins was decreased by inhibition of TGF-β1-Smad2/3 signal tranduction, which is the main mechanism of pulmonary fibrosis, in both the free SOD-administered group and the GF424 spore-administered group. On the other hand, the fibrosis markers α-SMA and Col1a1 showed a tendency to decrease in both administration groups, and Col1a1 was more effectively decreased in the GF424 spore-administered group (Lane 4).

### Example 3.7. Measurement of concentrations of reactive oxygen byproducts

The method for measuring concentrations of reactive oxygen byproducts (MDA and 4-HNE) in the extracted lung tissue was performed in the same manner as in Example 1.7.

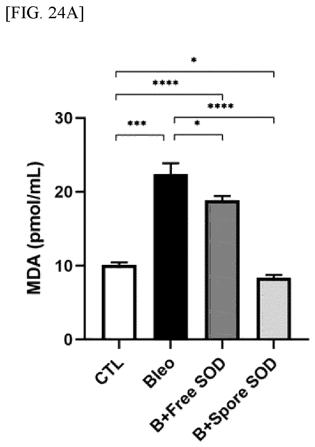

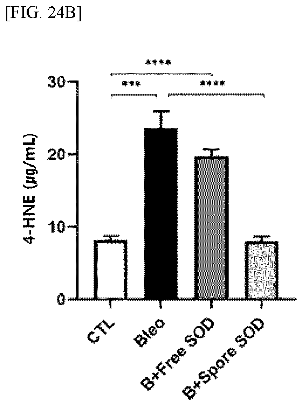

As a result, for the concentrations of the reactive oxygen byproducts MDA and 4-HNE, which had been increased by bleomycin, the free SOD-administered group showed almost no change, whereas the GF424 spore-administered group showed a significant decrease (FIGS. 24A and 24B).

### Example 4. Identification of preventive effects of SOD-overexpressing strain spores in primary lung fibroblasts isolated from pulmonary fibrosis animal model

### Example 4.1. Isolation and culture of mouse primary lung fibroblasts

The GF424 spore-administered group (B+Spore SOD) was orally administered with the spores prepared by the method described in Preparation Example 1 at a concentration of 1×10⁷ CFU, followed by induction of pulmonary fibrosis with bleomycin. Spore administration and bleomycin treatment were performed in the same manner as described in Example 1.1, and the control group and the bleomycin-treated group were prepared in the same manner. The experiment was terminated on the 21st day after the initial spore administration, and the mice in each group were sacrificed (see FIG. 4).

The chest of the pulmonary fibrosis model mice was opened, and then the lungs were sufficiently perfused with PBS (3% penicillin/streptomycin). The cleaned whole lungs were extracted, and then washed with fresh PBS containing 3% antibiotics (penicillin/streptomycin). The washed lung tissue was transferred to a 6-well plate, and then cut into pieces with sterilized scissors. The tissue pieces were transferred to a new 15 ml tube, and then 5 ml of RPMI-1640 (2% penicillin/streptomycin, glutamine-free) medium containing 600 units/ml DNase I (purchased from Sigma, Cat.#: DN25) and 0.14 units/ml liberase (purchased from Roche, Cat.#: 05401119001) enzymes was added thereto. Then, incubation was performed at 37°C for 1 hour. After completion of the reaction, the tissue-containing solution in the tube was filtered sequentially through a 40 µm filter, a 70 µm filter, and a 100 µm filter, and then the tube containing the finally filtered content was centrifuged at 520×g for 10 minutes. Finally, the supernatant was removed, and the remaining cells were suspended in an appropriate amount of DMEM (15% FBS, 2% penicillin/streptomycin) medium to prepare a cell suspension.

### Example 4.2. Identification of pulmonary fibrosis through immunofluorescence staining

In a case where lung fibroblasts are subjected to external stimulation and activation of TGF-β pathway occurs, morphological changes (elongated shape) and functional changes (increase in ECM products such as α-SMA and collagen) occur, which leads to progression of pulmonary fibrosis. To identify these changes, the primary lung fibroblasts isolated from the lungs of the mice with induced pulmonary fibrosis, were stained for α-SMA and Col1a1, which are representative indicators of pulmonary fibrosis, using the following immunofluorescence staining method.

The mouse cell suspension in Example 4.1 was used and cell counting was performed. Then, 2×10⁶ cells were placed in a 60 mm culture dish and cultured for one day. The next day, the medium was replaced with fresh DMEM (15% FBS, 2% penicillin/streptomycin) medium, and the medium replacement was repeated every 2 to 3 days until the cells filled 85 to 90% of the surface area of the 60 mm culture dish. Subsequently, the cells were detached from the dish, and then seeded at a density of 40,000 cells per well on an 8-channel slide. When the cells filled more than 80% of the surface area, the medium was removed, and then the cells were washed with fresh PBS (repeated 3 times). The cells were fixed with 4% PFA at room temperature for 1 hour, and then washed with PBS. Incubation was performed with blocking buffer (purchased from Invitrogen, Cat.#: 00-4952-54) at room temperature for 1 hour. The cells were washed with PBS (repeated 3 times). Then, the primary antibodies α-SMA (1:200, Mouse mAb, purchased from CST, Cat.#: 48938) and Col1a1 (1:200, Rabbit mAb, purchased from CST, Cat.#: 72026) were diluted in blocking buffer, and then added to each well. Incubation was performed overnight at 4°C. The next day, the cells were washed with PBS (repeated three times). The secondary antibodies anti-Rabbit-Alexa 555 and antiMouse-Alexa 488 were diluted 1:500 in PBS and added to each well. Then, incubation was performed at room temperature for 1 hour while protected from light. Subsequently, the cells were washed with PBS (3 times). Then, DAPI-containing mounting solution was added dropwise to the stained cells from which PBS was completely removed. A cover glass was placed over the cells, and then observation using a confocal microscope (purchased from Zeiss) was performed.

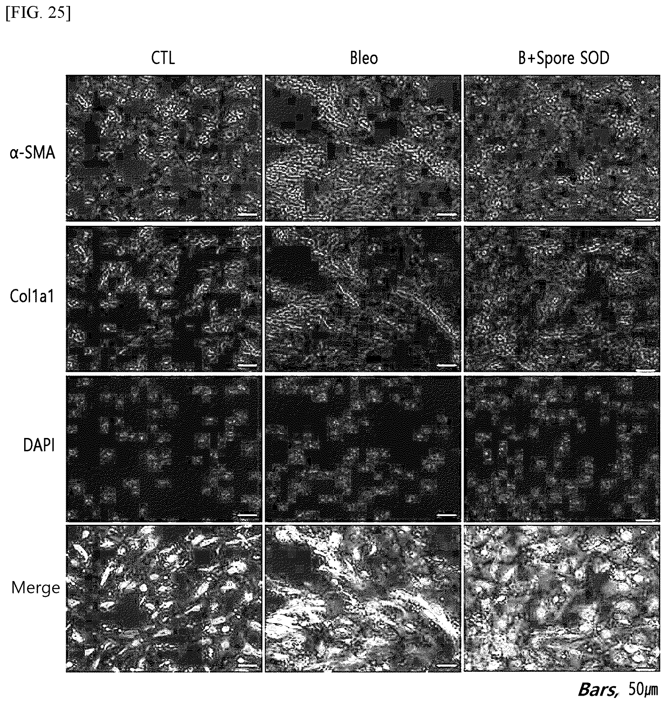

As a result of the measurement, referring to FIG. 25, in the bleomycin-treated group, the cell shape became elongated and the expression of α-SMA and Col1a1 increased. On the other hand, the GF424 spore-administered group showed morphological changes (shortened cell shape) and functional changes (decreased expression of α-SMA and Col1a1) similar to the control group, indicating preventive effects on pulmonary fibrosis.

### Example 4.3. Measurement of expression levels of pulmonary fibrosis gene markers

To verify whether there were changes in expression levels of pulmonary fibrosis genes in primary lung fibroblasts as in the lung tissue of the mice with induced pulmonary fibrosis, real-time polymerase chain reaction was performed using the primers in Table 7 (which is the same method as in Example 1.6).

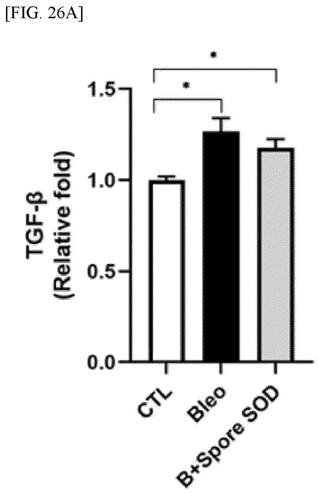

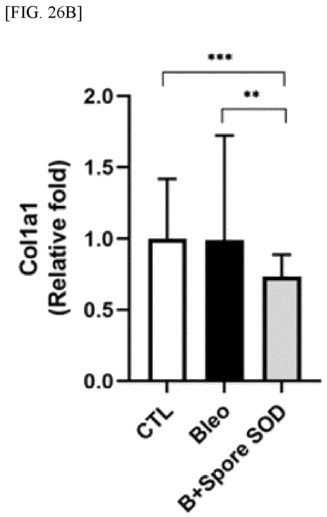

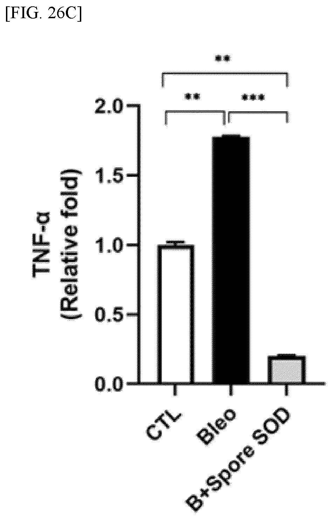

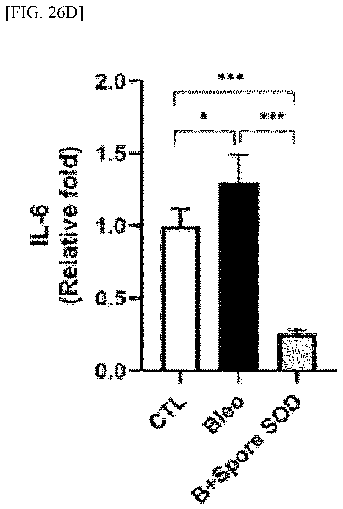

Expression levels of TGF-β1 (a factor that activates myofibroblasts and promotes fibrosis), Col1a1 (a factor that increases collagen synthesis), TNF-α and IL-6 (inflammatory cytokine) were measured, which are representative gene markers known to be activated as pulmonary fibrosis progresses. As a result, referring to FIGS. 26A to 26D, the pro-fibrosis markers (TGF-β and Col1a1) and pro-inflammatory cytokines (TNF-α and IL-6), which were increased by bleomycin, were all significantly decreased in the GF424 spore-administered group.

### Example 5. Identification of effects of combined administration of oral SOD and strain spores in pulmonary fibrosis animal model

### Example 5.1. Preparation of samples and testing

An experiment was prepared to identify preventive effects on pulmonary fibrosis by combined administration of oral SOD and strain spores. First, GF103, which is an oral SOD with increased stability against gastric acid, was prepared at three concentrations (1, 10, and 20 units/mouse), and the wild-type strain GF423 spores (B+Spore SOD(+)) produced in Preparation Example 1 were prepared at a concentration of 1×10⁷ CFU/mouse. In addition, the overexpression strain GF424 spores (B+Spore SOD(++), 1×10⁷ CFU/mouse) were prepared as a comparison group for the effects obtained by combined treatment.

GF103 (SEQ ID NO: 6; Met-deleted SodA2) was produced by subjecting methionine-deleted SodA2 to shellac-coating as follows. Shellac (EXCELACS Co., Ltd., Bangkok) was dissolved in 100% ethanol to a concentration of 3%, and filtered using a 0.2 um sterile filter. The resulting solution was diluted 1/20 times with sterile 1x PBS to prepare a shellac solution. Lyophilized purified SodA2 was dissolved to 20 mg/ml and filtered through a 0.2 µm filter to ensure sterility. The prepared shellac solution and the prepared dissolved SOD were mixed in a 1:1 ratio with stirring. Then, stirring was continued for 10 minutes. The well-stirred material was lyophilized. By doing so, the finally lyophilized shellac-coated SodA2 was prepared. The lyophilized shellac-coated SodA2 was mixed with dextrin in a ratio of 1:9 to 12 (shellac-coated SodA2: dextrin), and activity measurement was performed so that the mixture had final activity ranging from 90 to 110 U/mg.

For the free SOD, lyophilized purified SodA2 without shellac coating was used.

Spore administration and bleomycin treatment were performed in the same manner as described in Example 1.1, and the control group and the bleomycin-treated group were prepared in the same manner. The experiment was terminated on the 21st day after the initial spore administration, and the mice in each group were sacrificed (see FIG. 4).

### Example 5.2. Measurement of collagen concentration by measurement of hydroxyproline concentration

Measurement of hydroxyproline, which allows for determination of collagen level, was performed in the same manner as in Example 1.2.

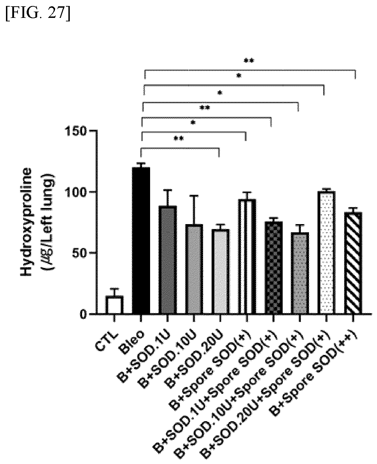

As a result of the measurement of hydroxyproline concentration, it was found that the hydroxyproline concentration decreased in all experimental groups compared to the bleomycin-treated group, that is, the hydroxyproline concentration decreased in a concentration-dependent manner even in the group administered with the oral SOD (GF103) alone. On the other hand, it was found that the groups administered with oral SOD and GF423 spores in combination showed significant effects at the concentrations of 1 unit/mouse and 10 units/mouse of the oral SOD. In particular, the best effect was observed in the group administered with oral SOD at a concentration of 10 units/mouse and GF423 spores in combiniation, and the hydroxyproline concentration in the group was measured to be lower than that in the group administered with the GF424 spores which are SOD-overexpressing strain spores (FIG. 27).

### Example 5.3. Measurement of concentration of growth factor beta 1 in bronchoalveolar lavage fluid

Measurement of the TGF-β1 concentration using bronchoalveolar lavage fluid was performed in the same manner as in Example 1.4.

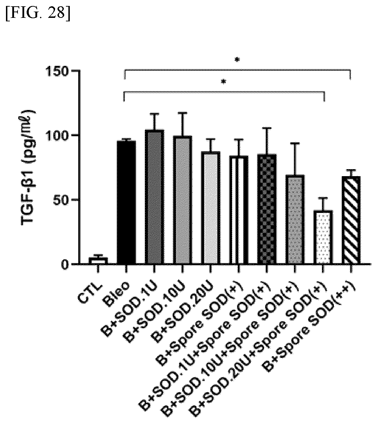

As a result of the measurement, referring to FIG. 28, it was found that the amount of TGF-β1, which had increased due to pulmonary fibrosis in the bleomycin-treated group, showed almost no change when the oral SOD (GF103) was administered alone, but was effectively decreased in the group administered with oral SOD and GF423 spores in combination. In particular, the best effect was observed in the group administered with the oral SOD at a concentration of 20 units/mouse and the GF423 spores in combination, and the TGF-β1 concentration in the group was measured to be lower than that in the group administered with the SOD-overexpressing strain GF424 spores. In contrast, for the group administered with the oral SOD alone, there was almost no change in the amount of TGF-β1.

### Example 5.4. Identification of pulmonary fibrosis through pathological tissue staining

The pathological tissue staining method using the extracted lung tissue was performed in the same manner as in Example 1.5.

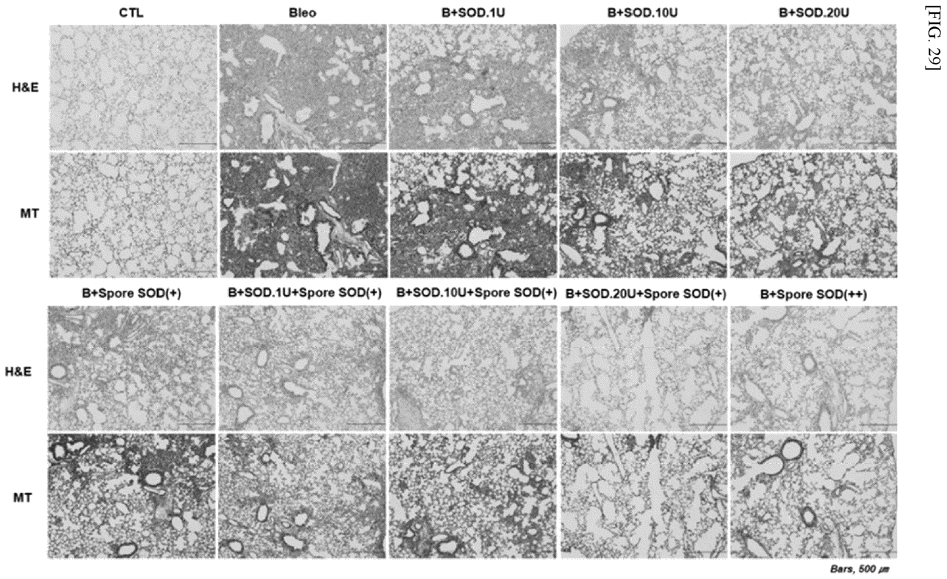

Referring to FIG. 29, which shows tissue staining results of the control group, the bleomycin-treated group, the groups administered with GF103 at different concentrations, the GF423 spore-administered group, and the groups administered with GF103 at different concentrations and GF423 spores in combination, and the GF424 spore-administered group, it was found that bleomycin-induced pulmonary fibrosis was improved in a concentration-dependent manner even in the group administered with the oral SOD alone, and it was also found that bleomycin-induced pulmonary fibrosis was improved in a concentration-dependent manner in the group administered with the oral SOD and the GF423 spores in combination, which indicates that the combined administration shows a synergistic effect.

### Example 5.5. Measurement of expression levels of pulmonary fibrosis gene markers

Gene expression of Col1a1, α-SMA, TGF-β1, IL-6, TNF-α, and IL-1β, which are representative gene markers known to be activated as pulmonary fibrosis progresses, was checked by real-time polymerase chain reaction using the primers in Table 7 (which is the same method as in Example 1.6).

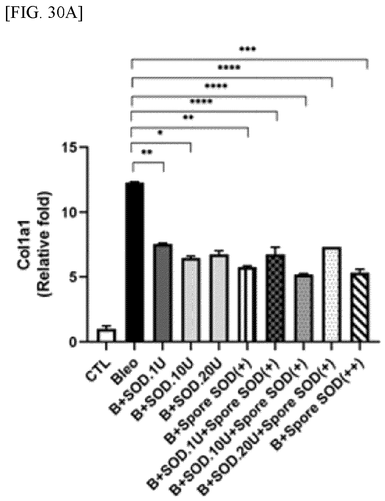

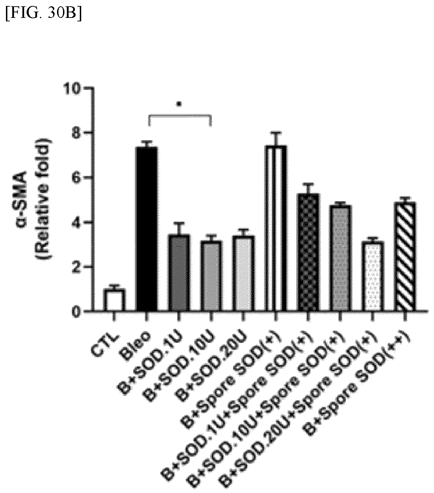

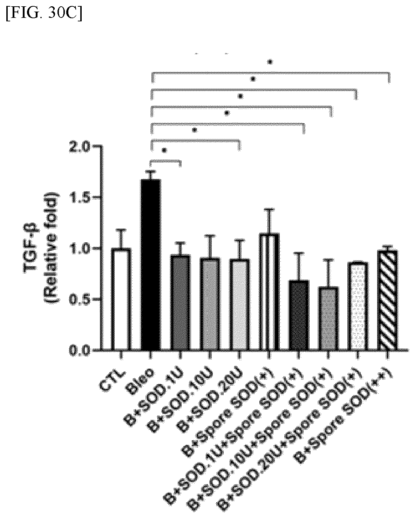

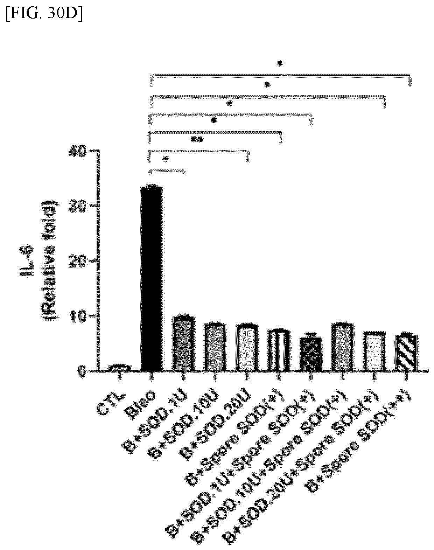

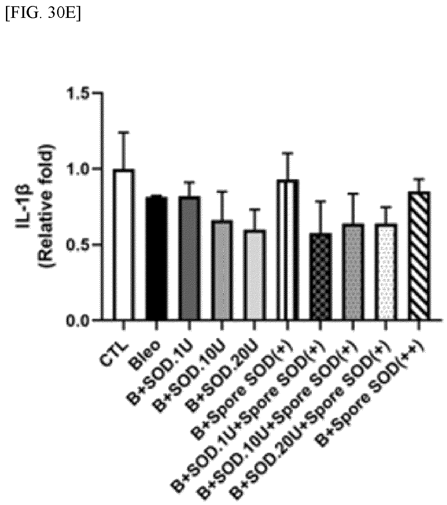

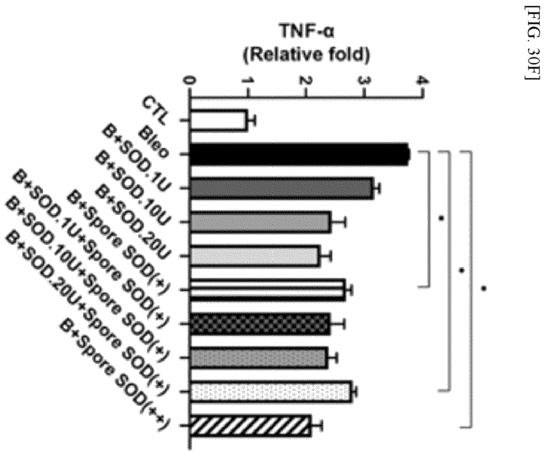

As a result of the measurement, referring to FIGS. 30A to 30F, the pro-fibrotic markers (Col1a1, α-SMA, and TGF-β) and pro-inflammatory cytokines (IL-6, TNF-α, and IL-1β), which had been increased by bleomycin, were effectively decreased by the oral SOD, but combined administration of the oral SOD and the GF423 spores had no synergistic effect.

### Example 5.6. Measurement of concentrations of reactive oxygen byproducts

In addition to MDA and 4-HNE, which are byproducts produced from lipid peroxides by reactive oxygen species, 8-hydroxydeoxyguanosine (8-OHdG), which is produced when oxidative DNA damage is induced by reactive oxygen species, is also an important marker of oxidative stress. Therefore, the concentrations of reactive oxygen byproducts (MDA and 4-HNE) in the extracted lung tissue were measured in the same manner as in Example 1.7, and the concentration of 8-OHdG was measured using the following method, thereby comparing the values between the experimental groups.

For 8-OhdG, the lung tissue was cut into small pieces and total DNA was extracted therefrom using the DNeasy Blood & Tissue Kit (Qiagen, Cat.# 69504). Then, an equal amout of DNA was used for Oxidative DNA damage ELISA kit (Cell Biolabs, Cat.# STA-320) according to the manufacturer's manual. The concentration of 8-OhdG was measured at 450 nm using a multiplate spectrometer.

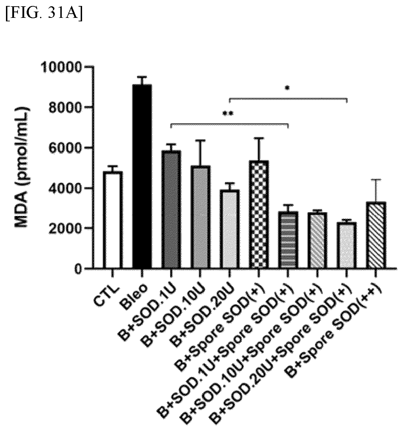

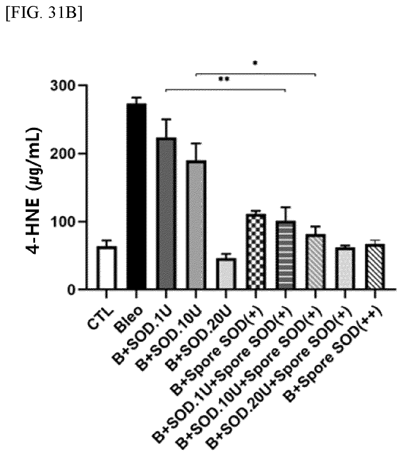

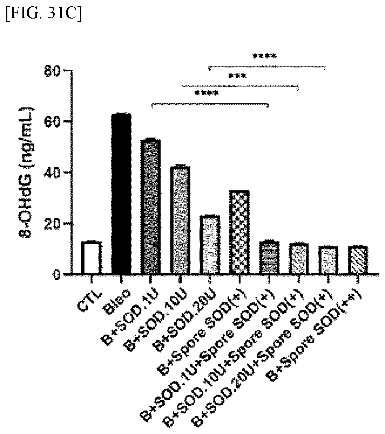

As a result, all of MDA, 4-HNE, and 8-OHdG, which are present in the lung tissue, showed a tendency to decrease in a concentration-dependent manner by treatment with the oral SOD; and such byproducts were generally further decreased by combined administration of the oral SOD and the GF423 spores (FIGS. 31A to 31C).

### Example 6. Identification of therapeutic effects of SOD-overexpressing strain spores on fibrosis in pulmonary fibrosis animal model

### Example 6.1. Establishment of pulmonary fibrosis animal model and testing

From the results obtained by administering GF424 spores to a pulmonary fibrosis animal model and then inducing pulmonary fibrosis, it was identified that administration of the spores had preventive effects on pulmonary fibrosis (see Example 1 and the like). Accordingly, animals were first subjected to induction of pulmonary fibrosis, and then administered with GF424 strain spores and spores from GF427 strain that was developed to produce about 4-fold more SOD than GF424 strain. Related indicators were checked in the animals. For production of the GF427 strain, see Preparation Example 2.

7-week-old C57BL/6 male mice were acclimated for one week. Then, the animals were observed for general conditions to determine their health status, and only the healthy animals were used in the experiment. The mice were divided into four groups: control group (CTL), bleomycin-treated group (Bleo), and test group (that is, GF424 spore-administered group and GF427 spore-administered group).

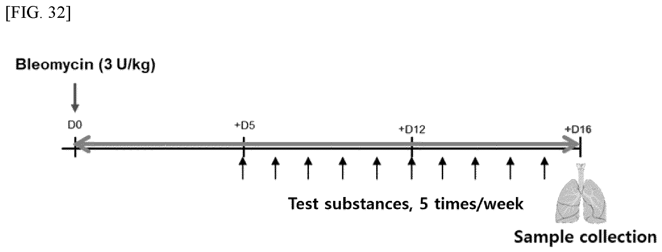

First, bleomycin (bleomycin sulfate, purchased from Millipore) was administered intratracheally once at a dose of 3 U/kg to induce pulmonary fibrosis (D0). Five days later, the respective mice in the test groups were orally administered once a day for 12 days (a total of 5 times per week) with GF424 spores, which were produced in Preparation Example 1, at a concentration of 1×10⁷ CFU, and GF427 spores, which were produced in Preparation Example 2, at three concentrations (1×10⁵ CFU, 1×10⁶ CFU, and 1×10⁷ CFU). The experiment was terminated on the 16th day from the initial administration of bleomycin, and the mice in each group were sacrificed. The control group (CTL) was neither subjected to induction of pulmonary fibrosis nor administered with the spores. For the administration schedule of spores and bleomycin over a total of 16 days, see FIG. 32.

### Example 6.2. Measurement of collagen concentration by measurement of hydroxyproline concentration

Measurement of hydroxyproline, which allows for determination of collagen level, was performed in the same manner as in Example 1.2.

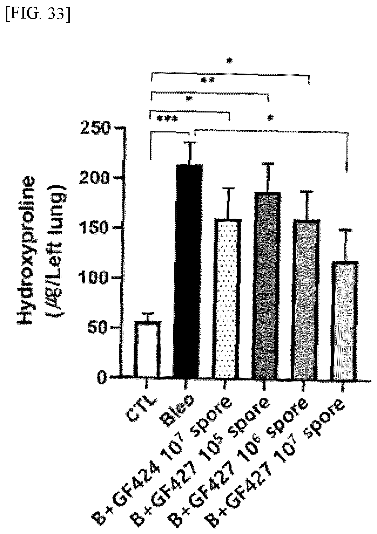

As a result of the measurement of hydroxyproline concentration, the hydroxyproline concentration decreased in all experimental groups compared to the bleomycin-treated group, and the GF427 spores decreased collagen production even at small doses. In particular, the group administered with GF427 spores at 1×10⁷ CFU, which is the same concentration as GF424 spores, showed a significant decrease compared to the group administered with GF424 spores (FIG. 33).

### Example 6.3. Measurement of concentration of growth factor beta 1 in bronchoalveolar lavage fluid

Measurement of the TGF-β1 concentration using bronchoalveolar lavage fluid was performed in the same manner as in Example 1.4.

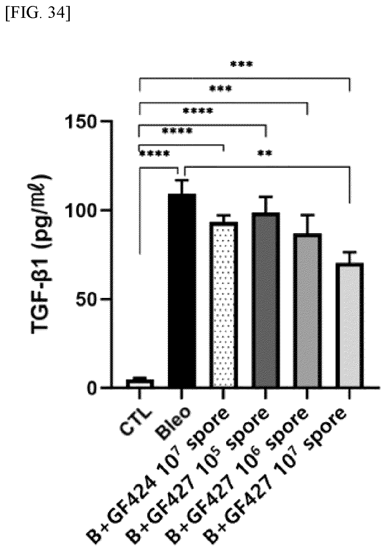

As a result of the measurement, referring to FIG. 34, it was found that the amount of TGF-β1, which had increased due to pulmonary fibrosis in the bleomycin-treated group, decreased even in the group administered with a small amount of GF427 spores (at a concentration of 1×10⁵ CFU). The groups administered with GF427 spores at concentrations of 1×10⁶ CFU and 1×10⁷ CFU showed a more significant decrease in the amount of TGF-β1 than the group administered with GF424 spores.

### Example 6.4. Identification of pulmonary fibrosis through pathological tissue staining

The pathological tissue staining method using the extracted lung tissue was performed in the same manner as in Example 1.5.

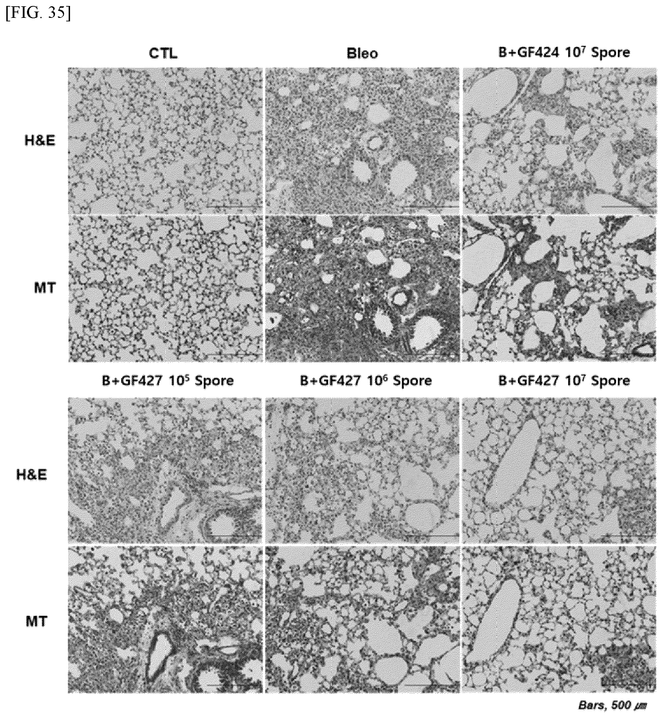

Referring to FIG. 35, which shows tissue staining results for the control group, the bleomycin-treated group, the GF424 spore-administered group, and the group administered with GF427 spores at different concentrations, it was found that bleomycin-induced pulmonary fibrosis was effectively decreased at all concentrations of GF427 spores; and in particular, the most significant improvement was observed at a concentration of 1×10⁷ CFU.

### Example 6.5. Measurement of expression levels of pulmonary fibrosis gene markers

Gene expression of Col1a1, TGF-β1, CTGF, IL-6, TNF-α, and IL-1β, which are representative gene markers known to be activated as pulmonary fibrosis progresses, was checked by real-time polymerase chain reaction using the primers in Table 7 (which is the same method as in Example 1.6).

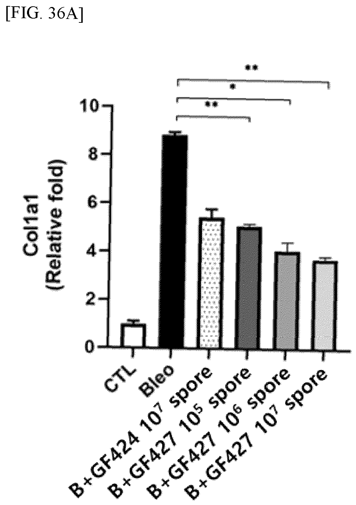

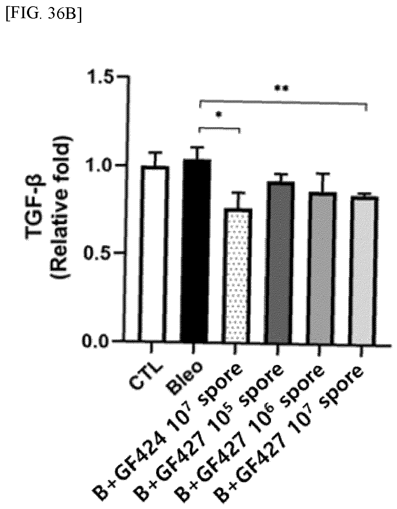

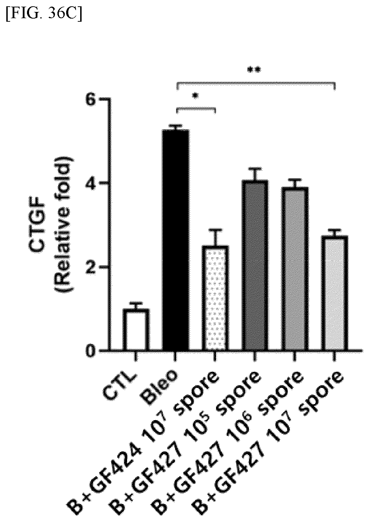

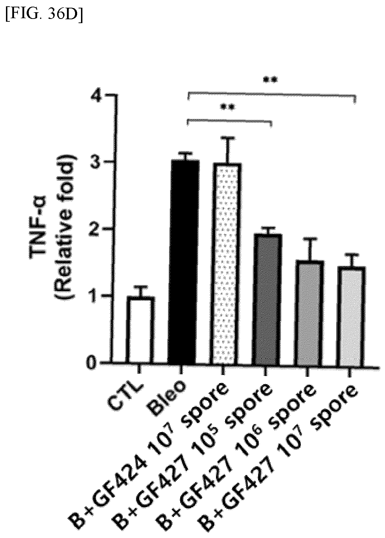

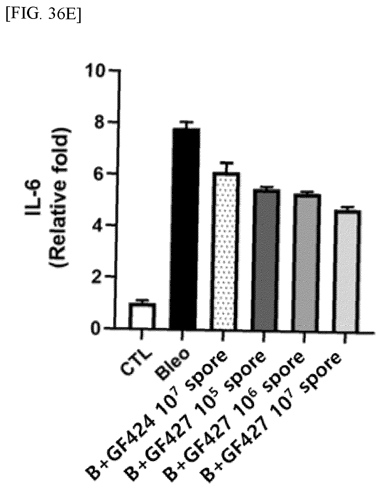

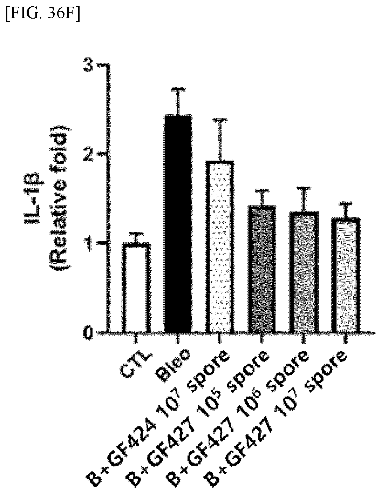

As a result of the measurement, referring to FIGS. 36A to 36F, the pro-fibrotic markers (Col1a1, TGF-β, and CTGF) and pro-inflammatory cytokines (IL-6, TNF-α, and IL-1β), which had been increased by bleomycin, showed a decrease in all groups administered with spores from SOD-overexpressing strains (GF424 and GF427); and in particular, these markers and cytokines were significantly decreased in the GF427 spore-administered group.

### Example 6.6. Measurement of protein expression levels of pulmonary fibrosis markers

Western blotting for measurement of protein expression levels of pulmonary fibrosis markers in pulmonary fibrosis model mice was performed in the same manner as in Example 2.7.

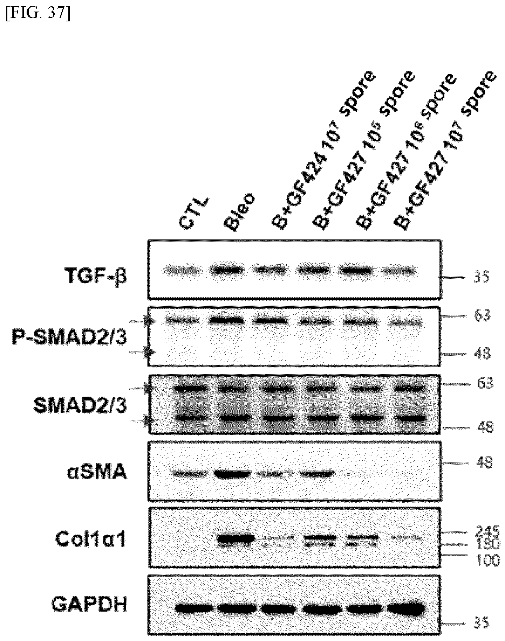

As a result of the measurement, referring to FIG. 37, both groups administered with spores from SOD-overexpressing strains (GF424 and GF427) showed a decrease in TGF-β1 expression and a decrease in phospho-Smad2/3 phosphorylation, due to inhibition of TGF-β1-Smad2/3 signal transduction that is the main mechanism of pulmonary fibrosis. In addition, the fibrosis markers α-SMA and Col1a1 were also decreased in both groups administered with spores from SOD-overexpressing strains (GF424 and GF427). In particular, expression levels of these proteins were significantly decreased when the GF427 spores were administered at a concentration of 1×10⁷ CFU.

### Example 6.7. Measurement of concentrations of reactive oxygen byproducts

The concentrations of MDA and 4-HNE, which are byproducts produced from lipid peroxides by reactive oxygen, were measured using the same method as in Example 1.7, and the concentration of 8-hydroxydeoxyguanosine (8-OHdG), which is produced when oxidative DNA damage is induced by reactive oxygen, was measured using the same method as in Example 5.6. In addition, the extent of thiol in the body depleted by ROS was measured to compare levels of oxidative stress.

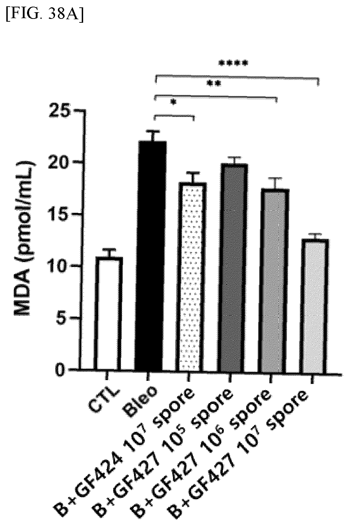

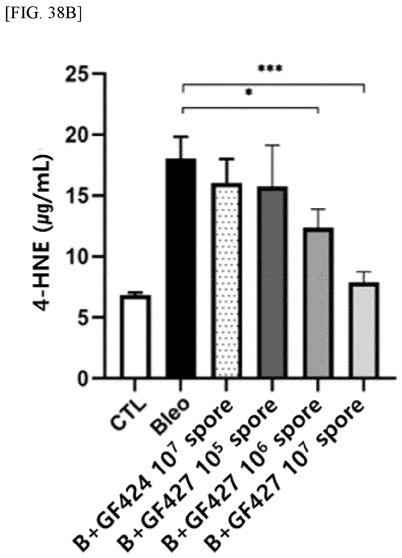

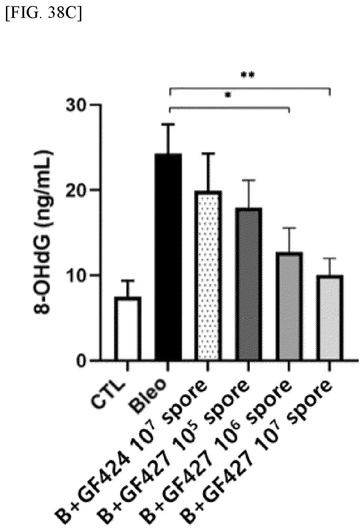

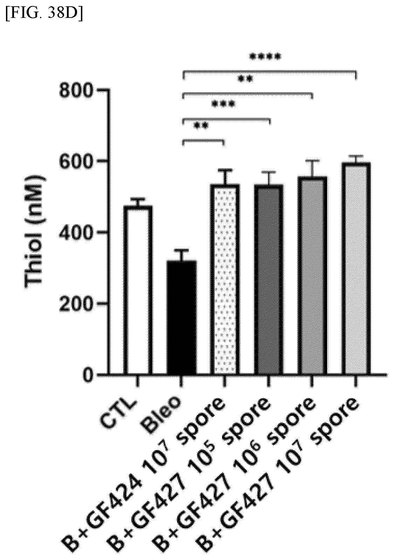

All of MDA, 4-HNE, and 8-OhdG, which are present in the lung tissue, showed a tendency to decrease in a concentration-dependent manner depending on the spores from SOD-overexpressing strains (GF424 and GF427). On the other hand, it was found that free thiols depleted by ROS were restored by the SOD-overexpressing strain spores. Depleted thiols were more effectively restored as the concentration of SOD-overexpressing strain spores increased (FIGS. 38A to 38D).

### Example 6.8. Biomarker Analysis

A piece of the lung tissue extracted from the mice sacrificed in Example 6.1 was finely homogenized in 1X RIPA buffer (containing protease inhibitor cocktail, purchased from Abcam). The tissue suspension was centrifuged at 4°C and 12,000 rpm for 10 minutes. Then, only the clean supernatant was separated and transferred to a new 1.5 mL tube. Protein quantification was carried out using a BCA assay kit (purchased from Thermo Fisher Scientific), and measurement was performed at 562 nm with a multiplate spectrometer. RNA obtained from a portion of the right lung lobe was subjected to real-time polymerase chain reaction to analyze SP-D, MMP-7, tenascin-C, periostin, CXCL13, and PAI-1. The relative mRNA expression levels were normalized using 18S rRNA as an internal control, and the values were calculated using the 2-ΔΔCq method. Information on the primers used in this experiment is summarized in Table 10.

**[Table 10]**

| **Gene name** | **Primer sequence (5'→3')** | **SEQ ID NO** |
|---|---|---|
| SP-D | F: AGGTCCAGTTGGACCCAAAGGA | 48 |
| | R: CTGGTTTGCCTTGAGGTCCTATG | 49 |
| MMP-7 | F: AGGTGTGGAGTGCCAGATGTTG | 50 |
| | R: CCACTACGATCCGAGGTAAGTC | 51 |
| Tenascin-C | F: GAGACCTGACACGGAGTATGAG | 52 |
| | R: CTCCAAGGTGATGCTGTTGTCTG | 53 |
| Periostin | F: CAGCAAACCACTTTCACCGACC | 54 |
| | R: AGAAGGCGTTGGTCCATGCTCA | 55 |
| CXCK13 | F: CATAGATCGGATTCAAGTTACGCC | 56 |
| | R: GTAACCATTTGGCACGAGGATTC | 57 |
| PAI-1 | F: CCTCTTCCACAAGTCTGATGGC | 58 |
| | R: GCAGTTCCACAACGTCATACTCG | 59 |
| 18S rRNA | F: CAGCGTGGTCAGGATAGAAC | 40 |
| | R: CTTGATTTGAATGCAGCGGAC | 41 |

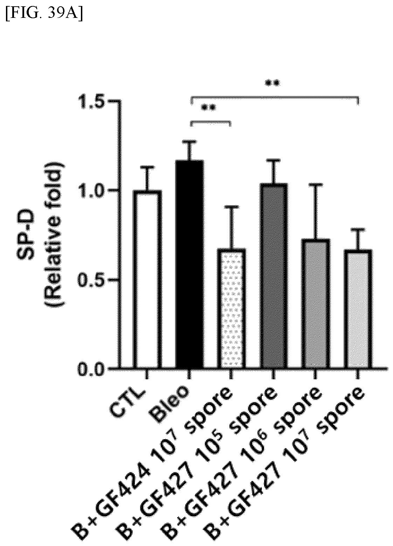

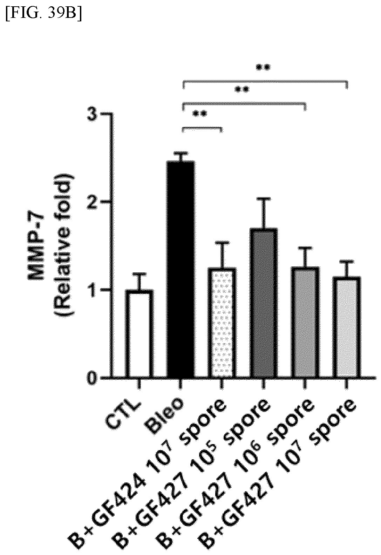

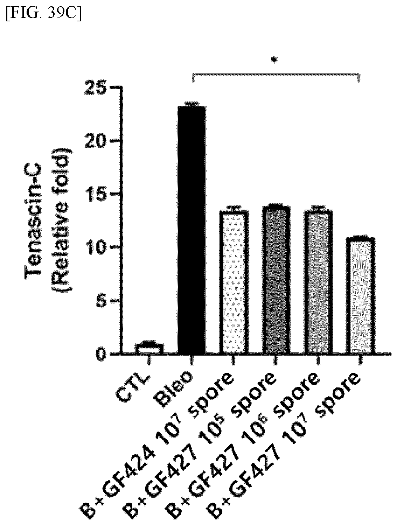

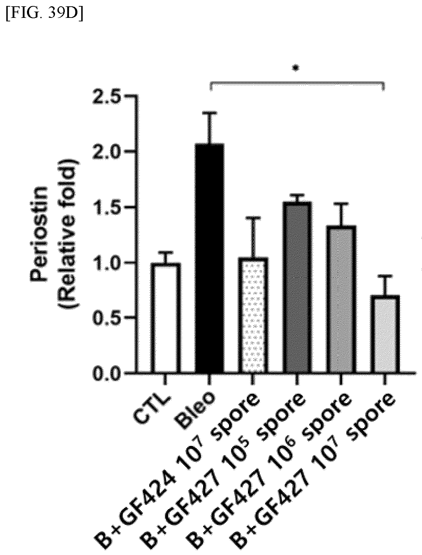

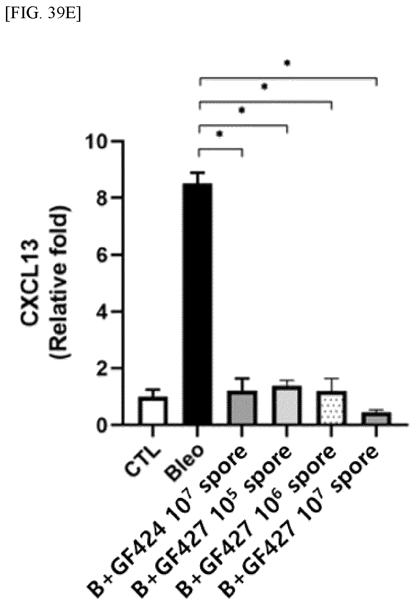

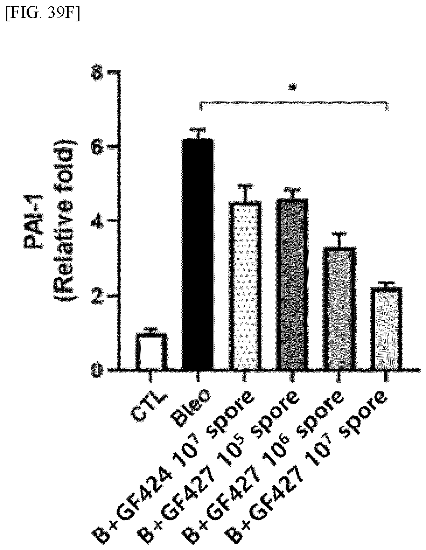

Gene expression levels of six key markers for identifying pulmonary fibrosis were compared. As a result, as shown in FIGS. 39A to 39F, epithelial damage marker (SP-D), fibrosis markers (MMP-7, Tenascin-C, and Periostin), inflammation marker (CXCL13), and thrombosis marker (PAI-1) were all decreased by the spores from SOD-overexpressing strains (GF424 and GF427), with a more effective decrease observed as the concentration of the SOD-overexpressing strain spores increased.

### Example 6.9. Measurement of survival rate

It was identified in a pulmonary fibrosis mouse model whether administration of the spores from SOD-overexpressing strains (GF424 and GF427) had an effect on survival rates of the mouse model. The survival rate was estimated by the Kaplan-Meier method in the same manner as in Example 2.8, and statistical differences were calculated using the log-rank test.

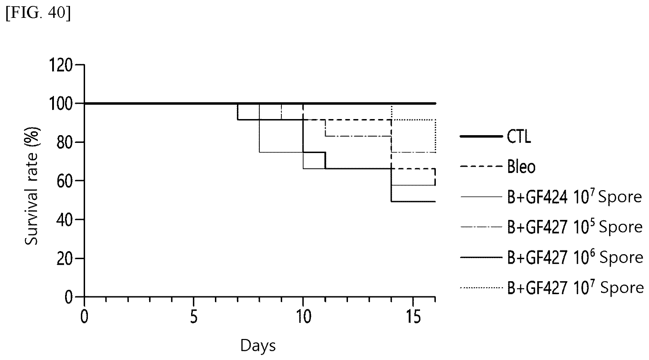

As a result of the estimation, referring to FIG. 40, the survival rate of the control group (CTL, N=6/6) was 100%, whereas the survival rate of the bleomycin-treated group (Bleo, N=7/12) decreased to 58.3%. On the other hand, the GF424 strain spore-administered group (B + GF424 spores at 1×10⁷ CFU, N = 7/12) showed a survival rate of 58.3%, which was not different from the bleomycin-treated group; the GF427 10⁵ spores-administered group (B + GF427 spores at 1×10⁵ CFU, N = 9/12) showed a survival rate of 75%; the GF427 10⁶ spores-administered group (B + GF427 spores at 1×10⁶ CFU, N = 6/12) showed a survival rate of 50%; and the GF427 10⁷ spores-administered group (B + GF427 spores at 1×10⁷ CFU, N = 9/12) showed a survival rate of 75%. In the GF427 10⁷ spore-administered group, death of individuals occurred the latest, and the highest number of surviving individuals remained, although the results were not statistically significant. These results suggest that administration of an SOD to a model with induced pulmonary fibrosis has therapeutic effects on pulmonary fibrosis and thus can lead to improved survival rates.

### Statistical Analysis

All numerical data were subjected to statistical analysis using the GraphPad Prism statistical program (Ver 9.0). All data are presented as mean ± SEM within each group, and the data was analyzed using a t-test (two-way analysis of variance). * indicates p < 0.05, ** indicates p < 0.01, *** indicates p < 0.001, and **** indicates p < 0.0001.

### Conclusion

In this example, the *Bacillus velezensis* strain spores and the oral SOD were used to verify that oral administration of the spores was effective in preventing or treating pulmonary fibrosis. Specifically, it was found that for both the mouse model (model for evaluating preventive effects), in which pulmonary fibrosis was induced after oral administration of GF424 spores for a total of 3 weeks, and the mouse model (model for evaluating therapeutic effects), in which GF424 spores were orally administered for a total of 12 days after induction of pulmonary fibrosis, progression of pulmonary fibrosis was inhibited, indicating an effect of ameliorating pulmonary fibrosis.

It has been found that induction of idiopathic pulmonary fibrosis by bleomycin is closely related to reactive oxygen species (ROS). Therefore, the concentrations of representative byproducts produced by reactive oxygen were measured in lung tissue. As a result, it was found that an SOD decreases reactive oxygen byproducts, demonstrating that an SOD effectively decreases ROS in the body.

It was found that pulmonary fibrosis was more effectively prevented or treated in the SOD-overexpressing strain spore-administered group or the SOD normal strain spore-administered group than the group administered with the spores from SOD knock-out strain with SOD removed; and in particular, a signifcant effect was observed in the SOD-overexpressing strain spore-administered group. From the results, it was found that an SOD exerted preventive and therapeutic effects on pulmonary fibrosis by removing reactive oxygen in the body, and such preventive and therapeutic effects also contribute to an increase in the mouse survival rate. On the other hand, an SOD in the form of spores overcame the disavantage (degraded before being absorbed in the body) that may occur when an SOD is orally administered in free form, which results in further increased bioavailability and thus significant therapeutic efficacy. In addition, the oral SOD (GF103) with increased stability against gastric acid in the body was effective in ameliorating pulmonary fibrosis even in a case of being administered alone; and a slightly increased effect was observed in a case where the oral SOD was administered in combination with the normal SOD strain spores (Spore SOD (+)), although the effect was not significant. From the results, it was found that the oral SOD (GF103) had the potential as a treatment for pulmonary fibrosis in both cases of being administered alone and in combination. In addition, the spores from GF427 strain, which was developed to produce about 4-fold more SOD than GF424, showed an effect of ameliorating pulmonary fibrosis even in a case of being administered in a smaller amount than the GF424 strain spores.

In conclusion, it was found that single and combined administration of *Bacillus velezensis* SOD-overexpressing strain spores (GF424 spores and GF427 spores), SOD-expressing normal strain spores, and oral SOD (GF103) could be effective in inhibiting and treating pulmonary fibrosis.

### Example 7. Identification of therapeutic effects of Bacillus velezensis strain spores on hepatic fibrosis in nonalcoholic steatohepatitis STAM^{™} model

### Example 7.1. Establishment of nonalcoholic steatohepatitis model

To identify whether the spores from GF427, which is an SOD-overexpressing strain, have a therapeutic effect on hepatic fibrosis in a nonalcoholic steatohepatitis (NASH) STAM^{™} animal model, a nonalcoholic steatohepatitis animal model was established, and the results obtained by using the spores were compared and analyzed with the results obtained by using the spores from GF423, which is an SOD-expressing normal strain, and the oral SOD (GF103).

C57BL/6J mice (14-day-pregnant female, 6-week-old male) (purchased from SLC, Japan) were maintained in a SPF facility with controlled temperature (23 ± 3°C), humidity (50 ± 20%), lighting (12-hour artificial light/dark cycle; light cycle from 08:00 to 20:00), and air exchange.

NASH was induced in all groups except the normal group of 8 mice as follows: On the second day after birth, the male mice were injected subcutaneously once with 200 µg streptozotocin (STZ, purchased from Sigma-Aldrich) solution. After the mice reached 4 weeks of age, a solid high-fat diet (HFD, 57 kcal% fat, purchased from CLEA Japan, Inc., Cat# HFD32) was placed on a metal lid on top of the cage so that the mice access it ad libitum, thereby inducing NASH

The 10-week-old mice with induced NASH were randomized to 9 groups, each containing 8 mice, based on their body weight on the day before the start of treatment. The randomization was performed by weight-stratified random sampling using Excel software. The NASH model mice were stratified by the body weight, and the standard deviation (SD) was calculated to minimize the difference in mean body weight between the groups. The mice were identified by ear punch, and each cage was labeled with a specific identification code for further identification.

The animal model for evaluating the therapeutic effect on hepatic fibrosis was divided into a total of 10 groups, as shown in Table 11, and 8 mice were randomized to each group as described above.

**[Table 11]**

| Group | Experimental group | Mouse (number) | Test substance | Concentration (units/mouse) | Dose (ml/kg) | Dosing regimen |
|---|---|---|---|---|---|---|
| 1 | Normal group | Normal 8) | - | - | - | - |
| 2 | Disease control group | STAM (8) | - | - | - | - |
| 3 | GF427 lowest-dose administration group | STAM (8) | GF427 | 0.05 x 10⁷ CFU | 100 µL | PO, QD, Weeks 10 to 13 |
| 4 | GF427 low-dose administration group | STAM (8) | GF427 | 0.1 x 10⁷ CFU | 100 µL | PO, QD, Weeks 10 to 13 |
| 5 | GF427 middle-dose administration group | STAM (8) | GF427 | 0.5 x 10⁷ CFU | 100 µL | PO, QD, Weeks 10 to 13 |
| 6 | GF427 high-dose administration group | STAM (8) | GF427 | 1 x 10⁷ CFU | 100 µL | PO, QD, Weeks 10 to 13 |
| 7 | GF427 higest-dose administration group | STAM (8) | GF427 | 5 x 10⁷ CFU | 100 µL | PO, QD, Weeks 10 to 13 |
| 8 | GF423-administered group | STAM (8) | GF423 | 1 x 10⁷ CFU | 100 µL | PO, QD, Weeks 10 to 13 |
| 9 | GF103-administered group | STAM (8) | GF103 | 10 U | 100 µL | PO, QD, Weeks 10 to 13 |
| 10 | Telmisartan-administered group | STAM (8) | Telmisartan | 10 mg/kg | 10 mL/kg | PO, QD, Weeks 10 to 13 |

Here, PO means oral administration and QD means once a day.

The GF427-administered groups were administered once a day at five dose levels: 0.05 x 10⁷ (lowest dose), 0.1 x 10⁷ (low dose), 0.5 x 10⁷ (middle dose), 1 x 10⁷ (high dose), and 5 x 10⁷ CFU (highest dose) per mouse. The GF423-administered group was administered once a day at a dose of 1 x 10⁷ CFU per mouse. GF103 was administered once a day at a dose of 10 U per mouse. Telmisartan was administered once a day at a dose of 10 mg/kg. GF427, GF423, and GF103 were administered orally in the form of a vehicle at a dose of 100 µL, and telmisartan was administered orally in the form of being supplemented with reverse osmosis water (RO water) at a dose of 10 mL/kg. The normal and disease control groups were not subjected to any treatment before sacrifice, and were sacrificed at 13 weeks of age by exsanguination via direct cardiac puncture under isoflurane (purchased from Pfizer Inc.) anesthesia. The animals were monitored daily for viability, clinical signs (lethargy, convulsion, respiratory distress), and behavior to observe whether there are any significant clinical signs of toxicity, coma, and death before and after administration of the test substance. If the animals showed a weight loss of less than 25% within a week or exhibited signs of coma, such as a prone posture, they were euthanized before the end of the study and no samples were collected. All animal care and experiments were conducted in accordance with laws and regulations related to animal welfare and management.

### Example 7.2. Preparation of test substances

The GF427 spores and GF423 spores used in Example 7.1 were prepared in the same manner as in Preparation Example 1, and the GF103 used in Example 7.1 was prepared in the same manner as in Example 5.1. Telmisartan (Micardis^{®}) was purchased from Boehringer Ingelheim GmbH (Germany). The SOD-expressing *Bacillus velezensis* GF427 strain, which expresses SOD used in this example, was prepared, from the *Bacillus velezensis* strain (Accession No.: KCTC 13222BP, Accession Date: 20170306) ("GF423 strain") deposited at the Korean Collection for Type Cultures (KCTC), by replacing the promoter sequence of the GF423 sodA gene with a nucleotide sequence having stronger promoter activity to increase SOD activity (see Preparation Example 2). The test substances GF427 and GF423 were vortexed for at least 30 seconds prior to use. When performing aliquoting, a well-stirred solution was used to ensure that an appropriate amount of CFU/mL was used for aliquoting to obtain a desired stock volume. For the test substance GF103, 10 mg of GF103 powder was diluted with an appropriate amount of distilled water before daily administration. The test substances GF427, GF423, and GF103 were stored and maintained at 4°C before administration. The test substance telmisartan was freshly prepared before administration. One tablet of telmisartan was transferred to a mortar, and then ground with a pestle while slowly adding RO water to obtain a homogeneous suspension of 1 mg/mL.

### Example 7.3. Preparation of samples

13-Week-old mice were sacrified. Then, frozen plasma samples, frozen liver samples, OCT-embedded liver blocks, and paraffin-embedded liver blocks were collected and stored.

### Preparation of plasma samples

At the end of the study, non-fasting blood was collected by direct cardiac puncture using a pre-chilled syringe. The collected blood was transferred to a pre-chilled polypropylene tube with anticoagulant (Novoheparin, purchased from Mochida Pharmaceutical Co. Ltd.) and stored on ice until centrifugation. The blood sample was centrifuged at 1,000×g for 15 minutes at 4°C. After centrifugation, the supernatant was collected and stored at -80°C for biochemical experiments and transportation.

### Preparation of liver samples

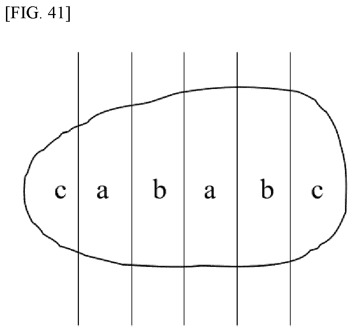

At sacrifice of the mice, the whole liver was collected and washed with cold saline. Photographs of the individual whole liver (parietal side and visceral side) were taken. Then, the liver weight was measured and the liver-to-body weight ratio was calculated. The left outerlobe of the liver was separated and dissected, and stored as shown in FIG. 41. The liver specimens, labeled with "a" in FIG. 41, were embedded in optimal cutting temperature (OCT) compound (purchased from Sakura Finetek, Japan) and stored at -80°C for further analysis or transportation. The liver specimens labeled with "b" were fixed in Bouin's solution (purchased from Sigma-Aldrich Japan) for 24 hours. After fixation, these specimens were paraffin-embedded for H&E and Sirius red staining. Liver specimens labeled with "c" were rapidly frozen in liquid nitrogen and stored at -80°C for gene expression analysis.

In addition, the left and right inner lobes were rapidly frozen in liquid nitrogen and stored at -80°C for transportation. The right lobe was rapidly frozen in liquid nitrogen and stored at -80°C for biochemical experiments. The caudate lobe was rapidly frozen in liquid nitrogen and stored at -80°C for transportation.

### Example 7.4. Monitoring of mouse status during experiment

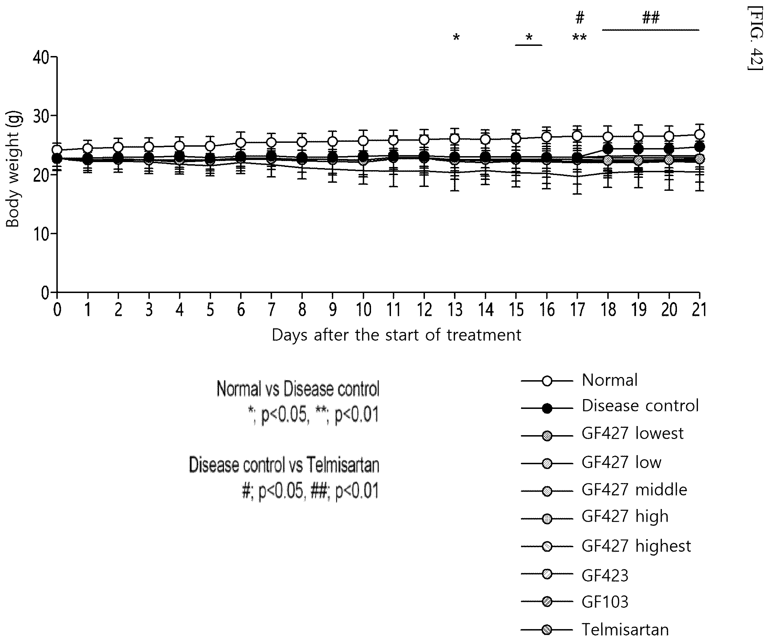

Body weight changes in the mice belonging to 10 groups were monitored over a 21-day period during which NASH was induced and treatment was performed. As a result, referring to FIG. 42, the mean body weight of the disease control group was significantly lower than that of the normal group on days 13, 15, 16, and 17. The mean body weight of the telmisartan-administered group was significantly lower than that of the disease control group from day 17 to day 21. On the other hand, there was no significant difference in the mean body weight change between the disease control group and other treatment groups (groups 3 to 9) on any day during the treatment period.

During the treatment period, the mice found dead before day 21 were as follows. In the disease control group, one of the eight mice was euthanized. In each of the GF427 middle-dose administration group and the GF427 high-dose administration group, one of the eight mice was found dead. In the GF423-administered group, two of the eight mice were found dead. In the telmisartan-administered group, two of the eight mice were euthanized.

### Example 7.5. Monitoring of organ weight changes

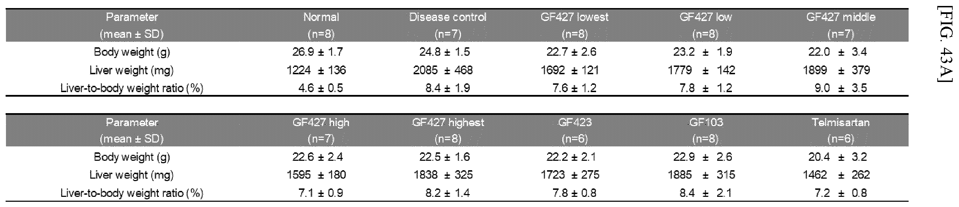

On the 21st day when all treatments were completed, the mice belonging to the 10 groups were subjected to measurement of daily mean body weight, liver weight, and liver-to-body weight ratio (FIG. 43A).

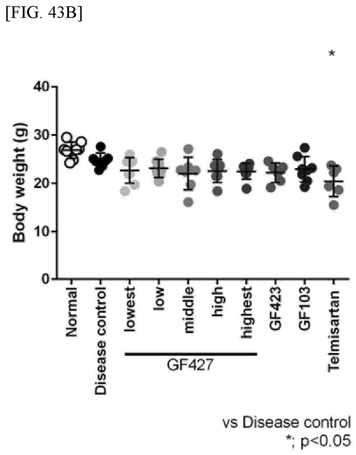

Referring to FIGS. 43A and 43B, the disease control group tended to show a decrease in daily mean body weight on the day of sacrifice compared to the normal group. The telmisartan-administered group showed a significant decrease in daily mean body weight on the day of sacrifice compared to the disease control group. All treatment groups tended to show a decrease in daily mean body weight on the day of sacrifice compared to the disease control group.

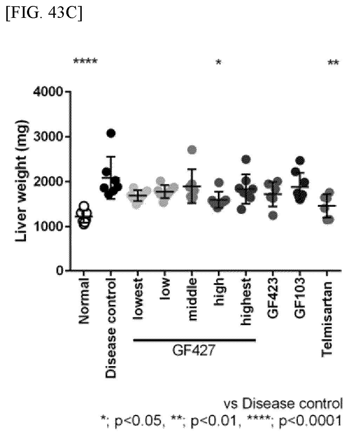

Referring to FIGS. 43A and 43C, the disease control group showed a significant increase in mean liver weight compared to the normal group. On the other hand, the GF427 high-dose administration group and the telmisartan-administered group showed a significant decrease in mean liver weight compared to the disease control group. The GF427 lowest-dose administration group, the GF427 low-dose administration group, and the GF423-administered group tended to show a decrease in mean liver weight compared to the disease control group. There was no significant difference in mean liver weight between the disease control group and the other groups.

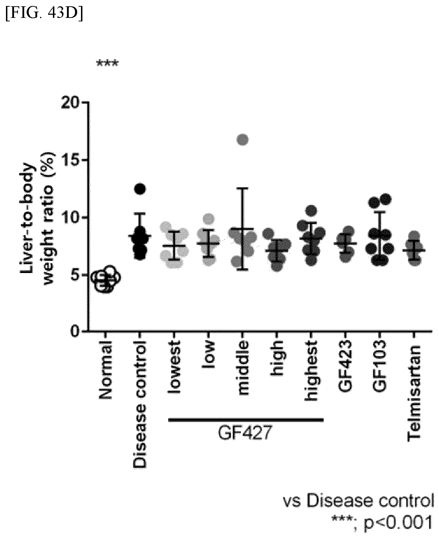

Referring to FIGS. 43A and 43D, the disease control group showed a significant increase in mean liver-to-body weight ratio compared to the normal group. On the other hand, the GF427 high-dose administration group and the telmisartan-administered group tended to show a decrease in mean liver-to-body weight ratio compared to the disease control group. There was no significant difference in mean liver-to-body weight ratio between the disease control and the other groups.

### Example 7.6. Biochemical analysis of plasma and liver tissue

Plasma or liver samples were collected from the mice in each group according to the preparation methods for plasma samples and liver samples as described in Example 7.3. Then, the whole blood glucose levels, plasma ALT levels, plasma CK-18 levels, and liver triglyceride contents were measured and compared (FIG. 44A).

### Measurement of whole blood glucose level

Non-fasting whole blood glucose level was measured using a Stat Strip glucose meter (purchased from NIPRO CORPORATION). As a result of the measurement, referring to FIG. 44B, the disease control group showed a significant increase in whole blood glucose level compared to the normal group. The GF427 lowest-dose administration group, the GF427 highest-dose administration group, and the GF423-administered group tended to show a decrease in whole blood glucose level compared to the disease control group. There was no significant difference in whole blood glucose level between the disease control group and the other groups.

### Measurement of plasma ALT level

Plasma ALT level was measured using FUJI DRI-CHEM 7000 (purchased from Fujifilm Corporation). As a result of the measurement, referring to FIG. 44C, the disease control group showed a significant increase in plasma ALT level compared to the normal group. The GF427 high-dose administration group showed a significant decrease in plasma ALT level compared to the disease control group. The GF427 lowest-dose administration group, the GF427 highest-dose administration group, and the telmisartan-administered group tended to show a decrease in plasma ALT level compared to the disease control group. There was no significant difference in plasma ALT level between the disease control group and the other groups.

### Measurement of plasma CK-18 level

Plasma CK-18 level was quantified using the mouse cytokeratin 18-M30 ELISA kit (purchased from Cusabio Biotech Co., Ltd). As a result of the measurement, referring to FIG. 44D, the disease control group showed a significant increase in plasma CK-18 level compared to the normal group. All groups showed a significant decrease in plasma CK-18 level compared to the disease control group.

### Measurement of liver triglyceride content

Total liver lipid extract was obtained by the Folch method (see Folch J. et al., J. Biol. Chem. 1957;226: 497). The liver sample was homogenized with chloroform-methanol (2:1, v/v) and incubated overnight at room temperature. Next, the sample was washed with chloroform-methanol-water (8:4:3, v/v/v). Then, the sample was evaporated to dryness and dissolved in isopropanol. The liver triglyceride content was measured using the Triglyceride E-Test (purchased from FUJIFILM Wako Pure Chemical Corporation).

As a result of the measurement, referring to FIG. 44E, the disease control group showed a significant increase in liver triglyceride content compared to the normal group. On the other hand, all groups except the GF427 lowest=dose administration group showed a significant decrease in hepatic triglyceride content compared to the disease control group. The GF427 lowest-dose administration group tended to show a decrease in liver triglyceride content compared to the disease control group.

### Example 7.7. Identification of hepatic fibrosis through H&E staining of liver tissue and evaluation of NAFLD activity score

The paraffin block of liver tissue prepared in Example 7.3 was sectioned using a rotary microtome (purchased from Leica Microsystems). After sectioning, each slide was coded with a number for blind evaluation. Each number was generated using the RAND function in Excel software, sorted in ascending order, and assigned to the slides. The tissue slides were subjected to H&E staining and evaluated by the investigator.

For H&E staining, the liver tissue paraffin block pre-fixed in Bouin's solution were sectioned and stained with Lillie-Mayer hematoxylin (purchased from Muto Pure Chemicals Co., Ltd.) and eosin solution (purchased from FUJIFILM Wako Pure Chemical Corporation). NAFLD activity score (NAS) was calculated according to the criteria of Kleiner as shown in Table 12 (see Kleiner DE. et al., Hepatology, 2005;41:1313]). For scoring of NAS, bright field images of H&E-stained sections were captured using a digital camera (Cat. #: DFC295, purchased from Leica) at 50- and 200-fold magnifications. Steatosis score in 1 section/mouse (representative 1 field at 50-fold magnification), inflammation score in 1 section/mouse (representative 1 field around the central vein at 200-fold magnification) and ballooning score in 1 section/mouse (representative 1 field around the central vein at 200-fold magnification) were estimated.

**[Table 12]**

| Item | Extent | | Score |
|---|---|---|---|
| Steatosis | Steatosis at 50-fold magnification | | |
| | | <5% | 0 |
| | | 5-33% | 1 |
| | | >33-66% | 2 |
| | | >66% | 3 |
| Lobular inflammation | Estimation of inflammatory foci | | |
| | | No foci | 0 |
| | | <2 foci/200x | 1 |
| | | 2-4 foci/200x | 2 |
| | | >4 foci/200x | 3 |
| Ballooning | Estimation of number of ballooning cells | | |
| | | None | 0 |
| | | Few ballooning cells | 1 |
| | | Many cells/prominent ballooning | 2 |

Representative photomicrographs of H&E-stained liver sections were taken (FIGS. 45A to 45C). Based on the photomicrographs, scores for respective parameters and NAS values were presented in a table (FIG. 45E) and graphs (FIGS. 45F to 45I) to assess the degree of steatosis, inflammatory cell infiltration, and hepatocyte ballooning in each group. Thorough examination of the liver sections from the disease control group showed microvesicular and macrovesicular fat deposition, hepatocyte ballooning, and inflammatory cell infiltration compared to the normal group (FIG. 45D). On the other hand, compared to the disease control group, the other administration groups tended to show a decrease in steatosis, inflammatory cell infiltration, and hepatocyte ballooning. Referring to FIGS. 45E to 45I, the disease control group showed a significant increase in NAS compared to the normal group. On the other hand, the GF427 low-dose, middle-dose, high-dose, and highest-dose administration groups, GF423-administerd group, and telmisartan-administered group showed a significant decrease in NAS compared to the disease control group. NAS in the GF 103-administered group tended to decreased compared to the disease control group. There was no significant difference in NAS between the disease control group and the GF427 lowest-dose administration group.

### Example 7.8. Identification of hepatic fibrosis through Sirius red staining of liver tissue

To visualize collagen deposition, Bouin-fixed liver sections were stained with picro-Sirius red solution (purchased from FUJIFILM Wako Pure Chemical Corporation). First, the sections were deparaffinized, hydrophilized sequentially with xylene, 100-70% alcohol series, and RO water, and then treated with 0.03% picro-Sirius Red solution (Cat No. 194-16202) for 60 minutes. The stained sections were washed with 0.5% acetic acid solution and RO water, and then dehydrated. The resulting sections were cleared with 70-100% alcohol series and xylene, and then sealed with Entellan^{®} new (Merck, Germany) and used for observation.

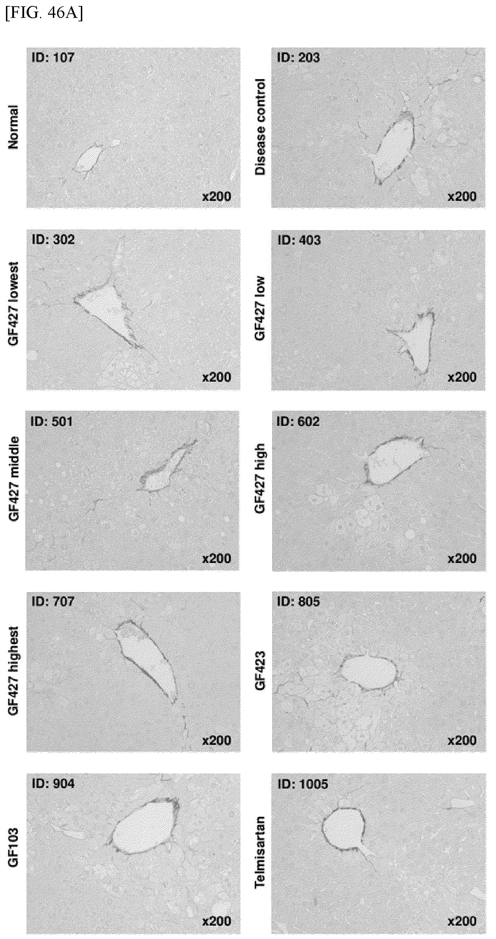

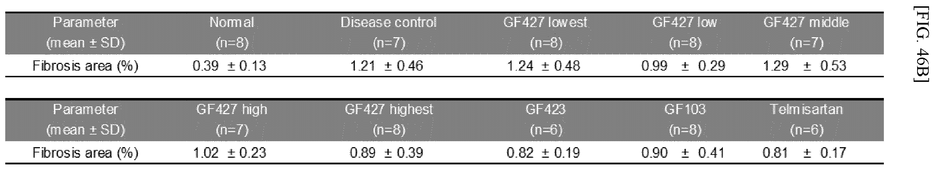

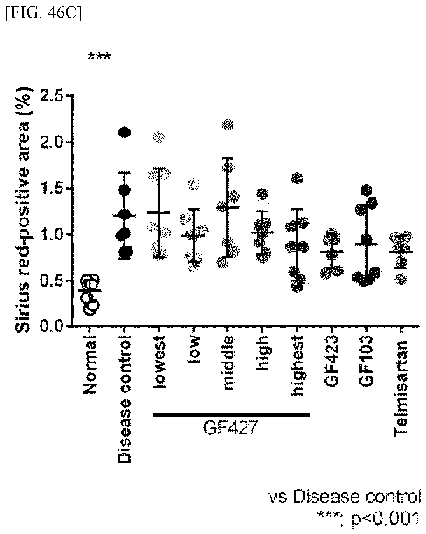

For quantitative analysis of fibrosis area, bright field images of the Sirius red-stained section were captured around the central vein using a digital camera (DFC295, purchased from Leica) at 200-fold magnification, and the positive areas in 5 fields/section were measured using ImageJ software (National Institute of Health, USA). Representative photomicrographs of the Sirius red-stained liver sections for evaluation of the fibrosis area are shown in FIG. 46A.

Referring to FIGS. 46A to 46C, liver sections from the disease control group showed increased collagen deposition (Sirius red positive) in the pericentral region of the liver lobule compared to the normal group. In addition, the disease control group showed a significant increase in fibrosis area (Sirius red-positive area) compared to the normal group. On the other hand, the fibrosis area in the GF427 highest-dose administration group, the GF423-administered group, the GF103-administered group, and the telmisartan-administered group tended to decrease compared to the disease control group. There was no significant difference in fibrosis area between the disease control group and the other groups.

### Statistical Analysis

Statistical analysis was performed using Prism Software 6 (GraphPad Software, USA) and the Bonferroni Multiple Comparison Test.

*P* values < 0.05 were considered statistically significant. Results are expressed as mean ± SD.

A trend or tendency was assumed when a one-sided t-test returned P values < 0.1. For all results, comparisons were made between the following groups:
1) Group 2 (disease control group) vs. Group 1 (normal group)
2) Group 2 (disease control group) vs. Group 3 (GF427 lowest-dose administration group)
3) Group 2 (disease control group) vs. Group 4 (GF427 low-dose administration group)
4) Group 2 (disease control group) vs. Group 5 (GF427 middle-dose administration group)
5) Group 2 (disease control group) vs. Group 6 (GF427 high-dose administration group)
6) Group 2 (disease control group) vs. Group 7 (GF427 highest-dose administration group)
7) Group 2 (disease control group) vs. Group 8 (GF423-administered group)
8) Group 2 (disease control group) vs. Group 9 (GF103-administered group)
9) Group 2 (disease control group) vs. Group 10 (telmisartan-administered group)

### Conclusion

This example was conducted using *Bacillus velezensis* GF427 and GF423 strains, and oral SOD (GF103). As a result, it was identified that administration of the spores from the strains or the oral SOD (GF103) was effective in treating hepatic fibrosis in a nonalcoholic steatohepatitis animal model. Specifically, in a case where a mouse model, in which NASH had been induced through streptozotocin solution and a high-fat diet, was orally administered with GF427 strain spores, GF423 strain spores, or GF103 in the form of a vehicle at a dose of 100 uL once a day for 21 days, it was found that such administration inhibited progression of hepatic fibrosis, indicating an effect of ameliorating NASH.

The GF427-administered group tended to show a decrease in whole blood glucose level and fibrosis area compared to the disease control group; and the GF427-administered group showed a significant decrease in plasma ALT level, plasma CK-18 level, liver triglyceride content, and NAS compared to the disease control group. The GF423-administered group showed a significant decrease in plasma CK-18 level, liver triglyceride content, and NAS compared to the disease control group. In addition, the GF423-administered group tended to show a decrease in whole blood glucose level and fibrosis area compared to the disease control group. The GF103-administered group showed a significant decrease in plasma CK-18 level and liver triglyceride content compared to the disease control group. In addition, the GF103-administered group tended to show a decrease in NAS and fibrosis area compared to the disease control group.

In conclusion, it was identified that the spores from the GF427 strain engineered to overexpress an SOD, the spores from the GF423 strain expressing wild-type SOD, and the oral SOD GF103 could all be useful materials for inhibition and treatment of hepatic fibrosis in a case of being administered alone.

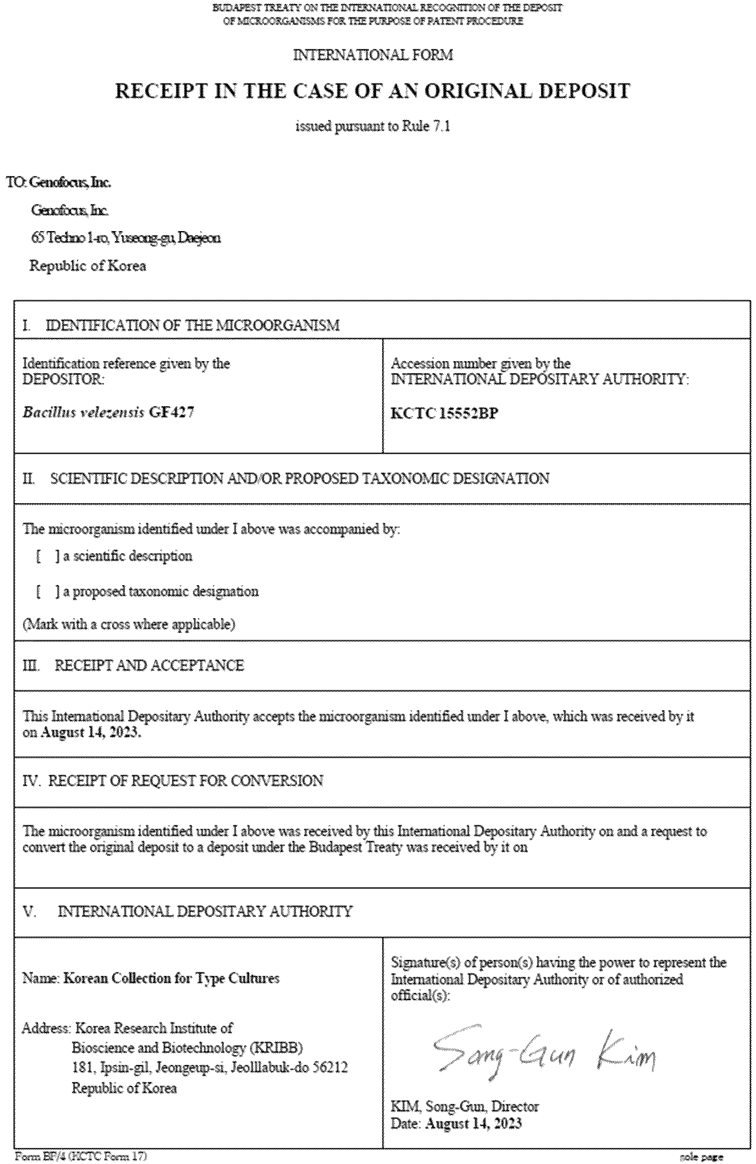
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Genofocus, Inc.

Genofocus, Inc.

65 Techno 1-ro, Yuseong-gu, Daejeon

Republic of Korea

I. IDENTIFICATION OF THE MICROORGANISM
Identification reference given by the
DEPOSITOR:

Bacillus velezensis GF427 | Accession number given by the
INTERNATIONAL DEPOSITARY AUTHORITY:

KCTC 15552BP
II. SCIENTIFIC DESCRIPTION AND/OR PROPOSED TAXONOMIC DESIGNATION
The microorganism identified under I above was accompanied by:

[ ] a scientific description

[ ] a proposed taxonomic designation

(Mark with a cross where applicable)
III. RECEIPT AND ACCEPTANCE
This International Depositary Authority accepts the microorganism identified under I above, which was received by it
on August 14, 2023.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: August 14, 2023

Form BP/4 (KCTC Form 17) sole page

## Claims

1. A pharmaceutical composition for treating or preventing a fibrotic disease, comprising, as an active ingredient, at least one selected from the group consisting of a *Bacillus sp.* strain, a *Bacillus sp.* strain spore, and a polypeptide having superoxide dismutase (SOD) activity.

2. The pharmaceutical composition of claim 1, wherein the *Bacillus sp.* strain is an SOD-expressing strain or a strain that has undergone mutation or recombination to overexpress an SOD.

3. The pharmaceutical composition of claim 1, wherein the *Bacillus sp.* strain spore comprises spores derived from an SOD-expressing strain, or a strain that has undergone mutation or recombination to overexpress an SOD.

4. The pharmaceutical composition of claim 1, wherein the *Bacillus sp.* strain is a *Bacillus velezensis* species strain.

5. The pharmaceutical composition of claim 1, wherein the *Bacillus sp.* strain is at least one selected from the *Bacillus velezensis* strain deposited under the accession number KCTC 13222 BP, the *Bacillus velezensis* strain deposited under the accession number KCTC 13227 BP, and the *Bacillus velezensis* strain deposited under the accession number KCTC 15552 BP.

6. The pharmaceutical composition of claim 1, wherein the polypeptide is an Mn-SOD.

7. The pharmaceutical composition of claim 1, wherein the polypeptide is a deamidated Mn-SOD.

8. The pharmaceutical composition of claim 1, wherein the polypeptide is derived from a *Bacillus sp.* strain.

9. The pharmaceutical composition of claim 8, wherein the *Bacillus sp.* strain is a *Bacillus velezensis* species strain.

10. The pharmaceutical composition of claim 8, wherein the *Bacillus sp.* strain is at least one selected from the *Bacillus velezensis* strain deposited under the accession number KCTC 13222 BP, the *Bacillus velezensis* strain deposited under the accession number KCTC 13227 BP, and the *Bacillus velezensis* strain deposited under the accession number KCTC 15552 BP.

11. The pharmaceutical composition of claim 1, wherein the polypeptide comprises the amino acid sequence of SEQ ID NO: 2, SEQ ID NO: 4, SEQ ID NO: 5, or SEQ ID NO: 6.

12. The pharmaceutical composition of claim 1, wherein the polypeptide is coated with a coating agent.

13. The pharmaceutical composition of claim 12, wherein the coating agent comprises shellac.

14. The pharmaceutical composition of claim 1, wherein the fibrotic disease is a pulmonary fibrotic disease or a hepatic fibrotic disease.

15. The pharmaceutical composition of claim 14, wherein the pulmonary fibrotic disease is selected from the group consisting of interstitial lung disease, pulmonary fibrosis, and idiopathic pulmonary fibrosis.

16. The pharmaceutical composition of claim 14, wherein the hepatic fibrotic disease is selected from the group consisting of nonalcoholic steatohepatitis, hepatic fibrosis, idiopathic hepatic fibrosis, cirrhosis, alcoholic steatohepatitis, chronic liver disease, and viral hepatitis.

17. The pharmaceutical composition of claim 1, wherein the active ingredient is orally administered.

18. The pharmaceutical composition of claim 1, wherein the composition comprises at least two components selected from the group consisting of the *Bacillus sp.* strain, the *Bacillus sp.* strain spore, and the polypeptide having SOD activity, and the at least two components are administered simultaneously, sequentially, or in reverse order.

19. A method for preventing, ameliorating, or treating a fibrotic disease, comprising administering, to a subject, a composition comprising at least one selected from the group consisting of a *Bacillus sp.* strain, a *Bacillus sp.* strain spore, and a polypeptide having superoxide dismutase activity.

20. A food composition for preventing or ameliorating a fibrotic disease, comprising at least one selected from the group consisting of a *Bacillus sp.* strain, a *Bacillus sp.* strain spore, and a polypeptide having superoxide dismutase activity.
